# EUROPEAN PATENT APPLICATION

(11) **EP 4 471 065 A1**
(43) Date of publication of application: **04.12.2024**
(21) Application number: 23746414.4
(22) Date of filing: 28.01.2023
(51) Int. Cl.: C07K 16/28, C12N 15/13, C12N 5/10, C12N 15/63, C12N 15/64, A61P 35/00, A61K 39/395

(54) **GPRC5D ANTIBODY AND USE THEREOF**

(30) Priority: 29.01.2022 CN 202210111504; 17.08.2022 CN 202210985594
(71) Applicant: Carsgen Life Sciences Co., Ltd., Shanghai 200131 (CN)
(72) Inventor: ZHOU, Liang, Shanghai 200231 (CN); WANG, Peng, Shanghai 200231 (CN); REN, Pengju, Shanghai 200231 (CN); JIANG, Hua, Shanghai 200231 (CN); LI, Zonghai, Shanghai 200231 (CN)
(74) Representative: Lavoix
(86) International application number: PCT/CN2023/073614
(87) International publication number: WO 2023/143537

(57) **Abstract**

The present application relates to an antibody that binds to GPRC5D and use thereof. The present application further relates to a cell, a pharmaceutical composition, and a combination therapy comprising the antibody that binds to GPRC5D, and a method for preparing the antibody.

## Description

### RELATED APPLICATIONS

This patent application claims priority to Chinese patent application number 202210111504.5 filed on January 29, 2022, and claims priority to Chinese patent application number 202210985594.0 filed on August 17, 2022.

### SEQUENCE LISTING FILE SUBMITTED SIMULTANEOUSLY

The entire contents of the following XML file are incorporated herein by reference in their entirety: Sequence Listing in Computer Readable Format (CRF) (Name: FF00719PCT-sequence listing.xml, Date: 20230122, Size: 95.6KB).

### TECHNICAL FIELD

The present application relates to the field of tumor immunotherapy or diagnosis, and more specifically, to antibodies specifically binding to GPRC5D and uses thereof.

### BACKGROUND

Currently, the targeted treatments for multiple myeloma in clinical practice mainly include immunomodulatory agent (thalidomide, lenalidomide, pomalidomide), proteasome inhibitors (bortezomib, carfilzomib, ixazomib) and anti-CD38 monoclonal antibodies, etc. Although front-line therapy has achieved good results, the efficacy of the last-line therapy is very limited. CAR-T cell therapy targeting BCMA has shown good efficacy in clinical trials, but new treatments are urgently needed for cases with low BCMA expression or relapse after BCMA CAR-T treatment. Studies have shown that 65% of multiple myeloma patients express GPRC5D, so the clinical value of developing a treatment pathway targeting GPRC5D is prominent. Since GPRC5D is a relatively new target and a multiple transmembrane receptor, the development of its antibodies has certain technical barriers. Therefore, the GPRC5D antibody developed in this application, especially the fully human GPRC5D antibody, has a higher application value.

### SUMMARY

The purpose of the present application is to provide antibodies that specifically recognize GPRC5D. The present application also relates to a method for preparing anti-GPRC5D specific antibodies, comprising phage display technology and hybridoma technology. The present application also relates to the study of the properties and specificity of anti-GPRC5D antibodies (including but not limited to forms of Fab, scFv, scFv-Fc). The present application provides a chimeric antigen receptor (CAR) targeting GPRC5D and a method for preparing the same. The present application also provides isolated nucleic acids encoding the anti-GPRC5D antibodies and chimeric antigen receptors targeting GPRC5D of the present application. The present application also provides a host cell, wherein the host cell comprises the nucleic acid of the present application. The method further comprises culturing the host cell of the present application to produce the antibody or the CAR. The antibodies and/or CARs of the present application are used to tumor therapy or tumor diagnosiss.

In the first aspect, the present application provides an antibody that recognizes GPRC5D, wherein the antibody is selescted from any one of the following groups:
(1) an antibody comprising a heavy chain variable region, wherein the heavy chain variable region comprises a HCDR1 as shown in any one of SEQ ID NOs: 1, 11, 17, 70 or 71, and/or comprises a HCDR2 as shown in any one of SEQ ID NOs: 2, 12, 18, 72 or 73, and/or comprises a HCDR3 as shown in any one of SEQ ID NOs: 3, 7, 9, 13 or 19;
(2) an antibody comprising a light chain variable region, wherein the light chain variable region comprises LCDR1 as shown in any one of SEQ ID NOs: 4, 14, 20 or 74, and/or comprises LCDR2 as shown in any one of SEQ ID NOs: 5, 15, 21 or 75, and/or comprises LCDR3 as shown in any one of SEQ ID NOs: 6, 8, 10, 16 or 22;
(3) an antibody comprising a heavy chain variable region of the antibody as described in (1) and a light chain variable region of the antibody as described in (2);
(4) an antibody, which is a variant of the antibody described in any one of (1) to (3), and has the same or similar activity as the antibody described in any one of (1) to (3).

In certain embodiments, it comprises at least one CDR in the heavy chain variable region, wherein the heavy chain variable region comprises an amino acid sequence as shown in any one of SEQ ID NOs:23, 27, 31, 35, 39, 76 or 80 or a variant thereof, or comprises an amino acid sequence having at least 80% similarity to any one of the above sequences; and/or it comprises at least one CDR in the light chain variable region, wherein the light chain variable region comprises an amino acid sequence as shown in any one of SEQ ID NOs:25, 29, 33, 37, 41 or 78 or a variant thereof, or comprises an amino acid sequence having at least 80% similarity to any one of the above sequences.

In certain embodiments, the antibody comprises HCDR1, HCDR2, and HCDR3 in the heavy chain variable region, wherein the heavy chain variable region comprises an amino acid sequence as shown in any one of SEQ ID NOs:23, 27, 31, 35, 39, 76 or 80 or a variant thereof, or comprises an amino acid sequence having at least 80% similarity to any one of the above sequences; and/or it comprises LCDR1, LCDR2, and LCDR3 in the light chain variable region, wherein the light chain variable region comprises an amino acid sequence as shown in any one of SEQ ID NOs:25, 29, 33, 37, 41 or 78 or a variant thereof, or comprises an amino acid sequence having at least 80% similarity to any one of the above sequences.

In certain embodiments, the CDR region of the antibody heavy chain variable region and/or the CDR region of the light chain variable region are selected from the sequence of any one of the following groups, or variants thereof:
(1) an antibody, comprising HCDR1 shown as SEQ ID NO: 1, HCDR2 shown as SEQ ID NO: 2, and HCDR3 shown as SEQ ID NO: 3; LCDR1 shown as SEQ ID NO: 4, LCDR2 shown as SEQ ID NO: 5, and LCDR3 shown as SEQ ID NO: 6; or
(2) an antibody, comprising HCDR1 shown as SEQ ID NO: 1, HCDR2 shown as SEQ ID NO: 2, and HCDR3 shown as SEQ ID NO: 7; LCDR1 shown as SEQ ID NO: 4, LCDR2 shown as SEQ ID NO: 5, and LCDR3 shown as SEQ ID NO: 8; or
(3) an antibody, comprising HCDR1 shown as SEQ ID NO: 1, HCDR2 shown as SEQ ID NO: 2, and HCDR3 shown as SEQ ID NO: 9; LCDR1 shown as SEQ ID NO: 4, LCDR2 shown as SEQ ID NO: 5, and LCDR3 shown as SEQ ID NO: 10; or
(4) an antibody, comprising HCDR1 shown as SEQ ID NO: 11, HCDR2 shown as SEQ ID NO: 12, and HCDR3 shown as SEQ ID NO: 13; LCDR1 shown as SEQ ID NO: 14, LCDR2 shown as SEQ ID NO: 15, and LCDR3 shown as SEQ ID NO: 16; or
(5) an antibody, comprising HCDR1 shown as SEQ ID NO: 17, HCDR2 shown as SEQ ID NO: 18, and HCDR3 shown as SEQ ID NO: 19; LCDR1 shown as SEQ ID NO: 20, LCDR2 shown as SEQ ID NO: 21, and LCDR3 shown as SEQ ID NO: 22;
(6) an antibody, comprising HCDR1 shown as SEQ ID NO:70, HCDR2 shown as SEQ ID NO:72, and HCDR3 shown as SEQ ID NO:3; LCDR1 shown as SEQ ID NO:4, LCDR2 shown as SEQ ID NO:5, and LCDR3 shown as SEQ ID NO:6; or
(7) an antibody, comprising HCDR1 shown as SEQ ID NO:71, HCDR2 shown as SEQ ID NO:73, and HCDR3 shown as SEQ ID NO:3; LCDR1 shown as SEQ ID NO:4, LCDR2 shown as SEQ ID NO:5, and LCDR3 shown as SEQ ID NO:6; or
(8) an antibody, comprising HCDR1 shown as SEQ ID NO: 1, HCDR2 shown as SEQ ID NO: 2, and HCDR3 shown as SEQ ID NO: 3; LCDR1 shown as SEQ ID NO: 74, LCDR2 shown as SEQ ID NO: 75, and LCDR3 shown as SEQ ID NO: 6;
(9) an antibody, which is a variant of the antibody of any one of (1) to (8), and has the same or similar activity as the antibody of any one of (1) to (8).

In certain embodiments, the antibody is selected from the sequence of any one of the following groups, or variants thereof:
(1) an antibody, wherein the heavy chain variable region of the antibody has an amino acid sequence of SEQ ID NO:23 or an amino acid sequence having at least 80%, 90%, 91%, 92%, 93%, 94%, 95%, 96%, 97%, 98%, 99% similarity to SEQ ID NO:23, and the light chain variable region of the antibody has an amino acid sequence of SEQ ID NO:25 or an amino acid sequence having at least 80%, 90%, 91%, 92%, 93%, 94%, 95%, 96%, 97%, 98%, 99% similarity to SEQ ID NO:25;
(2) an antibody, wherein the heavy chain variable region of the antibody has an amino acid sequence of SEQ ID NO:27 or an amino acid sequence having at least 80%, 90%, 91%, 92%, 93%, 94%, 95%, 96%, 97%, 98%, 99% similarity to SEQ ID NO:27, and the light chain variable region of the antibody has an amino acid sequence of SEQ ID NO:29 or an amino acid sequence having at least 80%, 90%, 91%, 92%, 93%, 94%, 95%, 96%, 97%, 98%, 99% similarity to SEQ ID NO:29;
(3) an antibody, wherein the heavy chain variable region of the antibody has an amino acid sequence of SEQ ID NO:31 or an amino acid sequence having at least 80%, 90%, 91%, 92%, 93%, 94%, 95%, 96%, 97%, 98%, 99% similarity to SEQ ID NO:31, and the light chain variable region of the antibody has an amino acid sequence of SEQ ID NO:33 or an amino acid sequence having at least 80%, 90%, 91%, 92%, 93%, 94%, 95%, 96%, 97%, 98%, 99% similarity to SEQ ID NO:33;
(4) an antibody, wherein the heavy chain variable region of the antibody has an amino acid sequence of SEQ ID NO:35 or an amino acid sequence having at least 80%, 90%, 91%, 92%, 93%, 94%, 95%, 96%, 97%, 98%, 99% similarity to SEQ ID NO:35, and the light chain variable region of the antibody has an amino acid of SEQ ID NO:37 or an amino acid sequence having at least 80%, 90%, 91%, 92%, 93%, 94%, 95%, 96%, 97%, 98%, 99% similarity to SEQ ID NO:37;
(5) an antibody, wherein the heavy chain variable region of the antibody has an amino acid sequence of SEQ ID NO:39 or an amino acid sequence having at least 80%, 90%, 91%, 92%, 93%, 94%, 95%, 96%, 97%, 98%, 99% similarity to SEQ ID NO:39, and the light chain variable region of the antibody has an amino acid sequence of SEQ ID NO:41 or an amino acid sequence having at least 80%, 90%, 91%, 92%, 93%, 94%, 95%, 96%, 97%, 98%, 99% similarity to SEQ ID NO:41;
(6) an antibody, wherein the heavy chain variable region of the antibody has an amino acid sequence of SEQ ID NO:76 or an amino acid sequence having at least 80%, 90%, 91%, 92%, 93%, 94%, 95%, 96%, 97%, 98%, 99% similarity to SEQ ID NO:76, and the light chain variable region of the antibody has an amino acid sequence of SEQ ID NO:25 or an amino acid sequence having at least 80%, 90%, 91%, 92%, 93%, 94%, 95%, 96%, 97%, 98%, 99% similarity to SEQ ID NO:25;
(7) an antibody, wherein the heavy chain variable region of the antibody has an amino acid sequence of SEQ ID NO:80 or an amino acid sequence having at least 80%, 90%, 91%, 92%, 93%, 94%, 95%, 96%, 97%, 98%, or 99% similarity to SEQ ID NO:80, and the light chain variable region of the antibody has an amino acid sequence of SEQ ID NO:25 or an amino acid sequence having at least 80%, 90%, 91%, 92%, 93%, 94%, 95%, 96%, 97%, 98%, or 99% similarity to SEQ ID NO:25;
(8) an antibody, wherein the heavy chain variable region of the antibody has the amino acid sequence of SEQ ID NO:23 or an amino acid sequence having at least 80%, 90%, 91%, 92%, 93%, 94%, 95%, 96%, 97%, 98%, or 99% similarity to SEQ ID NO:23, and the light chain variable region has the amino acid sequence of SEQ ID NO:78 or an amino acid sequence having at least 80%, 90%, 91%, 92%, 93%, 94%, 95%, 96%, 97%, 98%, or 99% similarity to SEQ ID NO:78;
(9) an antibody, which is a variant of the antibody of any one of (1) to (8), and has the same or similar activity as the antibody of any one of (1) to (8).

In certain embodiments, the antibody is selected from a whole antibody, a scFv, a single domain antibody, a Fab fragment, a Fab' fragment, a Fv fragment, a F(ab')₂ fragment, a Fd fragment, a dAb fragment, a multifunctional antibody or a scFv-Fc antibody, a hybridoma antibody, a chimeric antibody, a humanized antibody, a fully human antibody or a monoclonal antibody.

In certain embodiments, the antibody is a hybridoma antibody selected from the group consisting of:
(1) an antibody, wherein the heavy chain variable region of the antibody has an amino acid sequence of SEQ ID NO:35 or an amino acid sequence having at least 80%, 90%, 91%, 92%, 93%, 94%, 95%, 96%, 97%, 98%, 99% similarity to SEQ ID NO:35, and the light chain variable region of the antibody has an amino acid sequence of SEQ ID NO:37 or an amino acid sequence having at least 80%, 90%, 91%, 92%, 93%, 94%, 95%, 96%, 97%, 98%, 99% similarity to SEQ ID NO:37;
(2) an antibody, wherein the heavy chain variable region of the antibody has an amino acid sequence of SEQ ID NO:39 or an amino acid sequence having at least 80%, 90%, 91%, 92%, 93%, 94%, 95%, 96%, 97%, 98%, 99% similarity to SEQ ID NO:39, and the light chain variable region of the antibody has an amino acid sequence of SEQ ID NO:41 or an amino acid sequence having at least 80%, 90%, 91%, 92%, 93%, 94%, 95%, 96%, 97%, 98%, 99% similarity to SEQ ID NO:41;
(3) an antibody, which is a variant of the antibody of any one of (1) to (2), and has the same or similar activity as the antibody of any one of (1) to (2).

In certain embodiments, the antibody is a fully human antibody selected from the group consisting of:
(1) an antibody, wherein the heavy chain variable region of the antibody has an amino acid sequence of SEQ ID NO:23 or an amino acid sequence having at least 80%, 90%, 91%, 92%, 93%, 94%, 95%, 96%, 97%, 98%, 99% similarity to SEQ ID NO:23, and the light chain variable region of the antibody has an amino acid sequence of SEQ ID NO:25 or an amino acid sequence having at least 80%, 90%, 91%, 92%, 93%, 94%, 95%, 96%, 97%, 98%, 99% similarity to SEQ ID NO:25;
(2) an antibody, wherein the heavy chain variable region of the antibody has an amino acid sequence of SEQ ID NO:27 or an amino acid sequence having at least 80%, 90%, 91%, 92%, 93%, 94%, 95%, 96%, 97%, 98%, 99% similarity to SEQ ID NO:27, and the light chain variable region of the antibody has an amino acid sequence of SEQ ID NO:29 or an amino acid sequence having at least 80%, 90%, 91%, 92%, 93%, 94%, 95%, 96%, 97%, 98%, 99% similarity to SEQ ID NO:29;
(3) an antibody, wherein the heavy chain variable region of the antibody has an amino acid sequence of SEQ ID NO:31 or an amino acid sequence having at least 80%, 90%, 91%, 92%, 93%, 94%, 95%, 96%, 97%, 98%, 99% similarity to SEQ ID NO:31, and the light chain variable region of the antibody has an amino acid sequence of SEQ ID NO:33 or an amino acid sequence having at least 80%, 90%, 91%, 92%, 93%, 94%, 95%, 96%, 97%, 98%, 99% similarity to SEQ ID NO:33;
(4) an antibody, wherein the heavy chain variable region of the antibody has an amino acid sequence of SEQ ID NO:76 or an amino acid sequence having at least 80%, 90%, 91%, 92%, 93%, 94%, 95%, 96%, 97%, 98%, 99% similarity to SEQ ID NO:76, and the light chain variable region of the antibody has an amino acid sequence of SEQ ID NO:25 or an amino acid sequence having at least 80%, 90%, 91%, 92%, 93%, 94%, 95%, 96%, 97%, 98%, 99% similarity to SEQ ID NO:25;
(5) an antibody, wherein the heavy chain variable region of the antibody has an amino acid sequence of SEQ ID NO:80 or an amino acid sequence having at least 80%, 90%, 91%, 92%, 93%, 94%, 95%, 96%, 97%, 98%, 99% similarity to SEQ ID NO:80, and the light chain variable region of the antibody has an amino acid sequence of SEQ ID NO:25 or an amino acid sequence having at least 80%, 90%, 91%, 92%, 93%, 94%, 95%, 96%, 97%, 98%, 99% similarity to SEQ ID NO:25;
(6) an antibody, wherein the heavy chain variable region of the antibody has an amino acid sequence of SEQ ID NO:23 or an amino acid sequence having at least 80%, 90%, 91%, 92%, 93%, 94%, 95%, 96%, 97%, 98%, 99% similarity to SEQ ID NO:23, and the light chain variable region of the antibody has an amino acid sequence of SEQ ID NO:78 or an amino acid sequence having at least 80%, 90%, 91%, 92%, 93%, 94%, 95%, 96%, 97%, 98%, 99% similarity to SEQ ID NO:78;
(7) an antibody, which is a variant of the antibody of any one of (1) to (6), and has the same or similar activity as the antibody of any one of (1) to (6).

In certain embodiments, the antibody is selected from the group consisting of:
(1) an antibody, wherein the heavy chain of the antibody has the amino acid sequence of SEQ ID NO:45 or an amino acid sequence having at least 80%, 90%, 91%, 92%, 93%, 94%, 95%, 96%, 97%, 98%, 99% similarity to SEQ ID NO:45, and the light chain of the antibody has an amino acid sequence of SEQ ID NO:47 or an amino acid sequence having at least 80%, 90%, 91%, 92%, 93%, 94%, 95%, 96%, 97%, 98%, 99% similarity to SEQ ID NO:47;
(2) an antibody, wherein the heavy chain of the antibody has the amino acid sequence of SEQ ID NO:49 or an amino acid sequence having at least 80%, 90%, 91%, 92%, 93%, 94%, 95%, 96%, 97%, 98%, 99% similarity to SEQ ID NO:49, and the light chain of the antibody has an amino acid sequence of SEQ ID NO:51 or an amino acid sequence having at least 80%, 90%, 91%, 92%, 93%, 94%, 95%, 96%, 97%, 98%, 99% similarity to SEQ ID NO:51;
(3) an antibody, wherein the heavy chain of the antibody has the amino acid sequence of SEQ ID NO:53 or an amino acid sequence having at least 80%, 90%, 91%, 92%, 93%, 94%, 95%, 96%, 97%, 98%, 99% similarity to SEQ ID NO:53, and the light chain of the antibody has an amino acid sequence of SEQ ID NO:55 or an amino acid sequence having at least 80%, 90%, 91%, 92%, 93%, 94%, 95%, 96%, 97%, 98%, 99% similarity to SEQ ID NO:55;
(4) an antibody, which is a variant of the antibody of any one of (1) to (3), and has the same or similar activity as the antibody of any one of (1) to (3).

In certain embodiments, any one of the antibodies of the first aspect is a hybridoma antibody, a chimeric antibody, a humanized antibody or a fully human antibody; or the antibody is a monoclonal antibody; or the antibody is a whole antibody, scFv, a single domain antibody, a Fab fragment, a Fab' fragment, a Fv fragment, a F(ab')₂ fragment, a Fd fragment, a dAb fragment, a multifunctional antibody or a scFv-Fc antibody.

In certain embodiments, any one of the antibodies of the first aspect is a hybridoma antibody selected from the group consisting of:
(1) an antibody, wherein the heavy chain variable region of the antibody has the amino acid sequence of SEQ ID NO: 35, and the light chain variable region of the antibody has the amino acid sequence of SEQ ID NO: 37;
(2) an antibody, wherein the heavy chain variable region of the antibody has the amino acid sequence of SEQ ID NO: 39, and the light chain variable region of the antibody has the amino acid sequence of SEQ ID NO: 41.
(3) an antibody, which is a variant of the antibody of any one of (1) to (2), and has the same or similar activity as the antibody of any one of (1) to (2).

In certain embodiments, any one of the antibodies of the first aspect is a fully human antibody selected from the group consisting of:
(1) an antibody, wherein the heavy chain variable region of the antibody has the amino acid sequence of SEQ ID NO: 23, and the light chain variable region of the antibody has the amino acid sequence of SEQ ID NO: 25;
(2) an antibody, wherein the heavy chain variable region of the antibody has the amino acid sequence of SEQ ID NO: 27, and the light chain variable region of the antibody has the amino acid sequence of SEQ ID NO: 29;
(3) an antibody, wherein the heavy chain variable region of the antibody has the amino acid sequence of SEQ ID NO: 31, and the light chain variable region of the antibody has the amino acid sequence of SEQ ID NO: 33;
(4) an antibody, which is a variant of the antibody of any one of (1) to (3), and has the same or similar activity as the antibody of any one of (1) to (3).

In certain embodiments, any one of the antibodies of the first aspect is a fully human antibody selected from the group consisting of:
(1) an antibody, wherein the heavy chain of the antibody has the amino acid sequence of SEQ ID NO: 45, and the light chain of the antibody has the amino acid sequence of SEQ ID NO: 47;
(2) an antibody, wherein the heavy chain of the antibody has the amino acid sequence of SEQ ID NO:49, and the light chain of the antibody has the amino acid sequence of SEQ ID NO:51;
(3) an antibody, wherein the heavy chain of the antibody has the amino acid sequence of SEQ ID NO: 53, and the light chain of the antibody has the amino acid sequence of SEQ ID NO: 55;
(4) an antibody, which is a variant of the antibody of any one of (1) to (3), and has the same or similar activity as the antibody of any one of (1) to (3).

In the second aspect, the present application provides an immunoconjugate, wherein the immunoconjugate comprises any one of the antibodies of the first aspect, and a functional molecule linked thereto.

In the third aspect, the present application provides a chimeric receptor, an extracellular domain of the chimeric receptor comprises any one of the antibodies of the first aspect, and the chimeric receptor comprises: a chimeric antigen receptor (CAR), a chimeric T cell receptor, a T cell antigen coupler (TAC) or a combination thereof.

In certain embodiments, the chimeric receptor is a chimeric antigen receptor (CAR).

In certain embodiments, the CAR comprises any one of the antibodies of the first aspect, a transmembrane region and an intracellular signaling region.

In certain embodiments, the chimeric receptor comprises: any one of the antibodies of the first aspect, a transmembrane region and an intracellular signaling region, connected in sequence.

In certain embodiments, the intracellular signaling region is selected from: sequences of intracellular signaling regions of CD3ζ, FcεRIγ, CD27, CD28, CD137, CD134, MyD88, CD40 or a combination thereof; and/or the transmembrane region comprises the transmembrane region of CD8 or CD28.

In certain embodiments, the transmembrane region of CD8 comprises the amino acid sequence of SEQ ID NO:60.

In certain embodiments, the intracellular signaling region of CD137 comprises the amino acid sequence of SEQ ID NO: 61.

In certain embodiments, the intracellular signaling domain of CD3ζ comprises the amino acid sequence of SEQ ID NO:62.

In certain embodiments, the chimeric receptor comprises: any one of the antibodies of the first aspect, the transmembrane region of CD8/CD28, and CD3ζ; or any one of the antibodies of the first aspect, the transmembrane region of CD8/CD28, the intracellular signaling region of CD137, and CD3ζ; or any one of the antibodies of the first aspect, the transmembrane region of CD8/CD28, the intracellular signaling region of CD28, and CD3ζ; or any one of the antibodies of the first aspect, the transmembrane region of CD8/CD28, the intracellular signaling region of CD28, CD137, and CD3ζ.

In certain embodiments, the chimeric receptor further comprises a hinge region.

In certain embodiments, the hinge region comprises a CD8 hinge region.

In certain embodiments, the CD8 hinge region comprises the amino acid sequence of SEQ ID NO:59.

In certain embodiments, the chimeric receptor further comprises a signal peptide.

In certain embodiments, the signal peptide comprises a CD8 signal peptide.

In certain embodiments, the CD8 signal peptide comprises the amino acid sequence of SEQ ID NO:58.

In certain embodiments, the chimeric receptor comprises an amino acid sequence as shown in any one of SEQ ID NOs: 43, 82, 84 or 86.

In certain embodiments, the amino acid sequence of the chimeric receptor is shown in any one of SEQ ID NOs: 63, 88, 89, and 90.

In the fourth aspect, the present application provides a nucleic acid encoding any one of the antibodies of the first aspect, the immunoconjugate of the second aspect, and the chimeric receptor of the third aspect.

In certain embodiments, the nucleic acid comprises a sequence as shown in any one of SEQ ID NOs: 24, 26, 28, 30, 32, 34, 36, 38, 40, 42, 77, 79 or 81, or a combination thereof.

In certain embodiments, the nucleic acid comprises a sequence as shown in any one of SEQ ID NOs: 46, 48, 50, 52, 54 or 56, or a combination thereof.

In certain embodiments, the nucleic acid comprises a sequence as shown in any one of SEQ ID NOs: 44, 83, 85 or 87.

In the fifth aspect, the present application provides a vector comprising the nucleic acid of the fourth aspect.

In the sixth aspect, the present application provides a virus comprising the expression vector of the fifth aspect.

In the seventh aspect, the present application provides a composition comprising the immunoconjugate of the second aspect and/or the chimeric receptor of the third aspect, wherein the composition is cytotoxic to a cell expressing GPRC5D.

In certain embodiments, the cell expressing GPRC5D is a tumor cell and/or a pathogen cell.

In the eighth aspect, the present application provides a cell comprising the antibody of the first aspect, the immunoconjugate of the second aspect, the chimeric receptor of the third aspect, the nucleic acid of the fourth aspect, and/or the vector of the fifth aspect.

In certain embodiments, the cell is a host cell, which comprises the vector of the fifth aspect or the nucleic acid of the fourth aspect is integrated into its genome.

In certain embodiments, the cell/host cell expresses the chimeric receptor of the third aspect.

In certain embodiments, the cell/host cell is a T cell, a natural killer cell, a natural killer T cell, a NK92 cell, an immune effector cell comes from stem cell, or a combination thereof.

In certain embodiments, the T cell comprised a cytotoxic T lymphocyte, a DNT cell and/or a regulatory T cell.

In certain embodiments, the T cell comes from a natural T cell and/or a T cell induced from a pluripotent stem cell.

In certain embodiments, the T cell is an autologous/allogeneic T cell.

In certain embodiments, the T cell is a primary T cell.

In certain embodiments, the T cell come from a human autologous T cell.

In certain embodiments, the T cell comprises a stem cell-like memory T cell (Tscm cell), a central memory T cell (Tcm), an effector T cell (Tef), a regulatory T cell (Treg), an effector memory T cell (Tern), a γδ T cell, or a combination thereof.

In certain embodiments, the host cell binds to cells expressing GPRC5D and does not significantly bind to cells that do not express GPRC5D.

In certain embodiments, the host cell further carries the coding sequence of an exogenous cytokine; and/or further expresses a chimeric receptor for non GPRC5D targeted; and/or further expresses a chemokine; and/or further expresses a chemokine receptor; and/or further expresses a safety switch.

In certain embodiments, the host cell further carries an exogenous cytokine comprising a coding sequence of IL-7, IL-12, IL-15, IL-18, IL-21, or type I interferon.

In certain embodiments, the host cell further expresses a chemokine comprising CCL19 or CCL21.

In certain embodiments, the host cell further expresses a chemokine receptor comprising CCR2, CCR4, CCR5, CXCR2, CXCR4 or CXCR5.

In certain embodiments, the host cell further expresses a safety switch comprising iCaspase-9, Truancated EGFR or RQR8.

In the ninth aspect, the present application provides a pharmaceutical composition, which comprises the antibody of any one of the first aspect, the immunoconjugate of the second aspect, the chimeric receptor of the third aspect, the nucleic acid of the fourth aspect, the vector of the fifth aspect, the virus of the sixth aspect, the composition of the seventh aspect and/or the cell of the eighth aspect, and a pharmaceutically acceptable adjuvant.

In the tenth aspect, the present application provides a combination therapy, wherein any one of the antibodies of the first aspect, the immunoconjugate of the second aspect, the chimeric receptor of the third aspect, the composition of the seventh aspect, the cell of the eighth aspect, and the pharmaceutical composition of the ninth aspect are administered in combination with an agent that enhances their functions, preferably in combination with a chemotherapeutic agent; and/or in combination with an agent that improve one or more side effects associated therewith; and/or in combination with a host cell expressing chimeric antigen receptors other than those targeting GPRC5D.

In the eleventh aspect, the present application provides a method for preparing any one of the antibodies of the first aspect, the immunoconjugate of the second aspect, the chimeric receptor of the third aspect, the composition of the seventh aspect, and the pharmaceutical composition of the ninth aspect, the method comprises culturing the host cell of the eighth aspect under conditions suitable for expressing the antibody, immunoconjugate, chimeric receptor, and isolating the antibody, immunoconjugate, chimeric receptor expressed by the host cell, composition and/or pharmaceutical composition.

In the twelfth aspect, the present application provides a kit, comprising any one of the antibodies of the first aspect, the immunoconjugate of the second aspect, the chimeric receptor of the third aspect, the nucleic acid of the fourth aspect, the vector of the fifth aspect, the virus of the sixth aspect, the composition of the seventh aspect, the cell of the eighth aspect and/or the pharmaceutical composition of the ninth aspect.

In certain embodiments, the kit comprises:
a container, and the pharmaceutical composition of the ninth aspect contained in the container; or
a container, and any one selected from the below group contained in the container: any one of the antibodies of the first aspect or the nucleic acid encoding the antibody; or the immunoconjugate of the second aspect or the nucleic acid encoding the immunoconjugate; or the chimeric receptor of the third aspect or the nucleic acid encoding the chimeric receptor; or the host cell of the eighth aspect.

In the thirteenth aspect, the present application provides a method for treating/diagnosing a disease, comprising administering to a subject in need thereof an effective amount of any one of the antibodies of the first aspect, the immunoconjugate of the second aspect, the cell of the eighth aspect, the pharmaceutical composition of the ninth aspect, or the kit of the twelfth aspect.

In a specific embodiment, the disease is selected from the group consisting of: inflammatory disorders, infections, autoimmune diseases, and tumors;

In a specific embodiment, the subject is a human;

In a specific embodiment, wherein the host cell is an autologous or allogeneic T cell to the subject.

In the fourteenth aspect, the present application provides use of any one of the antibodies of the first aspect, or the immunoconjugate of the second aspect, or the host cell of the eighth aspect, or the pharmaceutical composition of the ninth aspect, or the kit of the twelfth aspect in the preparation of a medicament for treating/diagnosing a disease, characterized in that the disease comprises expression of GPRC5D; preferably, the disease is selected from the group consisting of: inflammatory disorders, infections, autoimmune diseases and tumors.

It should be understood that within the scope of the present application, the above-mentioned technical features of the present application and the technical features specifically described below (such as Examples) can be combined with each other to form new or preferred technical solutions. Due to limited space, they will not be elaborated one by one here.

### BRIEF DESCRIPTION OF THE DRAWINGS

Fig. 1 shows the EC50 of the binding of hybridoma antibodies AB1 and AB2 to CHOK1-huGPRC5D cells;
Fig. 2 shows that hybridoma antibodies AB1 and AB2 both significantly bind to human GPRC5D and mouse GPRC5D;
Fig. 3 shows that recombinant hybridoma antibodies AB1 (scFv-mFc) and AB2 (scFv-mFc) specifically bind to a cell line expressing human GPRC5D;
Fig. 4 shows that antibodies AB3, AB4, and AB5 all specifically bind to human GPRC5D;
Fig. 5 shows that antibodies AB3, AB4, and AB5 all specifically bind to 293T cells overexpressing human GPRC5D;
Fig. 6 shows the EC50 of binding of antibodies AB3, AB4, and AB5 to human GPRC5D;
Fig. 7 shows that antibodies AB3, AB4, and AB5 all significantly bind to cells expressing human GPRC5D (CHOK1-huGPRC5D, 293T-huGPRC5D, MM.1S, and NCI-H929), but do not bind to cells not expressing GPRC5D (CHOK1, 293T, and Daudi);
Fig. 8 shows the EC50 of binding of antibodies AB3, AB4, and AB5 to CHOK1-huGPRC5D cells;
Fig. 9 shows the EC50 of binding of antibodies AB3, AB4, and AB5 to MM.1S cells;
Fig. 10 shows the binding of antibody AB3 to CHOK1-muGPRCSD cells;
Fig. 11 shows the in vitro killing effect of AB3-BBZ CAR T cells on target cells;
Fig. 12 shows the effect of AB3-BBZ CAR T cells and 18-BBZ CAR T cells in inhibiting tumor growth in tumor-bearing mice;
Fig. 13 shows the changes in body weight of tumor-bearing mice treated with AB3-BBZ CAR T cells and 18-BBZ CAR T cells;
Fig. 14 shows the binding results of antibodies AB6, AB7 and AB8 to human GPRC5D;
Fig. 15 shows the detection results of antibodies AB3, AB6, AB7 and AB8 binding to GPRC5D cell lines of different species;
Fig. 16 shows the detection results of EC50 of antibodies AB3, AB6, AB7 and AB8 binding to 293T-huGPRC5D or MM.1S cells;
Fig. 17 shows the test results of affinity betweenantibodies AB3 or AB7 and Bio-GPRC5D;
Fig. 18 shows the in vitro killing effect of AB3-BBZ, AB6-BBZ, AB7-BBZ, or AB8-BBZ CAR T cells on target cells.

### DETAIL DESCRIPTION

After in-depth research and screening, the applicant obtained hybridoma antibodies and fully human antibodies that specifically recognize GPRC5D. The antibodies of the present application can be used to prepare targeted anti-tumor agents and agents for diagnosing tumors.

### Terms

Unless otherwise defined, all technical and scientific terms used herein have the same meanings as commonly understood by those skilled in the arts of gene therapy, biochemistry, genetics and molecular biology. All methods and materials similar or equivalent to those described herein can be used in the practice or testing of the present application, wherein appropriate methods and materials are described herein. All publications, patent applications, patents, and other references mentioned herein are incorporated by reference in their entirety. In case of conflict, the present specification, comprising definitions, will control. In addition, unless otherwise specified, the materials, methods, and examples are illustrative only and not intended to be limiting.

The practice of the present application will employ, unless otherwise specified, conventional techniques of cell biology, cell culture, molecular biology, transgenic biology, microbiology, recombinant DNA, and immunology, which are within the skill of the art. Such techniques are fully explained in the literatures. See, e.g., Current Protocols in Molecular Biology (Frederick M. AUSUBEL, 2000, Wiley and son Inc, Library of Congress, USA); Molecular Cloning: A Laboratory Manual, Third Edition, (Sambrook et al, 2001, Cold Spring Harbor, New York: Cold Spring Harbor Laboratory Press); Oligonucleotide Synthesis (M. J. Gaited., 1984); Mullis et al. U.S. Pat. No. 4,683,195; Nucleic Acid Hybridization (B. D. Harries & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), especially Vols. 154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Hand book Of Experimental Immunology, Volumes I-IV (D. M. Weir and C. C. Blackwell, eds., 1986); and Manipulating the Mouse Embryo (Cold Spring Harbor Laboratory Press, Cold Spring Harbor, N.Y., 1986).

In the present disclosure, various aspects of the claimed subject matters are presented in a range format. It should be understood that the description in range format is merely for convenience and brevity and should not be construed as an inflexible limitation on the scope of the claimed subject matters. Accordingly, the description of a range should be considered to have specifically disclosed all the possible subranges as well as individual numerical values within that range. For example, In the case of providing a range of values, it should be understood that each intervening value between the upper and lower limits of that range and any other stated value or intermediate value in the stated range are included within the claimed subject matter, and the upper and lower limits of the stated range also fall within the scope of the claimed subject matter. The upper and lower limits of these smaller ranges may independently be included in the smaller ranges and are also within the scope of the claimed subject matter, unless the upper and lower limits of the stated range are explicitly excluded. Where the stated range includes one or two of the limits, the claimed subject matter also includes ranges excluding one or two of those limits. This applies regardless of the width of the range.

The term "about" refers to the usual error range for each value that is readily known to one skilled in the art. Reference herein to "about" a value or parameter includes (and describes) embodiments directed to that value or parameter per se. For example, description of "about X" includes description of "X." For example, "about" or "comprising" may mean within 1 or more than 1 of a practical standard deviation in the art. Alternatively, "about" or "comprising" may mean a range of up to 10% (i.e., ±10%). For example, about 5 µM may include any number between 4.5 µM and 5.5 µM. When specific values or compositions are provided in the application and claim ranges, unless otherwise specified, "about" or "comprising" should be assumed to be within an acceptable error range for the specific value or composition.

Unless otherwise indicated, any concentration range, percentage range, ratio range or integer range described herein should be understood to include any integer within the range, and, where appropriate, fractional values thereof (e.g., tenths and hundredths of an integer).

In order to make it easier to understand the present application, some terms are first defined.

The term "GPRC5D" refers to group 5 classified into G protein coupled receptor family C, and is one of human GPCR proteins newly discovered through homology search of EST database using amino acid sequences of a series of human GPCRs. The protein has been registered under Genbank accession numbers: AF209923, NM_018654 and NP_0611124. GPRC5D is an orphan receptor with no known ligands or functions in humans and human cancers. For example, the full-length amino acid sequence of human GPRC5D is shown as SEQ ID NO:65, and the full-length amino acid sequence of mouse GPRC5D is shown as SEQ ID NO:66.

The terms "polypeptide," "peptide," and "protein" are used interchangeably to refer to polymers of amino acids of any length. The polymers may be linear, cyclic or branched, it may comprise modified amino acids, particularly conservatively modified amino acids, and it may be interrupted by non-amino acids. The term also includes modified amino acid polymers, e.g. those that have been modified by sulfation, glycosylation, lipidation, acetylation, phosphorylation, iodination, methylation, oxidation, protein hydrolysis processing, prenylation, racemization, selenoylation, transfer-RNA-mediated amino addition such as arginylation, ubiquitination, or any other manipulation such as conjugation with a labeling composition, etc. As used herein, the term "amino acid" refers to natural and/or unnatural or synthetic amino acids, comprising glycine and the D or L optical isomers, as well as amino acid analogs and peptidomimetics. A polypeptide or amino acid sequence "derived from" a specified protein refers to the source of the polypeptide. The term also includes polypeptides expressed by the designated nucleic acid sequence.

The term "antibody" is used herein in the broadest sense and includes various antibody structures, including but not limited to monoclonal antibodies, polyclonal antibodies, multispecific antibodies (e.g., bispecific antibodies), and antibody fragments, as long as they exhibit the desired antigen-binding activity.

"Antibody fragments" refer to molecules distinct from intact antibodies that comprise a portion of an intact antibody that binds to the antigen to which the intact antibody binds. Examples of antibody fragments include, but are not limited to: (i) Fab fragments consisting of the VL, VH, CL and CH1 domains, comprising Fab' and Fab'-SH, (ii) Fd fragments consisting of the VH and CH1 domains, (iii) Fv fragments consisting of the VL and VH domains of a single antibody; (iv) dAb fragments consisting of a single variable region (Ward et al., 1989, Nature 341:544-546); (v) F(ab')₂ fragments, bivalent fragments comprising two linked Fab fragments; (vi) binding sites of single-chain Fv molecule antigens; (vii) bispecific single-chain Fv dimers (PCT/US92/09965); (viii) "dibodies" or "tribodies", multivalent or multispecific fragments constructed by genetic fusion; and (ix) scFv genetically fused to the same or different antibodies.

The "class" of an antibody refers to the type of constant domain or constant region possessed by its heavy chain. There are five major classes of antibodies: IgA, IgD, IgE, IgG and IgM, and some of these can be further divided into subclasses (allotypes), for example, IgGl, IgG2, IgG3, IgG4, IgAl and IgA2. The heavy-chain constant domains that correspond to the different classes of immunoglobulins are called α, δ, ε, γ, and µ, respectively.

The term "variable region or variable domain" refers to the domain of an antibody heavy or light chain that is involved in antibody antigen binding. The heavy and light chain variable domains (VH and VL, respectively) of natural antibodies generally have similar structures, with each domain comprising four conserved FRs and three CDRs. (See, e.g., Kindt et al., Kuby Immunology, 6th ed., W. H. Freeman & Co., p. 91 (2007)). A single VH or VL domain may be sufficient to confer antigen-binding specificity. Furthermore, antibodies that bind a particular antigen can be isolated by screening a library of complementary VL or VH domains, respectively, using a VH or VL domain from an antibody that binds the antigen. See, e.g., Portolano et al., J. Immunol. 150:880-887 (1993); Clarkson et al., Nature 352:624-628 (1991).

The term "hypervariable region" or "complementarity determining region" or "CDR" refers to the regions of an antibody variable region that are hypervariable in sequence, and/or form structurally defined loops ("hypervariable loops"), and/or contain residues that contact the antigen ("antigenic contacts"). In certain embodiments, the CDRs may be identified by a numbering system selected from the group consisting of: Kabat, Chothia, IMGT, Gelfand, Aho, and Martin. For example, it can be determined by the Kabat numbering system. For example, an antibody may comprise six CDRs: three in VH (HCDR1, HCDR2, HCDR3) and three in VL (LCDR1, LCDR2, LCDR3).

The terms "Fc region" or "Fc" are used to define the C-terminal region of an immunoglobulin heavy chain that contains at least a portion of the constant region. The term comprises native sequence Fc regions and variant Fc regions.

"Framework (FR)" refers to residues in the variable domain distinct from those in the hypervariable region (CDR). FRs in a variable domain generally consists of four FR domains: FR1, FR2, FR3 and FR4. Thus, in VH (or VL), sequences of CDRs and FRs typically appear in the following order: FR1-HCDR1 (LCDR1)-FR2-HCDR2 (LCDR2)-FR3-HCDR3 (LCDR3)-FR4.

Unless otherwise indicated, in the present application, CDR residues and other residues in the variable domains (e.g., FR residues) are numbered according to Kabat et al., supra.

The term "natural antibody" refers to naturally occurring immunoglobulin molecules having a variety of structures. For example, natural IgG antibodies are heterotetrameric glycoproteins of approximately 150,000 daltons, composed of two identical light chains and two identical heavy chains bonded by disulfide bonds. From N-terminus to C-terminus, each heavy chain has a variable region (VH) (also called a variable heavy chain domain or a heavy chain variable domain), followed by three constant domains (CH1, CH2 and CH3). Similarly, from N-terminus to C-terminus, each light chain has a variable region (VL) (also called a variable light chain domain or a light chain variable domain), followed by a light chain constant (CL) domain. The light chains of antibodies can be assigned to one of two types, called kappa (κ) and lambda (λ), based on the amino acid sequences of their constant domains.

The terms "whole antibody", "full length antibody" and "intact antibody" are used interchangeably and refer to a complete full-length antibody having a structure substantially similar to that of a natural antibody or having a heavy chain containing an Fc region as defined herein or comprising an antigen-binding region.

The term "single domain antibody (sdAb)" refers to a type of antibody that lacks the antibody light chain and only has the heavy chain variable region. Because of its small molecular weight, it is also called a nanobody.

The term "single-domain antibody" refers to an antibody fragments comprising all or part of the heavy chain variable domain or all or part of the light chain variable domain of an antibody. In certain embodiments, a single domain antibody is a human single-domain antibody (Domantis, Inc., Waltham, MA; see, e.g., U.S. Pat. No. 6,248,516).

The term "monoclonal antibody" refers to an antibody obtained from a population of substantially homogeneous antibodies, i.e., the individual antibodies comprising the population are identical and/or bind to the same epitope, except for possible variant antibodies, e.g., containing naturally occurring mutations or arising during production of a monoclonal antibody preparation, such variants generally being present in minor amounts. In contrast to polyclonal antibody preparations (which typically comprise different antibodies directed against different determinants (epitopes)), each monoclonal antibody of a monoclonal antibody preparation is directed against a single determinant on the antigen. Thus, the designation "monoclonal" indicates the character of the antibody as being obtained from a substantially homogeneous population of antibodies, and is not to be construed as requiring production of the antibody by any particular method. For example, they can be prepared by a variety of techniques including, but not limited to the hybridoma method, recombinant DNA methods, phage-display methods, and methods utilizing transgenic animals containing all or part of the human immunoglobulin loci.

Exemplarily, the monoclonal antibodies of the present application can be produced by the hybridoma method, where hybridomas can be formed by isolating stimulated immune cells, such as those from the spleen of a vaccinated animal. These cells (such as myeloma cells or transformed cells) can then be fused with immortalized cells, which are capable of replicating indefinitely in cell culture, thereby producing an immortal cell line secreting immunoglobulin. The immortalized cell lines utilized are selected for their lack of enzymes necessary for utilization of certain nutrients. Many such cell lines, such as myelomas, are known to those skilled in the art and comprise, for example, thymidine kinase (TK) or hypoxanthine-guanine phosphoribosyltransferase (HGPRT). These defects allow selection of fused cells based on their ability to grow on, for example, hypoxanthine aminopterin thymidine medium (HAT).

The term "chimeric antibody" refers to an antibody in which a portion of the heavy chain and/or light chain is derived from a particular source or species, while the remainder of the heavy chain and/or light chain is derived from a different source or species. In certain embodiments, a chimeric antibody comprises a non-human variable region (e.g., a variable regions derived from a mouse, rat, hamster, rabbit, or a non-human primate such as a monkey) and a human constant region. In additional embodiments, a chimeric antibody is a "class switched" antibody, in which the class or subclass has been changed from that of the parent antibody. Chimeric antibodies comprise antigen-binding fragments thereof. In certain embodiments, a chimeric antibody is a "humanized antibody."

The term "humanized" is used for non-human antibodies, e.g. rodent or primate antibodies, and is a hybrid immunoglobulin, immunoglobulin chain or fragment thereof that contains minimal sequence derived from a non-human immunoglobulin. A "humanized antibody" refers to a chimeric antibody comprising amino acid residues from non-human CDRs and amino acid residues from human FRs. In certain embodiments, a humanized antibody will comprise at least one (typically two) of the substantially all variable domains, in which all or substantially all CDRs correspond to CDRs of a non-human antibody, and all or substantially all FRs correspond to FRs of a human antibody. A humanized antibody optionally may comprise at least a portion of an antibody constant region derived from a human antibody.

In some embodiments, a "humanized antibody" may comprise mutations, e.g. mutations introduced by random or site-directed mutagenesis in vitro or by somatic mutation in vivo.

The term "fully human antibody" is an antibody having an amino acid sequence corresponding to that of an antibody produced by a human or human cell, or derived from a non-human source utilizing a human antibody repertoire or other human antibody encoding sequences. The definition of fully human antibody specifically excludes humanized antibodies that contain non-human antigen-binding residues. In certain embodiments, the antibodies provided herein are "fully human antibodies" that are generated by phage display technology.

Antibodies of the present application can be isolated by screening combinatorial libraries for antibodies possessing one or more of the desired activities. For example, a variety of methods are known in the art for generating phage display libraries and screening such libraries for antibodies with desired binding properties. Such methods have been reviewed, for example, in Hoogenboom et al., Methods in Molecular Biology 178: 1-37 (O'Brien et al., Human Press, Totowa, NJ, 2001) and further described, for example, in McCafferty et al., Nature 348: 552-554; Clackson et al., Nature 352: 624-628 (1991); Marks et al., J. Mol. Biol. 222:581-597 (1992); Marks, Meth. Mol. Biol., 248:161-175 (Lo, ed., Human Press, Totowa, NJ, 2003); Sidhu et al., J. Mol. Biol. 338(2):299-310(2004); Lee et al., J. Mol. Biol. 340(5):1073-1093(2004); Fellouse, Proc. Natl. Acad. Sci. USA101(34):12467-12472(2004); Lee et al., J. Immunol. Methods 284(1-2):119-132(2004).

In certain phage display methods, the VH and VL gene libraries are cloned separately by polymerase chain reaction (PCR) and randomly recombined in a phage library, which can then be screened for phage binding to the antigen, as described in Winter et al., Ann. Rev. Immunol. 12:433-455 (1994). Phage typically display antibody fragments as single-chain Fv (scFv) fragments or Fab fragments. Libraries from immunized sources provide high affinity antibodies to the immunogen without the need to construct hybridomas. Alternatively, the naive libraries (e.g., from humans) can be cloned to provide a single source of antibodies to a variety of non-self antigens as well as self antigens without the need for any immunization, as described in Griffiths et al., EMBO J, 12:725-734 (1993). Finally, naive libraries can also be prepared synthetically by cloning unrearranged V-gene segments from stem cells and using PCR primers containing random sequences to encode the hypervariable CDR3 regions and achieving rearrangement in vitro, as described in Hoogenboom, J. Mol. Biol. 227:381-388 (1992).

As used herein, antibodies or antibody fragments isolated from a fully human antibody library are considered fully human antibodies or fully human antibody fragments.

In certain embodiments, provided herein are amino acid sequence variants of an antibody. The term "parent antibody" refers to the antibody provided in the present application or an antibody obtained after mutation or affinity maturation of the antibody provided in the present application. The parent antibody may be a naturally occurring antibody, or a variant or engineered version of a naturally occurring antibody. The parent antibody may refer to the antibody itself, a composition comprising the parent antibody, or the amino acid sequence encoded thereof.

The term "affinity matured" antibody refers to an antibody with one or more changes in one or more hypervariable regions (CDRs) compared to a parent antibody, these changes result in an improvement in the affinity of the antibody for antigen.

The term "variant" refers to a polypeptide having substantially the same amino acid sequence as the sequence of an antibody provided herein, or having one or more activities encoded by substantially the same nucleotide sequence. The variant has the same or similar activity as the antibody provided in the examples of the present application. For example, the variant can be a variant antibody or antibody variant based on the amino acid sequence of an antibody provided herein.

The term "variant antibody" or "antibody variant" comprises antibody sequences that differ from the sequence of a parent antibody by virtue of at least one amino acid modification compared to the parent. The variant antibody sequences herein preferably have at least about 80%, 81%, 82%, 83%, 84%, 85%, 86%, 87%, 88%, 89%, 90%, 91%, 92%, 93%, 94%, 95%, 96%, 97%, 98%, 99% amino acid sequence identity (similarity) to the parent antibody sequence. An antibody variant may refer to the antibody itself, or may refer to a composition comprising the antibody variant. Amino acid sequence variants of an antibody can be prepared by introducing appropriate modifications into the nucleotide sequence encoding the antibody or by peptide synthesis. The term "amino acid modification" comprises amino acid substitution, addition and/or deletion. "Amino acid substitution" and "amino acid replacement" mean replacing the amino acid at a specific position in the parent polypeptide sequence with another amino acid. "Amino acid insertion" means adding an amino acid at a specific position in the parent polypeptide sequence. "Amino acid deletion" means removing the amino acid at a specific position in the parent polypeptide sequence. Any combination of deletion, insertion, and substitution can be made to arrive at the final construct, provided that the final construct possesses the desired characteristics, e.g., antigen binding.

The term "modification" refers to a change in the state or structure of the protein or polypeptide of the present application. The ways of modifications can be chemical, structural or functional.

The term "conservative modification" or "conservative sequence modification" means amino acid modifications that do not significantly affect or change the binding characteristics of the antibody containing the amino acid sequence. Such conservative modifications comprise amino acid substitutions, insertions, and deletions. Modifications can be introduced into the antibodies of the present application by standard techniques known in the art, e.g. site-directed mutagenesis, PCR-mediated mutagenesis. Families of amino acid residues having similar side chains have been defined in the art and are shown in Table 1.

**Table 1: Families of amino acid residues having similar side chains**

| | |
|---|---|
| Amino acids containing alkaline side chains | Lys(K), Arg(R), His(H) |
| Amino acids containing acidic side chains | Asp(D), Glu(E) |
| Amino acids containing polar neutral side chains | Asn(N), Ser(S), Thr(T), Tyr(Y), Cys(C), Trp(W), Met(M), Gln(Q) |
| Amino acids containing non-polar side chains | Gly(G), Ala(A), Val(V), Leu(L), Ile(I), Pro(P), Phe(F) |
| Amino acids containing β-branched side chains | Thr(T), Val(V), Ile(I) |
| Amino acids containing aromatic side chains | Tyr(Y), Phe(F), Trp(W) |

Thus, one or more amino acid residues in the CDR region or in the framework region of the antibody of the present application can be replaced with other amino acid residues of the same side chain family, and the altered antibody (variant antibody) can be tested for function retained.

Non-conservative substitutions entail replacing a member of one of these groups with a member of another group.

One substitutional variant involves substituting one or more residues in the hypervariable region of a parent antibody (e.g., a humanized or human antibody). Typically, the resulting variants selected for further study will have changes (e.g., improvements) in certain biological properties (e.g., increased affinity, reduced immunogenicity) relative to the parent antibody and/or will substantially retain certain biological properties of the parent antibody. An exemplary substitutional variant is an affinity matured antibody, which can be routinely prepared, for example, using phage display-based affinity maturation techniques such as those described herein. Briefly, one or more CDR residues are mutated, the variant antibodies are displayed on phages and screened for a particular biological activity (e.g., binding affinity).

Changes (e.g., substitutions) can be made in the CDR regions, for example, to improve antibody affinity. Such changes can be made in CDR "hot spots," i.e., residues encoded by codons that are mutated at high frequency during the somatic maturation process (see, e.g., Chowdhury, Methods Mol. Biol. 207: 179-196 (2008)), and/or residues removing antigen, and test the binding affinity of the VH or VL of the resulting variant. Affinity maturation by construction of secondary libraries and reselection therefrom has been described, for example, in Hoogenboom et al., Methods in Molecular Biology 178: 1-37 (O'Brien et al., ed., Human press, Totowa, NJ, (2001)). In some embodiments of affinity maturation, diversity is introduced into the variable genes selected for maturation by any of a variety of methods (e.g., error-prone PCR, chain shuffling, or oligonucleotide-directed mutagenesis). A secondary library is then generated. The library is then screened to identify any antibody variants having the desired affinity. Another method to introduce diversity involves CDR-directed approach, wherein several CDR residues (e.g., 4-6 residues at a time) are randomized.

In certain embodiments, substitutions, insertions, or deletions may occur within one or more CDRs as long as such changes do not significantly reduce the ability of the antibody to bind antigen. For example, conservative changes (e.g., conservative modifications described herein) that do not significantly reduce binding affinity can be made in the CDRs. Such changes may, for example, be outside of the residues in the CDR that contact the antigen. In certain embodiments of the variant VH and VL sequences provided above, each CDR is unchanged, or contains no more than one, two or three amino acid substitutions.

The terms "anti-GPRC5D antibody", "antibody binding to GPRC5D", "GPRC5D antibody", "antibody that recognizes GPRC5D" refer to antibodies that can bind to GPRC5D with sufficient affinity, and the antibodies can be used as diagnostic agents and/or therapeutic agents for targeting GPRC5D. In one embodiment, the extent of binding of an anti-GPRC5D antibody to an unrelated, non-GPRC5D protein is less than about 10% of the extent of binding of the antibody to GPRC5D as determined by enzyme-linked immunosorbent assay (ELISA). In certain embodiments, an anti-GPRC5D antibody binds to an epitope of GPRC5D that is conserved between GPRC5D derived from different species.

The term "chimeric T cell receptor" comprises recombinant polypeptides derived from various polypeptides that make up the TCR, which are capable of binding to surface antigens on target cells and interacting with other polypeptides of the complete TCR complex, usually co-localized on the surface of T cells. Chimeric T cell receptor is composed of a TCR subunit and an antigen binding domain composed of a human or humanized antibody domain, wherein the TCR subunit comprises at least a portion of the TCR extracellular domain, the transmembrane domain, and the stimulatory domain of the intracellular signaling domain of the TCR intracellular domain; the TCR subunit and the antibody domain are operatively connected, wherein the extracellular, transmembrane, and intracellular signaling domains of the TCR subunit are derived from CD3ε or CD3γ, and the chimeric T cell receptor is integrated into the TCR expressed on the T cell.

The term "T cell antigen coupler (TAC)" comprises three functional domains: 1. antigen binding domain, comprising single-chain antibody, designed ankyrin repeat protein (DARPin) or other targeting groups; 2. extracellular domain, a single-chain antibody that binds to CD3, thereby bringing the TAC receptor into close proximity with the TCR receptor; 3. intracellular region of the co-receptor of transmembrane region and CD4, wherein the intracellular region is connected to protein kinase LCK, catalyzing the phosphorylation of immunoreceptor tyrosine activation motifs (ITAMs) of the TCR complex as the initial step of T cell activation.

The term "chimeric antigen receptor" (CAR) comprises an extracellular antigen binding domain, a transmembrane domain, and an intracellular signaling domain. The intracellular signaling domain can comprise a functional signaling domain of a stimulatory molecule (also referred to as a stimulating molecules, a primary signaling molecule) and/or a co-stimulatory molecule. For example, the stimulatory molecule can be a zeta chain that binds to a T cell receptor complex; for example, the cytoplasmic signaling domain further comprises a functional signaling domain of one or more co-stimulatory molecules, e.g. 4-1BB (i.e., CD137), CD27, and/or CD28.

In the present application, on the one hand, CAR comprises a chimeric fusion protein comprising an extracellular antigen recognition domain, a transmembrane domain, and an intracellular signaling domain, wherein the intracellular signaling domain comprises a functional signaling domain derived from a stimulatory molecule. In one aspect, the CAR comprises a chimeric fusion protein, the chimeric fusion protein comprises an extracellular antigen recognition domain, a transmembrane domain, and an intracellular signaling domain, wherein the intracellular signaling domain comprises a functional signaling domain derived from a co-stimulatory molecule and a functional signaling domain derived from a stimulatory molecule. In one aspect, the CAR comprises a chimeric fusion protein, the chimeric fusion protein comprises an extracellular antigen recognition domain, a transmembrane domain, and an intracellular signaling domain, wherein the intracellular signaling domain comprises at least two functional signaling domains derived from one or more co-stimulatory molecules and a functional signaling domain derived from a stimulatory molecule. In one aspect, the CAR comprises an optional leader sequence at the amino acid (ND-terminus) of the CAR fusion protein. In one aspect, the CAR further comprises a leader sequence at the N-terminus of the extracellular antigen recognition domain, wherein the leader sequence is optionally cleaved from the antigen recognition domain (e.g., scFv) during cellular processing and localization to the cell membrane of the CAR.

The term "primary signaling molecule" or "stimulatory molecule" regulates initial activation of TCR complex in a stimulatory manner. Typically, the primary signal is initiated by, for example, the binding of a TCR/CD3 complex to a peptide-loaded MHC molecule, thereby mediating a T cell response (including but not limited to, proliferation, activation, differentiation, etc.). Primary signaling molecules that act in a stimulatory manner may comprise immunoreceptor tyrosine activation motifs or ITAM signaling motifs. Examples of functional signaling domains (primary signaling domains) of primary signaling molecules comprising ITAMs that are particularly useful in the present application include, but are not limited to, sequences derived from CD3ζ, FcRγ, FcRβ, CD3γ, CD3δ, CD3ε, CD5, CD22, CD79a, CD79b, CD278 (also known as "ICOS") and CD66d. In a specific example of the CAR of the present application, the intracellular signaling domain in any one or more of the CARs of the present application comprises an intracellular signaling sequence, e.g. the primary signaling domain of CD3ζ.

The term "costimulatory signaling domain" generally refers to an intracellular domain of a co-stimulatory molecule that is capable of binding to a cell stimulatory signaling molecule (e.g. TCR/CD3) to cause T cell proliferation and/or upregulation or downregulation of signaling of key molecules. A co-stimulatory molecule is typically a cognate binding partner on a T cell that specifically binds a co-stimulatory ligand, thereby mediating a co-stimulatory response of the T cell, e.g., but not limited to, proliferation. Co-stimulatory molecules are cell surface molecules or ligands thereof required for effective immune response that are not antigen receptors. Co-stimulatory molecules include, but are not limited to, MHC class I molecules, BTLA and Toll ligand receptors, as well as OX40, CD2, CD27, CD28, CDS, ICAM-1, LFA-1 (CD11a/CD18) and 4-1BB (CD137).

The term "CD3ζ (also known as CD3 zeta)" is defined as the protein provided by GenBan Accession No. BAG36664.1, or equivalent residues from non-human species e.g. mouse, rodent, monkey, ape, and the like. The "CD3ζ domain" is defined as amino acid residues from the cytoplasmic domain of the ζ chain that are sufficient to functionally transmit the initial signals required for T cell activation. In one aspect, the cytoplasmic domain of ξ comprises residues 52 to 164 of GenBan Accession No. BAG36664.1, its functional ortholog-the equivalent residues from a non-human species e.g. mouse, rodent, monkey, ape, etc.

### DETAILED DESCRIPTION OF THE INVENTION

### Antibody

Described herein are the use of conventional hybridoma antibody preparation technology in the art to obtain hybridoma antibodies by immunizing mice, as well as construction of recombinant hybridoma antibodies (scFv-Fc). Also described are antigen-binding proteins with Fab (antigen-binding fragment)-based antigen-binding regions, including antibody Fab. Human GPRC5D antigen (Kactus Biosystems) was used, and Fab was selected from an all human natural Fab phage library. These molecules display precise specificity. For example, the antibody only recognizes GPRC5D, and 293T cells, CHO-K1 cells, MM.1S cells, and NCI-H929 cells that overexpress GPRC5D, but does not recognize cells that do not express GPRC5D. If not otherwise specified in the present application, GPRC5D herein refers to human GPRC5D or mouse GPRC5D.

In some embodiments, the present application comprises antibodies having a Fab sequence fused to one or more heavy chain constant regions to form an antibody having a human immunoglobulin Fc region to produce a bivalent protein, thereby increasing the overall affinity and stability of the antibody. In addition, the Fc portion allows direct conjugation of other molecules (including but not limited to fluorescent dyes, cytotoxins, radioisotopes, etc.) to the antibodies for use, for example, in antigen quantification studies, to immobilize antibodies for affinity test, for targeted delivery of therapeutic agents, testing of Fc-mediated cytotoxicity using immune effector cells, and many other applications.

The results presented herein highlight the specificity, sensitivity and utility of the antibodies of the present application in targeting GPRC5D.

The antibodies or antibody fragments of the present application are used to identify and select antigen-binding fragments (Fab) based on using of phage display, the amino acid sequences of the antigen-binding fragments confer specificity to the antibodies or antibody fragments for GPRC5D and form the basis of all antigen-binding proteins of the present disclosure. Therefore, the Fab can be used to design a series of different "antibodies or antibody fragments", including, for example, full-length antibodies, fragments thereof such as F(ab')₂, fusion proteins, multivalent antibodies, scFv-Fc antibodies, i.e., antibodies with more than one specificity for the same antigen or different antigens, for example, bispecific T cell-binding antibodies (BiTEs), triabodies, etc. (see Cuesta et al., Multivalent antibodies: when design surpasses evolution, Trends in Biotechnology 28: 355-362, 2010).

In specific embodiments, the present application provides full-length antibodies, whose heavy chains and light chains can be full-length (for example, the antibody can comprise at least one, preferably two complete heavy chains, and at least one, preferably two complete light chains) or can comprise an antigen binding portion (Fab, F(ab')₂, Fv or scFv). In other embodiments, the antibody heavy chain constant region is selected from the group consisting of, for example, IgGl, IgG2, IgG3, IgG4, IgM, IgAl, IgA2, IgD, and IgE. The choice of antibody type will depend on functions of the immune effector that the antibody is designed to elicit. When constructing recombinant immunoglobulins, appropriate amino acid sequences for constant regions of various immunoglobulin isotypes and methods for producing a wide variety of antibodies are known to those skilled in the art.

The present application provides an antibody that recognizes GPRC5D, which comprises at least one CDR in a heavy chain variable region, wherein the heavy chain variable region comprises an amino acid sequence as shown in any one of SEQ ID NOs: 23, 27, 31, 35, 39, 76 or 80 or a variant thereof, or comprises an amino acid sequence having at least 80% similarity to any one of the above sequences; and/or it comprises at least one CDR in a light chain variable region, wherein the light chain variable region comprises an amino acid sequence as shown in any one of SEQ ID NOs: 25, 29, 33, 37, 41 or 78 or a variant thereof, or comprises an amino acid sequence having at least 80% similarity to any one of the above sequences. For example, the heavy chain variable region comprises an amino acid sequence as shown in any one of SEQ ID NOs: 23, 27, 31, 35 or 39 or a variant thereof, or comprises an amino acid sequence having at least 80% similarity to any one of the above sequences; and/or the light chain variable region comprises an amino acid sequence as shown in any one of SEQ ID NOs: 25, 29, 33, 37 or 41 or a variant thereof, or comprises an amino acid sequence having at least 80% similarity to any one of the above sequences. For example, the heavy chain variable region comprises an amino acid sequence as shown in any one of SEQ ID NOs: 23, 76 or 80 or a variant thereof, or an amino acid sequence that has at least 80% similarity to any one of the above sequences; and/or the light chain variable region comprises an amino acid sequence as shown in any one of SEQ ID NOs: 25 or 78 or a variant thereof, or an amino acid sequence that has at least 80% similarity to any one of the above sequences. Wherein, the CDRs can be determined by a numbering system selected from the group consisting of Kabat, Chothia, IMGT, Gelfand, Aho and Martin. The antibody recognizing GPRC5D provided in the present application may comprise a CDR sequence determined by any of the above numbering systems or a combination thereof, and the CDRs in the heavy chain and/or light chain constituting the antibody do not have to be determined by the same numbering. For example, one or some CDRs in the antibody may be determined by the Kabat system, and other CDRs may be determined by any one of the above numbering systems or a combination thereof. In certain embodiments, the CDRs of the antibody can be determined by a numbering system, for example, by the Kabat numbering system; for example, by the Chothia numbering system; for example, by the IMGT numbering system; for example, by the Gelfand numbering system; for example, by the Aho numbering system; for example, by the Martin numbering system.

In certain embodiments, the antibody may comprise one CDR, two CDRs, or three CDRs in the heavy chain variable region.

In certain embodiments, the antibody may comprise one CDR, two CDRs, or three CDRs in the light chain variable region.

In certain embodiments, the antibody may comprise one CDR, two CDRs, or three CDRs in the heavy chain variable region and one CDR, two CDRs, or three CDRs in the light chain variable region. For example, the antibody may comprise one CDR in the heavy chain variable region and one, two or three CDRs in the light chain variable region; for example, it may comprise two CDRs in the heavy chain variable region and one, two or three CDRs in the light chain variable region; for example, it may comprise three CDRs in the heavy chain variable region and one, two or three CDRs in the light chain variable region; for example, it may comprise three CDRs in the heavy chain variable region and three CDRs in the light chain variable region.

In certain embodiments, the heavy chain variable region comprises an amino acid sequence as shown in any one of SEQ ID NOs: 23, 27, 31, 35, 39, 76 or 80 or a variant thereof.

In certain embodiments, the light chain variable region comprises an amino acid sequence as shown in any one of SEQ ID NOs: 25, 29, 33, 37, 41 or 78 or a variant thereof.

In certain embodiments, the heavy chain variable region comprises an amino acid sequence as shown in any one of SEQ ID NOs: 23, 27, 31, 35, 39, 76 or 80 or a variant thereof, and the light chain variable region comprises an amino acid sequence as shown in any one of SEQ ID NOs: 25, 29, 33, 37, 41 or 78 or a variant thereof. For example, the heavy chain variable region comprises the amino acid sequence of SEQ ID NO: 23 or a variant thereof, and the light chain variable region comprises the amino acid sequence as shown in any one of SEQ ID NOs: 25, 29, 33, 37, 41 or 78 or a variant thereof; for example, the heavy chain variable region comprises the amino acid sequence of SEQ ID NO: 27 or a variant thereof, and the light chain variable region comprises the amino acid sequence as shown in any one of SEQ ID NOs: 25, 29, 33, 37, 41 or 78 or a variant thereof; for example, the heavy chain variable region comprises the amino acid sequence of SEQ ID NO: 31 or a variant thereof, and the light chain variable region comprises the amino acid sequence as shown in any one of SEQ ID NOs: 25, 29, 33, 37, 41 or 78 or a variant thereof; for example, the heavy chain variable region comprises the amino acid sequence of SEQ ID NO: 35 or a variant thereof, and the light chain variable region comprises the amino acid sequence as shown in any one of SEQ ID NOs: 25, 29, 33, 37, 41 or 78 or a variant thereof; for example, the heavy chain variable region comprises the amino acid sequence of SEQ ID NO: 39 or a variant thereof, and the light chain variable region comprises the amino acid sequence as shown in any one of SEQ ID NOs: 25, 29, 33, 37, 41 or 78 or a variant thereof; for example, the heavy chain variable region comprises the amino acid sequence of SEQ ID NO:76 or a variant thereof, and the light chain variable region comprises the amino acid sequence as shown in any one of SEQ ID NOs:25, 29, 33, 37, 41 or 78 or a variant thereof; for example, the heavy chain variable region comprises the amino acid sequence of SEQ ID NO:80 or a variant thereof, and the light chain variable region comprises the amino acid sequence as shown in any one of SEQ ID NOs:25, 29, 33, 37, 41 or 78 or a variant thereof.

In certain embodiments, the heavy chain variable region comprises an amino acid sequence as shown in any one of SEQ ID NOs: 23, 27, 31, 35 or 39 or a variant thereof.

In certain embodiments, the light chain variable region comprises an amino acid sequence as shown in any one of SEQ ID NOs: 25, 29, 33, 37 or 41 or a variant thereof.

In certain embodiments, the heavy chain variable region comprises an amino acid sequence as shown in any one of SEQ ID NOs: 23, 27, 31, 35 or 39 or a variant thereof, and the light chain variable region comprises an amino acid sequence as shown in any one of SEQ ID NOs: 25, 29, 33, 37 or 41 or a variant thereof. For example, the heavy chain variable region comprises the amino acid sequence of SEQ ID NO: 23 or a variant thereof, and the light chain variable region comprises the amino acid sequence as shown in any one of SEQ ID NOs: 25, 29, 33, 37 or 41 or a variant thereof; for example, the heavy chain variable region comprises the amino acid sequence of SEQ ID NO: 27 or a variant thereof, and the light chain variable region comprises the amino acid sequence as shown in any one of SEQ ID NOs: 25, 29, 33, 37 or 41 or a variant thereof; for example, the heavy chain variable region comprises the amino acid sequence of SEQ ID NO: 31 or a variant thereof, and the light chain variable region comprises the amino acid sequence as shown in any one of SEQ ID NOs: 25, 29, 33, 37 or 41 or a variant thereof; for example, the heavy chain variable region comprises the amino acid sequence of SEQ ID NO: 35 or a variant thereof, and the light chain variable region comprises the amino acid sequence as shown in any one of SEQ ID NOs: 25, 29, 33, 37 or 41 or a variant thereof; for example, the heavy chain variable region comprises the amino acid sequence of SEQ ID NO: 39 or a variant thereof, and the light chain variable region comprises the amino acid sequence as shown in any one of SEQ ID NOs: 25, 29, 33, 37 or 41 or a variant thereof.

In certain embodiments, the heavy chain variable region comprises an amino acid sequence as shown in any one of SEQ ID NOs: 23, 76 or 80 or a variant thereof.

In certain embodiments, the light chain variable region comprises an amino acid sequence as shown in any one of SEQ ID NOs: 25 or 78 or a variant thereof.

In certain embodiments, the heavy chain variable region comprises an amino acid sequence as shown in any one of SEQ ID NOs: 23, 76 or 80 or a variant thereof, and the light chain variable region comprises an amino acid sequence as shown in any one of SEQ ID NOs: 25 or 78 or a variant thereof. For example, the heavy chain variable region comprises the amino acid sequence of SEQ ID NO:23 or a variant thereof, and the light chain variable region comprises the amino acid sequence as shown in any one of SEQ ID NOs:25 or 78 or a variant thereof; for example, the heavy chain variable region comprises the amino acid sequence of SEQ ID NO:76 or a variant thereof, and the light chain variable region comprises the amino acid sequence as shown in any one of SEQ ID NOs:25 or 78 or a variant thereof; for example, the heavy chain variable region comprises the amino acid sequence of SEQ ID NO:80 or a variant thereof, and the light chain variable region comprises the amino acid sequence as shown in any one of SEQ ID NOs:25 or 78 or a variant thereof.

For example, the heavy chain variable region comprises the amino acid sequence of SEQ ID NO:23, and the light chain variable region comprises the amino acid sequence of SEQ ID NO:25. For example, the heavy chain variable region comprises the amino acid sequence of SEQ ID NO:27, and the light chain variable region comprises the amino acid sequence of SEQ ID NO:29. For example, the heavy chain variable region comprises the amino acid sequence of SEQ ID NO:31, and the light chain variable region comprises the amino acid sequence of SEQ ID NO:33. For example, the heavy chain variable region comprises the amino acid sequence of SEQ ID NO:35, and the light chain variable region comprises the amino acid sequence of SEQ ID NO:37. For example, the heavy chain variable region comprises the amino acid sequence of SEQ ID NO:39, and the light chain variable region comprises the amino acid sequence of SEQ ID NO:41. For example, the heavy chain variable region comprises the amino acid sequence of SEQ ID NO:76, and the light chain variable region comprises the amino acid sequence of SEQ ID NO:25. For example, the heavy chain variable region comprises the amino acid sequence of SEQ ID NO:80, and the light chain variable region comprises the amino acid sequence of SEQ ID NO:25. For example, the heavy chain variable region comprises the amino acid sequence of SEQ ID NO:23, and the light chain variable region comprises the amino acid sequence of SEQ ID NO:78.

In certain embodiments, the antibody may comprise the heavy chain variable region and/or the light chain variable region. For example, the heavy chain variable region may comprise a heavy chain CDR1 (HCDR1), and the HCDR1 may comprise an amino acid sequence as shown in any one of SEQ ID NOs: 1, 11, 17, 70 or 71. For example, the heavy chain variable region may comprise a heavy chain CDR2 (HCDR2), and the HCDR2 may comprise an amino acid sequence as shown in any one of SEQ ID NOs: 2, 12, 18, 72 or 73. For example, the heavy chain variable region may comprise a heavy chain CDR3 (HCDR3), and the HCDR3 may comprise an amino acid sequence as shown in any one of SEQ ID NOs: 3, 7, 9, 13 or 19. For example, the heavy chain variable region may comprise HCDR1 and HCDR3, the HCDR1 may comprise an amino acid sequence as shown in any one of SEQ ID NOs: 1, 11, 17, 70 or 71, and the HCDR3 may comprise an amino acid sequence as shown in any one of SEQ ID NOs: 3, 7, 9, 13 or 19. For example, the heavy chain variable region may comprise HCDR1 and HCDR2, wherein the HCDR1 may comprise an amino acid sequence as shown in any one of SEQ ID NOs: 1, 11, 17, 70 or 71, and the HCDR2 may comprise an amino acid sequence as shown in any one of SEQ ID NOs: 2, 12, 18, 72 or 73. For example, the heavy chain variable region may comprise HCDR2 and HCDR3, the HCDR2 may comprise an amino acid sequence as shown in any one of SEQ ID NOs: 2, 12, 18, 72 or 73, and the HCDR3 may comprise an amino acid sequence as shown in any one of SEQ ID NOs: 3, 7, 9, 13 or 19. For example, the heavy chain variable region may comprise HCDR1, HCDR2 and HCDR3, the HCDR1 may comprise an amino acid sequence as shown in any one of SEQ ID NOs:1, 11, 17, 70 or 71, the HCDR2 may comprise an amino acid sequence as shown in any one of SEQ ID NOs:2, 12, 18, 72 or 73, and the HCDR3 may comprise an amino acid sequence as shown in any one of SEQ ID NOs:3, 7, 9, 13 or 19. For example, the light chain variable region may comprise a light chain CDR1 (LCDR1), and the LCDR1 may comprise an amino acid sequence as shown in any one of SEQ ID NOs: 4, 14, 20 or 74. For example, the light chain variable region may comprise a light chain CDR2 (LCDR2), and the LCDR2 may comprise an amino acid sequence as shown in any one of SEQ ID NOs: 5, 15, 21 or 75. For example, the light chain variable region may comprise a light chain CDR3 (LCDR3), and the LCDR3 may comprise an amino acid sequence as shown in any one of SEQ ID NOs: 6, 8, 10, 16 or 22. For example, the light chain variable region may comprise LCDR1 and LCDR3, wherein the LCDR1 may comprise an amino acid sequence as shown in any one of SEQ ID NOs: 4, 14, 20 or 74, and the LCDR3 may comprise an amino acid sequence as shown in any one of SEQ ID NOs: 6, 8, 10, 16 or 22. For example, the light chain variable region may comprise LCDR1 and LCDR2, wherein the LCDR1 may comprise an amino acid sequence as shown in any one of SEQ ID NOs:4, 14, 20 or 74, and the LCDR2 may comprise an amino acid sequence as shown in any one of SEQ ID NOs:5, 15, 21 or 75. For example, the light chain variable region may comprise LCDR2 and LCDR3, wherein the LCDR2 may comprise an amino acid sequence as shown in any one of SEQ ID NOs: 5, 15, 21 or 75, and the LCDR3 may comprise an amino acid sequence as shown in any one of SEQ ID NOs: 6, 8, 10, 16 or 22. For example, the light chain variable region may comprise LCDR1, LCDR2 and LCDR3, wherein the LCDR1 may comprise an amino acid sequence as shown in any one of SEQ ID NOs:4, 14, 20 or 74, the LCDR2 may comprise an amino acid sequence as shown in any one of SEQ ID NOs:5, 15, 21 or 75, and the LCDR3 may comprise an amino acid sequence as shown in any one of SEQ ID NOs:6, 8, 10, 16 or 22.

In certain embodiments, the antibody may comprise the heavy chain variable region and/or the light chain variable region. For example, the heavy chain variable region may comprise a heavy chain CDR1 (HCDR1), and the HCDR1 may comprise an amino acid sequence as shown in any one of SEQ ID NOs: 1, 11 or 17. For example, the heavy chain variable region may comprise a heavy chain CDR2 (HCDR2), and the HCDR2 may comprise an amino acid sequence as shown in any one of SEQ ID NOs: 2, 12 or 18. For example, the heavy chain variable region may comprise a heavy chain CDR3 (HCDR3), and the HCDR3 may comprise an amino acid sequence as shown in any one of SEQ ID NOs: 3, 7, 9, 13 or 19. For example, the heavy chain variable region may comprise HCDR1 and HCDR3, wherein the HCDR1 may comprise an amino acid sequence as shown in any one of SEQ ID NOs: 1, 11 or 17, and the HCDR3 may comprise an amino acid sequence as shown in any one of SEQ ID NOs: 3, 7, 9, 13 or 19. For example, the heavy chain variable region may comprise HCDR1 and HCDR2, wherein the HCDR1 may comprise an amino acid sequence as shown in any one of SEQ ID NOs: 1, 11 or 17, and the HCDR2 may comprise an amino acid sequence as shown in any one of SEQ ID NOs: 2, 12 or 18. For example, the heavy chain variable region may comprise HCDR2 and HCDR3, wherein the HCDR2 may comprise an amino acid sequence as shown in any one of SEQ ID NOs: 2, 12 or 18, and the HCDR3 may comprise an amino acid sequence as shown in any one of SEQ ID NOs: 3, 7, 9, 13 or 19. For example, the heavy chain variable region may comprise HCDR1, HCDR2 and HCDR3, the HCDR1 may comprise an amino acid sequence as shown in any one of SEQ ID NOs:1, 11 or 17, the HCDR2 may comprise an amino acid sequence as shown in any one of SEQ ID NOs:2, 12 or 18, and the HCDR3 may comprise an amino acid sequence as shown in any one of SEQ ID NOs:3, 7, 9, 13 or 19. For example, the light chain variable region may comprise a light chain CDR1 (LCDR1), and the LCDR1 may comprise an amino acid sequence as shown in any one of SEQ ID NOs: 4, 14 or 20. For example, the light chain variable region may comprise a light chain CDR2 (LCDR2), and the LCDR2 may comprise an amino acid sequence as shown in any one of SEQ ID NOs: 5, 15 or 21. For example, the light chain variable region may comprise a light chain CDR3 (LCDR3), and the LCDR3 may comprise an amino acid sequence as shown in any one of SEQ ID NOs: 6, 8, 10, 16 or 22. For example, the light chain variable region may comprise LCDR1 and LCDR3, wherein the LCDR1 may comprise an amino acid sequence as shown in any one of SEQ ID NOs: 4, 14 or 20, and the LCDR3 may comprise an amino acid sequence as shown in any one of SEQ ID NOs: 6, 8, 10, 16 or 22. For example, the light chain variable region may comprise LCDR1 and LCDR2, wherein the LCDR1 may comprise an amino acid sequence as shown in any one of SEQ ID NOs: 4, 14 or 20, and LCDR2 may comprise an amino acid sequence as shown in any one of SEQ ID NOs: 5, 15 or 21. For example, the light chain variable region may comprise LCDR2 and LCDR3, wherein the LCDR2 may comprise an amino acid sequence as shown in any one of SEQ ID NOs: 5, 15 or 21, and the LCDR3 may comprise an amino acid sequence as shown in any one of SEQ ID NOs: 6, 8, 10, 16 or 22. For example, the light chain variable region may comprise LCDR1, LCDR2 and LCDR3, wherein the LCDR1 may comprise an amino acid sequence as shown in any one of SEQ ID NOs:4, 14 or 20, the LCDR2 may comprise an amino acid sequence as shown in any one of SEQ ID NOs:5, 15 or 21, and the LCDR3 may comprise an amino acid sequence as shown in any one of SEQ ID NOs:6, 8, 10, 16 or 22.

In certain embodiments, the antibody may comprise the heavy chain variable region and/or the light chain variable region. For example, the heavy chain variable region may comprise a heavy chain CDR1 (HCDR1), and the HCDR1 may comprise an amino acid sequence as shown in any one of SEQ ID NOs: 1, 70 or 71. For example, the heavy chain variable region may comprise a heavy chain CDR2 (HCDR2), and the HCDR2 may comprise an amino acid sequence as shown in any one of SEQ ID NOs: 2, 72 or 73. For example, the heavy chain variable region may comprise a heavy chain CDR3 (HCDR3), and the HCDR3 may comprise the amino acid sequence of SEQ ID NO:3. For example, the heavy chain variable region may comprise HCDR1 and HCDR3, the HCDR1 may comprise the amino acid sequence as shown in any one of SEQ ID NOs: 1, 70 or 71, and the HCDR3 may comprise the amino acid sequence of SEQ ID NO: 3. For example, the heavy chain variable region may comprise HCDR1 and HCDR2, wherein the HCDR1 may comprise an amino acid sequence as shown in any one of SEQ ID NOs: 1, 70 or 71, and the HCDR2 may comprise an amino acid sequence as shown in any one of SEQ ID NOs: 2, 72 or 73. For example, the heavy chain variable region may comprise HCDR2 and HCDR3, the HCDR2 may comprise the amino acid sequence as shown in any one of SEQ ID NOs: 2, 72 or 73, and the HCDR3 may comprise the amino acid sequence of SEQ ID NO: 3. For example, the heavy chain variable region may comprise HCDR1, HCDR2 and HCDR3, the HCDR1 may comprise an amino acid sequence as shown in any one of SEQ ID NOs:1, 70 or 71, the HCDR2 may comprise an amino acid sequence as shown in any one of SEQ ID NOs:2, 72 or 73, and the HCDR3 may comprise an amino acid sequence of SEQ ID NO:3. For example, the light chain variable region may comprise a light chain CDR1 (LCDR1), and the LCDR1 may comprise the amino acid sequence as shown in any one of SEQ ID NOs: 4 or 74. For example, the light chain variable region may comprise a light chain CDR2 (LCDR2), and the LCDR2 may comprise an amino acid sequence as shown in any one of SEQ ID NOs: 5 or 75. For example, the light chain variable region may comprise a light chain CDR3 (LCDR3), and the LCDR3 may comprise the amino acid sequence of SEQ ID NO:6. For example, the light chain variable region may comprise LCDR1 and LCDR3, wherein the LCDR1 may comprise the amino acid sequence as shown in any one of SEQ ID NOs: 4 or 74, and the LCDR3 may comprise the amino acid sequence of SEQ ID NO: 6. For example, the light chain variable region may comprise LCDR1 and LCDR2, wherein the LCDR1 may comprise the amino acid sequence as shown in any one of SEQ ID NOs: 4 or 74, and the LCDR2 may comprise the amino acid sequence as shown in any one of SEQ ID NOs: 5 or 75. For example, the light chain variable region may comprise LCDR2 and LCDR3, wherein the LCDR2 may comprise the amino acid sequence as shown in any one of SEQ ID NOs: 5 or 75, and the LCDR3 may comprise the amino acid sequence of SEQ ID NO: 6. For example, the light chain variable region may comprise LCDR1, LCDR2 and LCDR3, wherein the LCDR1 may comprise an amino acid sequence as shown in any one of SEQ ID NOs:4 or 74, the LCDR2 may comprise an amino acid sequence as shown in any one of SEQ ID NOs:5 or 75, and the LCDR3 may comprise an amino acid sequence of SEQ ID NO:6.

For example, the antibody may comprise the heavy chain variable region and the light chain variable region, the heavy chain variable region may comprise the HCDR1, the HCDR2, the HCDR3, and the light chain variable region may comprise the LCDR1, the LCDR2, the LCDR3. For example, the HCDR1 may comprise the amino acid sequence of SEQ ID NO:1, the HCDR2 may comprise the amino acid sequence of SEQ ID NO:2, the HCDR3 may comprise the amino acid sequence of SEQ ID NO:3, and the LCDR1 may comprise the amino acid sequence of SEQ ID NO:4, the LCDR2 may comprise the amino acid sequence of SEQ ID NO:5, and the LCDR3 may comprise the amino acid sequence of SEQ ID NO:6. For example, the HCDR1 may comprise the amino acid sequence of SEQ ID NO:1, the HCDR2 may comprise the amino acid sequence of SEQ ID NO:2, the HCDR3 may comprise the amino acid sequence of SEQ ID NO:7, and the LCDR1 may comprise the amino acid sequence of SEQ ID NO:4, the LCDR2 may comprise the amino acid sequence of SEQ ID NO:5, and the LCDR3 may comprise the amino acid sequence of SEQ ID NO:8. For example, the HCDR1 may comprise the amino acid sequence of SEQ ID NO:1, the HCDR2 may comprise the amino acid sequence of SEQ ID NO:2, the HCDR3 may comprise the amino acid sequence of SEQ ID NO:9, and the LCDR1 may comprise the amino acid sequence of SEQ ID NO:4, the LCDR2 may comprise the amino acid sequence of SEQ ID NO:5, and the LCDR3 may comprise the amino acid sequence of SEQ ID NO:10. For example, the HCDR1 may comprise the amino acid sequence of SEQ ID NO:11, the HCDR2 may comprise the amino acid sequence of SEQ ID NO:12, the HCDR3 may comprise the amino acid sequence of SEQ ID NO:13, and the LCDR1 may comprise the amino acid sequence of SEQ ID NO:14, the LCDR2 may comprise the amino acid sequence of SEQ ID NO:15, and the LCDR3 may comprise the amino acid sequence of SEQ ID NO:16. For example, the HCDR1 may comprise the amino acid sequence of SEQ ID NO:17, the HCDR2 may comprise the amino acid sequence of SEQ ID NO:18, the HCDR3 may comprise the amino acid sequence of SEQ ID NO:19, and the LCDR1 may comprise the amino acid sequence of SEQ ID NO:20, the LCDR2 may comprise the amino acid sequence of SEQ ID NO:21, and the LCDR3 may comprise the amino acid sequence of SEQ ID NO:22. For example, the HCDR1 may comprise the amino acid sequence of SEQ ID NO:70, the HCDR2 may comprise the amino acid sequence of SEQ ID NO:72, the HCDR3 may comprise the amino acid sequence of SEQ ID NO:3, and the LCDR1 may comprise the amino acid sequence of SEQ ID NO:4, the LCDR2 may comprise the amino acid sequence of SEQ ID NO:5, and the LCDR3 may comprise the amino acid sequence of SEQ ID NO:6. For example, the HCDR1 may comprise the amino acid sequence of SEQ ID NO:71, the HCDR2 may comprise the amino acid sequence of SEQ ID NO:73, the HCDR3 may comprise the amino acid sequence of SEQ ID NO:3, and the LCDR1 may comprise the amino acid sequence of SEQ ID NO:4, the LCDR2 may comprise the amino acid sequence of SEQ ID NO:5, and the LCDR3 may comprise the amino acid sequence of SEQ ID NO:6.

In certain embodiments, the antibody comprises a heavy chain variable region, which comprises a heavy chain CDR1 comprising the amino acid sequence shown in any one of SEQ ID NOs:1, 11 or 17, and/or a heavy chain CDR2 comprising the amino acid sequence shown in any one of SEQ ID NOs:2, 12 or 18, and/or a heavy chain CDR3 comprising the amino acid sequence shown in any one of SEQ ID NOs:3, 7, 9, 13 or 19. In certain embodiments, the present application provides an antibody that recognizes GPRC5D, comprising: a light chain CDR1 comprising an amino acid sequence shown in any one of SEQ ID NOs:4, 14 or 20, and/or a light chain CDR2 comprising an amino acid sequence shown in any one of SEQ ID NOs:5, 15 or 21, and/or a light chain CDR3 comprising an amino acid sequence shown in any one of SEQ ID NOs:6, 8, 10, 16 or 22. In certain embodiments, the present application provides an antibody that recognizes GPRC5D, comprising: a heavy chain CDR1 comprising an amino acid sequence shown in any one of SEQ ID NOs: 1, 11 or 17, and/or a heavy chain CDR2 comprising an amino acid sequence shown in any one of SEQ ID NOs: 2, 12 or 18, and/or a heavy chain CDR3 comprising an amino acid sequence shown in any one of SEQ ID NOs: 3, 7, 9, 13 or 19, and/or a light chain CDR1 comprising an amino acid sequence shown in any one of SEQ ID NOs: 4, 14 or 20, and/or a light chain CDR2 comprising an amino acid sequence shown in any one of SEQ ID NOs: 5, 15 or 21, and/or a light chain CDR3 comprising an amino acid sequence shown in any one of SEQ ID NOs: 6, 8, 10, 16 or 22. For example, the antibody recognizing GPRC5D comprises: a heavy chain CDR1 comprising an amino acid sequence shown in any one of SEQ ID NOs: 1, 11 or 17, and a heavy chain CDR2 comprising an amino acid sequence shown in any one of SEQ ID NOs: 2, 12 or 18, and a heavy chain CDR3 comprising an amino acid sequence shown in any one of SEQ ID NOs: 3, 7, 9, 13 or 19, and/or a light chain CDR1 comprising an amino acid sequence shown in any one of SEQ ID NOs: 4, 14 or 20, and a light chain CDR2 comprising an amino acid sequence shown in any one of SEQ ID NOs: 5, 15 or 21, and a light chain CDR3 comprising an amino acid sequence shown in any one of SEQ ID NOs: 6, 8, 10, 16 or 22. For example, the antibody recognizing GPRC5D comprises: a heavy chain CDR1 comprising an amino acid sequence shown in any one of SEQ ID NOs: 1, 11 or 17, and a heavy chain CDR2 comprising an amino acid sequence shown in any one of SEQ ID NOs: 2, 12 or 18, and a heavy chain CDR3 comprising an amino acid sequence shown in any one of SEQ ID NOs: 3, 7, 9, 13 or 19, and a light chain CDR1 comprising an amino acid sequence shown in any one of SEQ ID NOs: 4, 14 or 20, and a light chain CDR2 comprising an amino acid sequence shown in any one of SEQ ID NOs: 5, 15 or 21, and a light chain CDR3 comprising an amino acid sequence shown in any one of SEQ ID NOs: 6, 8, 10, 16 or 22. For example, the antibody recognizing GPRC5D comprises: HCDR1 shown as SEQ ID NO:1, HCDR2 shown as SEQ ID NO:2, and HCDR3 shown as SEQ ID NO:3; LCDR1 shown as SEQ ID NO:4, LCDR2 shown as SEQ ID NO:5, and LCDR3 shown as SEQ ID NO:6; or HCDR1 shown as SEQ ID NO:1, HCDR2 shown as SEQ ID NO:2, and HCDR3 shown as SEQ ID NO:7; LCDR1 shown as SEQ ID NO:4, LCDR2 shown as SEQ ID NO:5, and LCDR3 shown as SEQ ID NO:8; or HCDR1 shown as SEQ ID NO:1, HCDR2 shown as SEQ ID NO:2, and HCDR3 shown as SEQ ID NO:9; LCDR1 shown as SEQ ID NO:4, LCDR2 shown as SEQ ID NO:5, and LCDR3 shown as SEQ ID NO:10.

In certain embodiments, the antibody may comprise the heavy chain variable region and/or the light chain variable region. For example, the heavy chain variable region may comprise an amino acid sequence as shown in any one of SEQ ID NOs: 23, 27, 31, 35, 39, 76 or 80. For example, the light chain variable region may comprise an amino acid sequence as shown in any one of SEQ ID NOs: 25, 29, 33, 37, 41 or 78.

In certain embodiments, the antibody may comprise the heavy chain variable region and/or the light chain variable region. For example, the heavy chain variable region may comprise an amino acid sequence as shown in any one of SEQ ID NOs: 23, 27, 31, 35 or 39. For example, the light chain variable region may comprise an amino acid sequence as shown in any one of SEQ ID NOs: 25, 29, 33, 37 or 41.

In certain embodiments, the antibody may comprise the heavy chain variable region and/or the light chain variable region. For example, the heavy chain variable region may comprise an amino acid sequence as shown in any one of SEQ ID NOs: 23, 76 or 80. For example, the light chain variable region may comprise an amino acid sequence as shown in any one of SEQ ID NOs: 25 or 78.

For example, the antibody may comprise the heavy chain variable region and the light chain variable region. For example, the heavy chain variable region comprises the amino acid sequence of SEQ ID NO:23, and the light chain variable region comprises the amino acid sequence of SEQ ID NO:25. For example, the heavy chain variable region comprises the amino acid sequence of SEQ ID NO:27, and the light chain variable region comprises the amino acid sequence of SEQ ID NO:29. For example, the heavy chain variable region comprises the amino acid sequence of SEQ ID NO:31, and the light chain variable region comprises the amino acid sequence of SEQ ID NO:33. For example, the heavy chain variable region comprises the amino acid sequence of SEQ ID NO:35, and the light chain variable region comprises the amino acid sequence of SEQ ID NO:37. For example, the heavy chain variable region comprises the amino acid sequence of SEQ ID NO:39, and the light chain variable region comprises the amino acid sequence of SEQ ID NO:41. For example, the heavy chain variable region comprises the amino acid sequence of SEQ ID NO:76, and the light chain variable region comprises the amino acid sequence of SEQ ID NO:25. For example, the heavy chain variable region comprises the amino acid sequence of SEQ ID NO:80, and the light chain variable region comprises the amino acid sequence of SEQ ID NO:25. For example, the heavy chain variable region comprises the amino acid sequence of SEQ ID NO:23, and the light chain variable region comprises the amino acid sequence of SEQ ID NO:78.

In another aspect, the present application provides an antibody that recognizes GPRC5D, comprising a heavy chain variable region, wherein the heavy chain variable region comprises an amino acid sequence shown in any one of SEQ ID NOs: 23, 27, 31, 35 or 39, or a variant of the above sequence.

In another aspect, the present application provides an antibody that recognizes GPRC5D, comprising a light chain variable region, wherein the light chain variable region comprises an amino acid sequence shown in any one of SEQ ID NOs: 25, 29, 33, 37 or 41, or a variant of the above sequence.

In another aspect, the present application provides an antibody that recognizes GPRC5D, comprising the above-mentioned heavy chain variable region and light chain variable region.

Considering that each of these heavy chain variable region and light chain variable region sequences can bind to GPRC5D, the heavy chain and light chain variable region sequences can be "mixed and matched" to generate the anti-GPRC5D binding molecules of the present application.

In another aspect, the present application provides variants of antibodies or variants of fragments thereof that bind to GPRC5D. Thus, the present application provides antibodies or fragments thereof having a heavy chain and/or light chain variable region that is at least 80% identical to the sequence of variable regions of the heavy chain or light chain. Preferably, the amino acid sequence identity of the heavy and/or light chain variable regions is at least 85%, more preferably at least 90%, most preferably at least 95%, particularly 96%, more particularly 97%, even more particularly 98%, most particularly 99%, including for example 80%, 81%, 82%, 83%, 84%, 85%, 86%, 87%, 88%, 89%, 90%, 91%, 92%, 93%, 94%, 95%, 96%, 97%, 98%, 99% and 100%. Variants can be obtained by using the antibodies described in this application as parent antibodies through methods such as yeast library screening, phage library screening, point mutation and the like.

In another aspect, the present application provides an antibody that recognizes the same antigenic determinant as the aforementioned anti-GPRC5D antibody.

In another aspect, the present application provides an antibody that competitively binds to GPRC5D with the aforementioned anti-GPRC5D antibody.

In another aspect, the present application provides an antibody that specifically binds to GPRC5D, which is a whole antibody, scFv, single domain antibody, Fab fragment, Fab' fragment, Fv fragment, F(ab')₂ fragment, Fd fragment, dAb fragment, multifunctional antibody or scFv-Fc antibody.

### Antibody assay

The anti-GPRC5D antibodies provided herein can be identified, screened, or characterized for their physical/chemical properties and/or biological activities by a variety of assays known in the art. These include, for example, ELISA, biacore, Western blot, and flow cytometric analysis. Suitable assays are described in detail in the Examples.

The term "affinity" refers to the sum of the strength of non-covalent interactions between a single binding site of a molecule (e.g., an antibody) and its binding partner (e.g., an antigen). Unless otherwise specified, "binding affinity" as used herein refers to intrinsic binding affinity, which reflects a 1:1 interaction between members of a binding pair (e.g., antibody and antigen). The affinity of a molecule X for its ligand Y can generally be represented by the dissociation constant (Kd). Affinity can be measured by routine methods known in the art, including determining the affinity of an antibody using Biacore as described herein. The "affinity" of an antibody for GPRC5D herein is expressed as the KD of the antibody. The KD of an antibody refers to the equilibrium dissociation constant of the antibody-antigen interaction. The larger the KD value of an antibody binding to its antigen, the weaker its binding affinity for that particular antigen.

The term "EC50", concentration for 50% of maximal effect, refers to the concentration that can cause 50% of the maximal effect.

### Antigen

The term "antigen" refers to a substance that is recognized and specifically bound by an antigen binding unit. Antigens may comprise peptides, proteins, glycoproteins, polysaccharides and lipids, portions thereof and combinations thereof. Non-limiting exemplary antigens include tumor antigens or pathogen antigens. "Antigen" may also refer to a molecule that elicits an immune response. This immune response may involve the production of antibodies or the activation of specific immunologically-competent cells, or both. Those skilled in the art will appreciate that any macromolecule, including virtually all proteins or peptides, can serve as an antigen.

The term "epitope" refers to an antigen or part of an antigen that can be recognized by an antibody, B cell, T cell or engineered cell. For example, an epitope can be a tumor epitope or a pathogen epitope recognized by an antibody; an antibody recognizes multiple epitopes within an antigen. Epitopes can also be mutated.

The term "antigenic determinant" is also known as "antigenic epitope" or "epitope", and comprises any determinant or region capable of being bound by an antibody. An antigenic epitope is a region of an antigen that is bound by an antibody targeted to the antigen, comprising specific amino acids that are in direct contact with the antibody. Exemplarily, the antigenic epitope may consist of continuous sequence of the GPRC5D protein sequence, or may consist of three-dimensional structure of discontinuous sequence of the GPRC5D protein. Exemplarily, the antigen used herein is human or murine GPRC5D.

### Immunoconjugates

The present application also provides an immunoconjugate, which comprises the antibody described herein and a functional molecule linked thereto. The antibodies provided in the present application have been described above, and the immunoconjugates provided in the present application comprise all of the technical solutions thereof. The antibody and the functional molecule can form a conjugate by covalent bonding, coupling, attachment, cross-linking, etc.

"Connect" or "fuse" are used interchangeably herein. It generally refers to the joining together of two or more chemical elements or components by any means including chemical conjugation or recombinant methods. "In-frame fusion" refers to the joining of two or more open reading frames (ORFs) to form a continuous longer ORF in a manner that maintains the correct reading frame of the original ORFs. Thus, the resulting recombinant fusion protein is a single protein containing two or more fragments corresponding to the polypeptides encoded by the original ORFs (these fragments are normally not so linked in nature). Although the reading frame is thus continuous throughout the fused segments, the segments may be physically or spatially separated by, for example, in-frame linker sequences (e.g., "flexons").

Another aspect of the present application provides a nucleic acid molecule encoding at least one antibody, a functional variant thereof or an immunoconjugate of the present application. Once the relevant sequence is obtained, large amount of the relevant sequence can be obtained through recombinant method. This is usually done by cloning it into a vector, then transferring it into cells, and then isolating the relevant sequence from the proliferated host cells by conventional methods.

The present application also relates to a vector comprising the above-mentioned appropriate DNA sequence and an appropriate promoter or control sequence. These vectors can be used to transform appropriate host cells to enable them to express proteins. The host cell can be a prokaryotic cell, such as a bacterial cell; or a lower eukaryotic cell, such as a yeast cell; or a higher eukaryotic cell, such as a mammalian cell.

### Chimeric receptor

The present application also provides a chimeric receptor, which generally refers to an expression product of a fusion molecule formed by connecting DNA fragments or corresponding cDNAs of proteins from different sources using gene recombination technology, and may comprise an extracellular domain, a transmembrane domain, and an intracellular domain. The extracellular domain comprises the antibody provided in the present application, which has been described above in detail, and the chimeric receptor provided in the present application comprises all technical solutions thereof. The chimeric receptors include, but are not limited to, chimeric antigen receptors (CARs), chimeric T cell receptors, and T cell antigen couplers (TACs).

In some embodiments, the chimeric receptor of the present application is a chimeric antigen receptor (CAR). In certain embodiments, the extracellular antigen binding region (or extracellular domain) of CAR is derived from a mouse or humanized or human monoclonal antibody.

The chimeric antigen receptor generally comprises an extracellular antigen binding region or an antibody. In some embodiments, the extracellular antigen binding region may be fully human. In other cases, the extracellular antigen binding region may be humanized. In other cases, the extracellular antigen-binding region may be of murine origin, or a chimera in which the extracellular antigen-binding region is composed of amino acid sequences from at least two different animals. In some embodiments, the extracellular antigen binding region may be non-human.

In certain embodiments, the chimeric antigen receptor can also be designed to contain a variety of antigen binding regions, including single-chain variable fragments (scFv) derived from antibodies, fragment antigen binding regions (Fab) selected from libraries, single domain fragments, or natural ligands that bind to their cognate receptors. In some embodiments, the extracellular antigen binding region may comprise scFv, Fab or natural ligand, and any derivatives thereof. The extracellular antigen-binding region may refer to a molecule other than an intact antibody, which may comprise a portion of an intact antibody and which can bind to the antigen to which the intact antibody binds. Examples of antibody fragments may include, but are not limited to, Fv, Fab, Fab', Fab'-SH, F(ab')₂; bifunctional antibodies, linear antibodies; single-chain antibody molecules (e.g., scFv); and multispecific antibodies formed from antibody fragments. The extracellular antigen binding region, e.g. scFv, Fab or natural ligand, can be the part of the CAR that determines the antigen specificity. The extracellular antigen binding region can bind to any complementary target. The extracellular antigen binding region can be derived from an antibody with known variable region sequence. The extracellular antigen binding region can be obtained from antibody sequences obtained from available mouse hybridomas. Alternatively, the extracellular antigen binding region can be obtained from whole exosome cleavage sequencing of tumor cells or primary cells e.g. tumor infiltrating lymphocytes (TILs).

In some embodiments, the binding specificity of the extracellular antigen binding region of the CAR can be determined by a complementarity determining region or CDR, such as a light chain CDR or a heavy chain CDR. In many cases, binding specificity can be determined by both the light chain CDRs and the heavy chain CDRs.

In some embodiments, the extracellular antigen binding region of the CAR comprises a hinge or a spacer, wherein the hinge and spacer can be used interchangeably. A hinge can be considered as the part of the CAR that provides flexibility to the extracellular antigen binding region. In some embodiments, the hinge can be used to detect CAR on the cell surface of a cell, particularly when antibodies detecting the extracellular antigen binding region are ineffective or unavailable. In some embodiments, the hinge may not belong to an immunoglobulin, but rather to another kind of molecule, such as the native hinge of the CD8α molecule. The CD8α hinge may contain cysteine and proline residues known to play a role in the interaction of CD8 coreceptors and MHC molecules. The hinge can be adjusted depending on the extracellular antigen binding region used. The hinge can be of any length. For example, the hinge may comprise the amino acid sequence of SEQ ID NO:59.

The transmembrane domain (or structural region) of a CAR can anchor CAR on the plasma membrane of a cell. The native transmembrane portion of CD28 can be used for CAR. In other cases, the native transmembrane portion of CD8α can also be used in CAR. "CD8" may be a protein having at least 85, 90, 95, 96, 97, 98, 99 or 100% identical to NCBI Reference No: NP_001759 or a fragment thereof having stimulatory activity. A "CD8 nucleic acid molecule" can be a polynucleotide encoding a CD8 polypeptide, and in some cases, the transmembrane region can be the native transmembrane portion of CD28, and "CD28" can refer to a protein having at least 85, 90, 95, 96, 97, 98, 99 or 100% identical to NCBI Reference Number: NP_006130 or a fragment thereof having stimulatory activity. A "CD28 nucleic acid molecule" may be a polynucleotide encoding a CD28 polypeptide. In some embodiments, the transmembrane portion may comprise a CD8α region. For example, the transmembrane domain may comprise the amino acid sequence of SEQ ID NO:60.

The intracellular signaling region of a CAR may be responsible for activating at least one of the effector functions of an immune response cell comprising the CAR. A CAR can induce effector functions of T cells, for example, the effector functions are cytolytic activity or adjuvant activity, including secretion of cytokines, such as IL-2, TNF-α, γ-IFN, etc. Thus, the term "intracellular signaling domain" refers to the portion of a protein that transduces the effector function signal and directs the cell to perform a specific function. Although the entire intracellular signaling region can usually be used, in many cases it is not necessary to use the entire chain of signaling domains. In some embodiments, truncated portions of the intracellular signaling region are used. In some embodiments, the term "intracellular signaling region" is thus intended to comprise any truncated portion of the intracellular signaling region that is sufficient to transduce the effector function signal.

Preferred examples of the signaling domain (or structural region) used in CAR may comprise the cytoplasmic sequence of the T cell receptor (TCR) and a co-receptor that cooperates to initiate signal transduction after target-receptor binding, as well as any derivative or variant sequence thereof and any synthetic sequence of these sequences having the same functionality.

In some embodiments, the intracellular signaling region of the CAR may comprise a signaling motif of a known immunoreceptor tyrosine activation motif (ITAM). Examples of ITAMs containing cytoplasmic signaling sequences comprise those derived from CD3ζ, FcRγ, FcRβ, CD3γ, CD3δ, CD3ε, CD5, CD22, CD79a, CD79b, and CD66d. However, in preferred embodiments, the intracellular signaling domain is derived from the CD3ζ chain.

An example of a T cell signaling domain containing one or more ITAM motifs is the CD3ζ domain, also known as the T cell receptor CD3ζ chain or CD247. This domain is part of the T cell receptor-CD3 complex and plays an important role in coupling antigen recognition of several intracellular signaling pathways with primary effector activation of T cells. As used herein, CD3ζ refers primarily to human CD3ζ and isoforms thereof (as known from Swissprot entry P20963) and comprises proteins having substantially the same sequence. As part of a chimeric antigen receptor, the entire T cell receptor CD3ζ chain is not required and any derivative thereof comprising the signalling domain of the T cell receptor CD3ζ chain is suitable, including any functional equivalents thereof. For example, the signal domain of the CD3ζ chain may comprise the amino acid sequence of SEQ ID NO:62.

In certain embodiments, the intracellular signaling domain (or structural region) of the CAR can be selected from any one of the co-stimulatory domains in Table 2. In some embodiments, a domain can be modified such that the identity to a reference domain can be from about 50% to about 100%. Any one of the domains in Table 2 may be modified such that the modified form may contain about 50%, 60%, 70%, 80%, 90%, 95%, 96%, 97%, 98%, 99% or up to about 100% identity.

In certain embodiments, the intracellular signaling region of the CAR may further comprise one or more costimulatory domains. The intracellular signaling region can comprise a single co-stimulatory domain, e.g. ζ chain (CAR of the first generation) or it and CD28 or 4-1BB (CAR of the second generation). In other embodiments, the intracellular signaling region can comprise two co-stimulatory domains, e.g. CD28/OX40 or CD28/4-1BB (the third generation). For example, the costimulatory domain of 4-1BB may comprise the amino acid sequence of SEQ ID NO:61.

In certain embodiments, together with intracellular signaling domains such as CD8, these co-stimulatory domains can produce downstream activation of kinase pathways, thereby supporting gene transcription and functional cellular responses. The co-stimulatory domain of CAR can activate proximal signaling proteins associated with the CD28 (phosphatidylinositol-4,5-bisphosphate 3-kinase) or 4-1BB/OX40 (TNF-receptor-associated factor adaptor protein) pathways as well as MAPK and Akt activation.

In certain cases, signaling produced via CAR may be combined with auxiliary or co-stimulatory signals. Regarding the co-stimulatory signaling domain, the chimeric antigen receptor-like complex can be designed to comprise several possible co-stimulatory signaling domains. It is well known in the art that in naive T cells, binding of the T cell receptor alone is not sufficient to induce full activation of the T cell into a cytotoxic T cell. Full productive T cell activation requires a second co-stimulatory signal. Several receptors have been reported to provide co-stimulation for T cell activation, including but not limited to CD28, OX40, CD27, CD2, CD5, ICAM-1, LFA-1 (CD11a/CD18), 4-1BBL, MyD88, and 4-1BB. All of the signaling pathways used by these costimulatory molecules can act synergistically with the activation signal of the primary T cell receptor. The signals provided by these co-stimulatory signaling domains can synergize with the primary effector activation signals derived from one or more ITAM motifs (e.g., the CD3 zeta signaling domain) and can fulfill the requirements of T cell activation.

In some embodiments, the addition of a co-stimulatory domain to the chimeric antigen receptor-like complex can enhance the efficacy and durability of the engineered cells. In other embodiments, the T cell signaling domain and the co-stimulatory domain are fused to each other to form a signaling region.

**Table 2. Costimulatory domain**

| Gene markers | abbreviation | name |
|---|---|---|
| CD27 | CD27; T14; S152; Tp55; TNFRSF7; S152.LPFS2 | CD27 molecule |
| CD28 | Tp44; CD28; CD28 antigen | CD28 molecule |
| TNFRSF9 | ILA; 4-1BB; CD137; CDw137 | the tumor necrosis factor receptor superfamily member 9 |
| TNFRSF4 | OX40; ACT35; CD134; IMD16; TXGP1L | the tumor necrosis factor receptor superfamily member 4 |
| TNFRSF8 | CD30; Ki-1; D1S166E | the tumor necrosis factor receptor superfamily member 8 |
| CD40LG | IGM; IMD3; TRAP; gp39; CD154; CD40L; HIGM1; T-BAM; TNFSF5; hCD40L | CD40 ligand |
| ICOS | AILIM; CD278; CVID1 | Inducible T cell co-stimulators |
| ITGB2 | LAD; CD18; MF17; MFI7; LCAMB; LFA-1; MAC-1 | Integrin β2 (No. 3 and 4 subunits of the receptor of complement component 3 ) |
| CD2 | T11; SRBC; LFA-2 | CD2 molecule |
| CD7 | GP40; TP41; Tp40; LEU-9 | CD7 molecule |
| KLRC2 | NKG2C; CD159c; NKG2-C | Killer cell lectin like receptor subfamily C, member 2 |
| TNFRSF18 | AITR; GITR; CD357; GITR-D | the tumor necrosis factor receptor superfamily member 18 |
| TNFRSF14 | TR2; ATAR; HVEA; HVEM; CD270; LIGHTR | the tumor necrosis factor receptor superfamily member 14 |
| HAVCR1 | TIM; KIM1; TIM1; CD365; HAVCR; KIM-1; TIM-1; TIMD1; TIMD-1; HAVCR-1 | Hepatitis A virus cell receptor 1 |
| LGALS9 | HUAT; LGALS9A, Galectin-9 | Lectin, galactoside binding, soluble, 9 |
| CD83 | BL11; HB15 | CD83 molecule |

In some embodiments, cells (e.g., T cells) are transduced with a viral vector encoding a CAR. In some embodiments, the viral vector is a lentiviral vector. In some embodiments, the cells can stably express the CAR.

In certain embodiments, the GPRC5D binding portion of the CAR is a scFv that maintains equivalent affinity binding compared to the Fab antibody from which it is derived, e.g., it binds the same antigen with comparable efficacy. The antibody fragment is functional in that it provides a biochemical response, e.g. activating an immune response, inhibiting the initiation of signaling from its target antigen, inhibiting kinase activity, etc. For example, the scFv may comprise a sequence as shown in any one of SEQ ID NOs: 43, 82, 84 or 86.

In some embodiments, the anti-GPRC5D antigen binding domain of the CAR is a fully human antibody fragment.

In some embodiments, the CAR of the present application combines the antigen binding domain of a specific antibody with an intracellular signaling molecule. For example, the intracellular signaling molecules include, but are not limited to, CD3ζ chain, 4-1BB and CD28 signaling modules and combinations thereof.

In certain embodiments, the GPRC5D-CAR comprises at least one intracellular signaling domain selected from the group consisting of: a CD137 (4-1BB) signaling domain, a CD28 signaling domain, a CD3ζ signaling domain, and any combination thereof. In one aspect, the GPRC5D-CAR comprises at least one intracellular signaling domain derived from one or more co-stimulatory molecules other than CD137 (4-1BB) or CD28.

As an example, the sequence of GPRC5D-CAR can be:

with an extracellular domain of SEQ ID NO:43, a hinge domain of SEQ ID NO:59, a transmembrane domain of SEQ ID NO:60, a costimulatory signal domain of SEQ ID NO:61, and a primary signal domain (AB3-BBZ) of SEQ ID NO:62.

With an extracellular domain of SEQ ID NO:82, a hinge domain of SEQ ID NO:59, a transmembrane domain of SEQ ID NO:60, a costimulatory signal domain of SEQ ID NO:61, and a primary signal domain (AB6-BBZ) of SEQ ID NO:62.

With an extracellular domain of SEQ ID NO:84, a hinge domain of SEQ ID NO:59, a transmembrane domain of SEQ ID NO:60, a costimulatory signal domain of SEQ ID NO:61, and a primary signal domain (AB7-BBZ) of SEQ ID NO:62.

With an extracellular domain of SEQ ID NO:86, a hinge domain of SEQ ID NO:59, a transmembrane domain of SEQ ID NO:60, a costimulatory signal domain of SEQ ID NO:61, and a primary signal domain (AB8-BBZ) of SEQ ID NO:62.

Exemplarily, the amino acid sequence of the chimeric antigen receptor is as shown in any one of SEQ ID NOs: 63, 88, 89, or 90.

The transmembrane domain and intracellular domain of the chimeric antigen receptor mentioned above can be replaced by conventional transmembrane domains and intracellular domains selected by those skilled in the art, and all of them fall within the protection scope of the present application.

### Nucleic acids, vectors, viruses, host cells

The terms "encoded by nucleic acid molecule", "encoding DNA sequence," and "encoding DNA" refer to the sequence or order of deoxyribonucleotides along a deoxyribonucleic acid chain. The order of these deoxyribonucleotides determines the order of the amino acids along the polypeptide (protein) chain. Thus, the nucleic acid sequence encodes the amino acid sequence.

When used to refer to a nucleotide sequence, the term "sequence" as used herein comprises DNA or RNA, and may be single-stranded or double-stranded.

The term "target sequence" refers to a sequence that is complementary to a guide sequence, and complementary pairing between the target sequence and the guide sequence promotes formation of a CRISPR complex. A target sequence can comprise any polynucleotide, such as a DNA or RNA polynucleotide. In some embodiments, the target sequence is located in the nucleus or cytoplasm of a cell.

The term sequence "identity" is determined by comparing two best matched sequences over a comparison window (e.g., at least 20 positions), wherein the portion of the polynucleotide or polypeptide sequence in the comparison window may contain additions or deletions (i.e., gaps), for example, 20% or less gaps (e.g., 5 to 15%, or 10 to 12%) for the two best matched sequences compared to the reference sequence (which does not contain additions or deletions). The percentage is typically calculated by determining the number of positions at which the identical nucleic acid base or amino acid residue occurs in the two sequences to produce the number of correctly matched positions, dividing the number of correctly matched positions by the total number of positions in the reference sequence (i.e., the window size), and multiplying the result by 100 to produce the percentage of sequence identity.

The term "transfection" refers to introduction of exogenous nucleic acid into eukaryotic cells. Transfection can be achieved by various means known in the art, including calcium phosphate-DNA coprecipitation, transfection mediated by DEAE-dextran, transfection mediated by polybrene, electroporation, microinjection, liposome fusion, lipofection, protoplast fusion, retroviral infection, and biolistics.

The term "expression vector" as used herein refers to a vector comprising a recombinant polynucleotide, it comprises an expression control sequence effectively linked to a nucleotide sequence to be expressed. The expression vector comprises sufficient cis-acting elements for expression; other elements for expression can be provided by the host cell or in vitro expression system. Expression vectors comprise all those known in the art, such as plasmids, viruses (e.g., lentiviruses, retroviruses, adenoviruses, and adeno-associated viruses).

The term "vector" as used herein is a composition that contains an isolated nucleic acid and can be used to deliver the isolated nucleic acid to the interior of a cell. Many vectors are known in the art, including but not limited to linear polynucleotides, polynucleotides associated with ionic or amphiphilic compounds, plasmids, and viruses. Thus, the term "vector" comprises an autonomously replicating plasmid or virus. Non-plasmid and non-viral compounds that facilitate transfer of nucleic acids into cells may also be comprised, e.g. polylysine compounds, liposomes, and so on.

As used herein, the term "lentivirus" refers to the genus of the Retroviridae. Retroviruses are unique among retroviruses in their ability to infect non-dividing cells; they can deliver large amount of genetic information into the DNA of host cells and therefore they are one of the most efficient methods of gene delivery vectors. HIV, SIV, and FIV are all examples of lentiviruses. Vectors derived from lentiviruses provide a means to achieve significant levels of gene transfer in vivo.

The term "endogenous" refers to a nucleic acid molecule, polypeptide, etc. that originates from the organism itself.

The term "exogenous" as used herein refers to a nucleic acid molecule or polypeptide, cell, tissue, etc. that is not endogenously expressed in the organism itself, or the expression level is insufficient to realize the function it has when overexpressed.

The term "heterologous protein" or "exogenous protein" used herein may be a protein that is exogenously introduced into cells and recognizes a target antigen, such as an exogenous receptor (i.e., the aforementioned "chimeric receptor" herein).

The term "host" as used herein refers to a recipient of a transplant, and in some embodiments, may be an individual, such as a human, into which exogenous cells are implanted.

The term "isolated" means separation from cellular or other components with which the polynucleotide, peptide, polypeptide, protein, antibody or a fragment thereof is normally associated in nature. As will be appreciated by those skilled in the art, a non-naturally occurring polynucleotide, peptide, polypeptide, protein, antibody or a fragment thereof need not be "isolated" to distinguish it from its naturally occurring counterpart. In addition, a "concentrated," "isolated," or "diluted" polynucleotide, peptide, polypeptide, protein, antibody, or a fragment thereof can be distinguished from its naturally occurring counterpart because the concentration or number of molecules per volume is greater ("concentrated") or less ("diluted") than that of its naturally occurring counterpart. The degree of enrichment can be measured on an absolute basis, e.g. weight per solution volume, or can be measured relative to another potential interferent present in the original mixture. In some embodiments, the technical solution of the present application preferably has a higher degree of enrichment. Thus, for example, preferably a 2-fold enrichment, more preferably a 10-fold enrichment, even more preferably a 100-fold enrichment, and even more preferably a 1000-fold enrichment. An "isolated" substance may also be provided by artificial assembly methods, e.g. by chemical synthesis or recombinant expression.

The present application provides isolated nucleic acids and vectors encoding antibodies or fragments thereof that recognize GPRC5D, and host cells comprising the nucleic acids or vectors. The nucleic acid can be in intact cells, in cell lysis solution, or in a partially purified or substantially purified form.

The nucleic acid of the present application can be obtained using standard molecular biology techniques. For example, cDNA encoding the light and heavy chains of antibodies or encoding VH and VL segments can be obtained by standard PCR amplification or cDNA cloning techniques. For antibodies obtained from an immunoglobulin gene library (e.g., using phage display technology), one or more nucleic acids encoding the antibodies can be recovered from the library. Methods for introducing exogenous nucleic acids into host cells are generally known in the art and may vary depending on the host cell used.

Preferably, the nucleic acid molecule of the present application is selected from SEQ ID NOs: 24, 28, 32, 36, 40, 77 or 81 encoding the heavy chain variable region, and/or selected from SEQ ID NOs: 26, 30, 34, 38, 42 or 79 encoding the light chain variable region. More preferably, it is a nucleic acid molecule comprising a heavy chain variable region sequence of SEQ ID NO:24, and a light chain variable region sequence of SEQ ID NO:26; or comprising a heavy chain variable region sequence of SEQ ID NO:28, and comprising a light chain variable region sequence of SEQ ID NO:30; or comprising a heavy chain variable region sequence of SEQ ID NO:32, and comprising a light chain variable region sequence of SEQ ID NO:34; or comprising a heavy chain variable region sequence of SEQ ID NO:36, and comprising a light chain variable region sequence of SEQ ID NO:38; or comprising a heavy chain variable region sequence of SEQ ID NO:40, and comprising a light chain variable region sequence of SEQ ID NO:42; or comprising a heavy chain variable region sequence of SEQ ID NO:77, and comprising a light chain variable region sequence of SEQ ID NO:26; or comprising a heavy chain variable region sequence of SEQ ID NO:81, and comprising a light chain variable region sequence of SEQ ID NO:26; or comprising a heavy chain variable region sequence of SEQ ID NO:24, and comprising a light chain variable region sequence of SEQ ID NO:79.

In one embodiment, one or more vectors (e.g., expression vectors) comprising the above-described nucleic acids are provided.

The term "cell" refers to a cell of human or non-human animal origin.

The term "host cell" refers to a cell into which exogenous nucleic acid is introduced, including the progeny of such a cell. Host cells comprise "transformants" and "transformed cells," which comprise the transformed primary cells and progeny derived therefrom without regard to number of passages. The nucleic acid content of progeny may not be completely identical to that of a parent cell and may contain mutations. Mutant progeny that have the same function or biological activity as those screened or selected from the originally transformed cell are included herein.

The term "GPRC5D-positive host cells" refers to host cells that express GPRC5D on the cell surface, which cells can be detected by, for example, flow cytometry using antibodies that specifically recognize epitopes on GPRC5D.

In some embodiments, the host cell is an immune effector cell.

The term "immune effector cells" refers to cells that participate in immune responses and produce immune effects, such as T cells, B cells, natural killer (NK) cells, natural killer T (NKT) cells, dendritic cells, CIK cells, macrophages, mastocytes, etc. In some embodiments, the immune effector cells are T cells, NK cells, or NKT cells. In some embodiments, the T cells can be autologous T cells, xenogeneic T cells, or allogeneic T cells. In some embodiments, the NK cells can be allogeneic NK cells. "Immune effector function or immune effector response" refers to a function or response of an immune effector cell, e.g. one that enhances or promotes an immune attack on a target cell. For example, immune effector function or response refers to the property of T cells or NK cells that promotes killing of target cells or inhibits growth or proliferation.

The term "artificially engineered cells having immune effector cell function" refers to cells or cell lines without immune effects that have acquired immune effector cell function after being artificially modified or stimulated by stimulants. For example, 293T cells are artificially modified to have the function of immune effector cells; for example, stem cells are induced in vitro to differentiate into immune effector cells.

In some cases, "T cells" may be pluripotent stem cells from the bone marrow that differentiate and mature into mature T cells with immune activity in thymus. In some cases, "T cells" can be a cell population with specific phenotypic characteristics, or a mixed cell population with different phenotypic characteristics, such as "T cells" can be cells comprising at least one T cell subpopulation: stem cell-like memory T cells (Tscm cells), central memory T cells (Tcm), effector T cells (Tef, Teff), regulatory T cells (tregs) and/or effector memory T cells (Tern). In some cases, a "T cell" may be a T cell of a specific subtype, such as a γδ T cell.

T cells can be obtained from many sources, including PBMCs, bone marrow, lymph node tissue, umbilical cord blood, thymus tissue, and tissue from sites of infection, ascites, pleural effusions, spleen tissue, and tumors. In some cases, T cells can be obtained from blood collected from an individual using any number of techniques known to those of skill in the art, e.g. Ficoll^{™} separation. In one embodiment, cells from the circulating blood of an individual are obtained by apheresis. Apheresis products typically contain lymphocytes, including T cells, monocytes, granulocytes, B cells, other nucleated white blood cells, red blood cells, and platelets. In one embodiment, cells collected by apheresis can be washed to remove plasma molecules and placed in an appropriate buffer or culture medium for subsequent processing steps. In one embodiment, T cells can be obtained from healthy donors, or from derived cells from patients diagnosed with tumor.

The term "peripheral blood mononuclear cell" (PBMC) refers to cells with a single nucleus in peripheral blood, including lymphocytes, monocytes, etc.

The terms "activating" and "activation" are used interchangeably and can refer to the process during which cells transforming from a quiescent state to an active state. The process may comprise response to antigen, phenotypic or genetic changes in migration and/or functional activity state. For example, the term "activation" may refer to the process of gradual activation of NK cells and T cells.

The term "T cell activation" or "T cell activating" refers to the state of T cells that are sufficiently stimulated to induce detectable cell proliferation, cytokine production, and/or detectable effector function.

The term "chemokine" is a polypeptide with a molecular weight of 8 to 10 kDa. It is the largest cytokine family. Its main function is to recruit monocytes, neutrophils, lymphocytes, etc. in the blood into specific lymphoid organs and tissues as well as sites of infection. "Chemokine receptors" are a type of seven-transmembrane G protein-coupled receptors (GPCRs) that mediate functions of chemokines and are usually expressed on the cell membranes of immune cells, neutrophils, endothelial cells, etc. Chemokines and chemokine receptors play an important role in mediating cell migration, proliferation and resistance to pathogen invasion, and are closely related to the occurrence and development of inflammation and cancer in the immune environment.

The term "safety switch" is intended to improve the safety of CAR-T therapy by designing a rapid and reversible "off" or "on" safety switch for CAR-T cells to minimize treatment-related toxicity. Although CAR-T cell therapy has excellent clinical characteristics, it can cause severe CRS and other potentially fatal side effects when tumor burden is unpredictable and T cell activity is uncontrollable. To control toxicity, severe CRS requires the use of the CRS grading system as guidance for appropriate monitoring and precise modulation using small-molecule-based safety switches.

In another embodiment, a host cell comprising the above-described nucleic acid is provided. The host cell comprises (e.g., is transduced with): (1) a vector comprising nucleic acids encoding an amino acid sequence comprising the VL of the antibody and an amino acid sequence comprising the VH of the antibody, or (2) a first vector comprising a nucleic acid encoding an amino acid sequence comprising the VL of the antibody and a second vector comprising a nucleic acid encoding an amino acid sequence comprising the VH of the antibody. In one embodiment, the host cell is eukaryotic, e.g., a Chinese hamster ovary (CHO) cell, a 293T cell, a NIH3T3 cell, or a lymphocyte (e.g., a YO, NSO, Sp20 cell).

In another embodiment, the host cell expresses the chimeric receptor described herein.

In another embodiment, the host cell comprises a T cell, a natural killer cell, a cytotoxic T lymphocyte, a natural killer T cell, a DNT cell, a regulatory T cell, a NK92 cell, and/or an immune effector cell comes from stem cell.

In another embodiment, the T cells come from natural T cells and/or T cells induced by pluripotent stem cells; preferably, the T cells are autologous/allogeneic T cells; preferably, the T cells are primary T cells; preferably, the T cells come from human autologous T cells.

In another embodiment, the T cells comprise stem cell-like memory T cells (Tscm cells), central memory T cells (Tcm), effector T cells (Tef), regulatory T cells (Tregs), effector memory T cells (Tern), γδ T cells, or a combination thereof.

In another embodiment, the host cell binds to cells expressing GPRC5D and does not significantly bind to cells that do not express GPRC5D.

In another embodiment, the host cell further carries coding sequence of an exogenous cytokine; and/or further expresses a chimeric receptor that non-targeting GPRC5D; and/or further expresses a chemokine; and/or further expresses a chemokine receptor; and/or further expresses a safety switch.

In another embodiment, the host cell further carries encoding sequences of exogenous cytokines including coding sequences of IL-7, IL-12, IL-15, IL-18, IL-21, or type I interferon.

In another embodiment, the host cell may also express a chimeric receptor that binds another antigen in addition to the receptor that binds GPRC5D described above.

In another embodiment, the host cell further expresses a chemokine including CCL19 or CCL21. In another embodiment, the host cell further expresses a chemokine receptor including CCR2, CCR4, CCR5, CXCR2, CXCR4 or CXCR5. In another embodiment, the host cell further expresses a safety switch including iCaspase-9, Truancated EGFR or RQR8.

In one embodiment, provided is a method of preparing an anti-GPRC5D antibody, wherein the method comprises culturing a host cell comprising a nucleic acid encoding the antibody under conditions suitable for expressing the antibody as described above, and optionally recovering the antibody from the host cell (or host cell culture medium).

In order to express the protein, the nucleic acid encoding the antibody of the present application can be integrated into an expression vector. A variety of expression vectors are available for protein expression. Expression vectors may comprise self-replicating extrachromosomal vectors, or vectors that integrate into the host genome. Expression vectors used in the present application include, but are not limited to, those that enable protein expression in mammalian cells, bacteria, insect cells, yeast, and in vitro systems. As is known in the art, a variety of expression vectors are available commercially or otherwise and can be used in the present application to express antibodies.

In a preferred embodiment, the host cell is administered in combination with an agent that enhances its function, preferably, in combination with a chemotherapeutic agent; and/or the host cell is administered in combination with an agent that improves one or more side effects associated with it; and/or the host cell is administered in combination with a host cell expressing a chimeric antigen receptor other than that targeting GPRC5D.

### Pharmaceutical composition

The antibodies, immunoconjugates, chimeric receptors, and host cells of the present application comprising the antibodies can be used to prepare pharmaceutical compositions or diagnostic reagents. In addition to an effective amount of the antibody, immunoconjugate, chimeric receptor, nucleic acid or host cell, the composition may also contain a pharmaceutically acceptable carrier.

The term "pharmaceutically acceptable" refers to the absence of adverse, allergic or other untoward reactions when the molecular entities and compositions are appropriately administered to animals or humans.

In some embodiments, the composition comprises an additional therapeutic agent. In some embodiments, the other therapeutic agent is a chemotherapeutic agent, such as those described in US20140271820 and/or a pharmaceutically acceptable salt or analog thereof. In some embodiments, the therapeutic agent includes, but is not limited to, mitotic inhibitors (vinca alkaloids), comprising vincristine, vinblastine, vindesine, and novibin (TM) (vinorelbine, 5'-dehydrogensulfide); topoisomerase I inhibitors, e.g. camptothecin compounds, comprising Camptosar^{™} (irinotecan HCl), Hycamtin^{™} (topotecan HCl), and other compounds derived from camptothecin and analogs thereof; podophyllotoxin derivatives, e.g. etoposide, teniposide, and midoxazolidinone; alkylating agents such as cisplatin, cyclophosphamide, nitrogen mustard, trimethylene thiophosphoramide, carmustine, busulfan, chlorambucil, briquinolizine, uracil mustard, cloprofen and dacarbazine; antimetabolites, including cytarabine, 5-fluorouracil, methotrexate, mercaptopurine, azathioprine and procarbazine; antibiotics, including but not limited to doxorubicin, bleomycin, dactinomycin, daunorubicin, mycin, mitomycin, sarkomycin C and daunomycin; and other chemotherapeutic agents, including but not limited to anti-tumor antibodies, dacarbazine, azacytidine, amsacon, melphalan, ifosfamide and mitoxantrone. In some embodiments, the additional therapeutic agent is selected from one or more of epirubicin, oxaliplatin, and 5-fluorouracil. In some embodiments, the additional therapeutic agents include, but are not limited to, anti-angiogenic agents, including anti-VEGF antibodies (including humanized and chimeric antibodies, anti-VEGF aptamers and antisense oligonucleotides), and other angiogenesis inhibitors, e.g. angiostatin, endostatin, interferon, interleukin 1 (including α and β) interleukin 12, retinoic acid, and tissue inhibitors of metalloproteinases-1 and -2, etc.

Specific examples of some substances that can serve as pharmaceutically acceptable carriers or their components are sugars, such as lactose, glucose and sucrose; starches, such as corn starch and potato starch; cellulose and derivatives thereof, such as sodium carboxymethylcellulose, ethylcellulose and methylcellulose; tragacanth powder; malt; gelatin; talc; solid lubricants, such as stearic acid and magnesium stearate; calcium sulfate; vegetable oils, such as peanut oil, cottonseed oil, sesame oil, olive oil, corn oil and cocoa butter; polyols, such as propylene glycol, glycerol, sorbitol, mannitol and polyethylene glycol; alginic acid; emulsifiers, such as Tween; wetting agents, such as sodium lauryl sulfate; colorants; flavorings; tableting agents, stabilizers; antioxidants; preservatives; pyrogen-free water; isotonic saline solution; and phosphate buffer, etc.

The pharmaceutical compositions described herein may comprise one or more pharmaceutically acceptable salts. A "pharmaceutically acceptable salt" refers to a salt that retains the desired biological activity of the parent compound and does not produce any adverse toxicological effects (see, e.g., Berge, S.M. et al., 1977, J. Pharm. Sci.66:1-19). Examples of such salts comprise acid addition salts and base addition salts.

Acid addition salts comprise those derived from nontoxic inorganic acids, such as hydrochloric acid, nitric acid, phosphoric acid, sulfuric acid, hydrobromic acid, hydroiodic acid, phosphorous acid, etc.; and salts derived from nontoxic organic acids, such as aliphatic monocarboxylic acids and dicarboxylic acids, phenyl-substituted alkanoic acids, hydroxyalkanoic acids, aromatic acids, aliphatic and aromatic sulfonic acids, etc. Base addition salts comprise salts derived from alkaline earth metals such as sodium, potassium, magnesium, calcium, and the like, as well as salts derived from non-toxic organic amines such as N,N'-dibenzylethylenediamine, N-methylglucamine, chloroprocaine, choline, diethanolamine, ethylenediamine, procaine, etc.

The pharmaceutical compositions described herein may also comprise an antioxidant. Examples of antioxidants include, but are not limited to: water-soluble antioxidants, such as ascorbic acid, cysteine hydrochloride, sodium bisulfate, sodium metabisulfite, sodium sulfite, etc.; oil-soluble antioxidants, such as ascorbyl palmitate, butylated hydroxyanisole (BHA), butylated hydroxytoluene (BHT), lecithin, propyl gallate, α-tocopherol, etc.; and metal chelators, such as citric acid, ethylenediaminetetraacetic acid (EDTA), sorbitol, tartaric acid, phosphoric acid, etc.

The composition of the present application can be formulated into various dosage forms as needed, and administered after determining a dose beneficial for a patient by a physician based on factors such as the patient type, age, body weight, general disease condition, and mode of administration, etc. The mode of administration may be, for example, parenteral administration (such as injection) or other treatment manners.

"Parenteral" administration of an immunogenic composition comprises, for example, subcutaneous (s.c.), intravenous (i.v.), intramuscular (i.m.) or intrasternal injection or infusion techniques.

In some embodiments, the compositions may be isotonic, i.e., they may have the same osmotic pressure as blood and tears. The desired isotonicity of the compositions of the present application can be achieved using sodium chloride, or other pharmaceutically acceptable agents such as glucose, boric acid, sodium tartrate, propylene glycol or other inorganic or organic solutes. If desired, the viscosity of the composition can be maintained at a selected level using a pharmaceutically acceptable thickener. Suitable thickeners comprise, for example, methylcellulose, xanthan gum, carboxymethylcellulose, hydroxypropylcellulose, carbomer, and the like. The preferred concentration of thickener will depend on the reagent selected. Obviously, the choice of appropriate carriers and other additives will depend on the the exact route of administration and the nature of the particular dosage form, e.g. a liquid dosage form.

### Kit

The present application also provides a kit comprising the antibody, immunoconjugate, chimeric receptor, nucleic acid or host cells described herein. In some embodiments, a kit may comprise a therapeutic or prophylactic composition containing an effective amount of an antibody, chimeric receptor, nucleic acid, or host cell described herein in one or more unit dosage forms. In some embodiments, the kit comprises a sterile container which may contain the therapeutic or prophylactic composition; such container may be in the form of a box, ampoule, bottle, vial, tube, bag, blister pack, or other suitable container forms known in the art. Such containers may be made of plastic, glass, laminated paper, metal foil, or other material suitable for holding the agent. In some embodiments, the kit comprises an antibody, immunoconjugate, chimeric receptor, nucleic acid, or host cell described herein, and instructions indicating administration of the antibody, immunoconjugate, chimeric receptor, nucleic acid, or host cell described herein to an individual. The instructions generally comprise methods of using the antibodies, immunoconjugates, chimeric receptors, nucleic acids or host cells described herein to treat or prevent cancer or tumors. In some embodiments, the kit comprises the host cell described herein, and can comprise about 1×10⁴ cells to about 1×10⁶ cells. In some embodiments, the kit can comprise at least about 1×10⁵ cells, at least about 1×10⁶ cells, at least about 1×10⁷ cells, at least about 4×10⁷ cells, at least about 5×10⁷ cells, at least about 6×10⁷ cells, at least about 6×10⁷ cells, 8×10⁷ cells, at least about 9×10⁷ cells, at least about 1× 10⁸ cells, at least about 2×10⁸ cells, at least about 3×10⁸ cells, at least about 4×10⁸ cells, at least about 5×10⁸ cells, at least about 6×10⁸ cells, at least about 6×10⁸ cells, at least about 8×10⁸ cells, at least about 9×10⁸ cells, at least about 1×10⁹ cells, at least about 2×10⁹ cells, at least about 3×10⁹ cells, at least about 4×10⁹ cells, at least about 5×10⁹ cells, at least about 6×10⁹ cells, at least about 8×10⁹ cells, at least about 9×10⁹ cells, at least about 1×10¹⁰ cells, at least about 2×10¹⁰ cells, at least about 3×10¹⁰ cells, at least about 4×10¹⁰ cells, at least about 5×10¹⁰ cells, at least about 6×10¹⁰ cells, at least about 7×10¹⁰ cells, at least about 8×10¹⁰ cells, At least about 9×10¹⁰ cells, at least about 1×10¹¹ cells, at least about 2×10¹¹ cells, at least about 3×10¹¹ cells, at least about 4×10¹¹ cells, at least about 5×10¹¹ cells, at least about 8×10¹¹ cells, at least about 9×10¹¹ cells, or at least about 1×10¹² cells. For example, approximately 5×10¹⁰ cells can be comprised in the kit. In another example, a kit can comprise 3×10⁶ cells; the cells can be expanded to about 5×10¹⁰ cells and administered to a subject.

In some embodiments, the kit can comprise allogeneic cells. In some embodiments, a kit can comprise cells that can comprise a genomic modification. In some embodiments, the kit may contain "ready-to-use" cells. In some embodiments, the kits can comprise cells that can be expanded for clinical use. In certain cases, the kit may contain contents for research purposes.

In some embodiments, the instructions comprise at least one of: a description of the therapeutic agent; dosage regimens and administration for treating or preventing a tumor or symptoms thereof; precautions, warnings, contraindications, overdose information, adverse reactions, animal pharmacology, clinical studies, and/or citations. The instructions may be printed directly on the container (if any), or as a label on the container, or as a separate sheet, booklet, card or folder provided within or in the container. In some embodiments, the instructions provide method for administering the antibodies described herein for treating or preventing tumors. In some cases, the instructions provide methods for administering an antibody of the present application before, after, or simultaneously with the administration of a chemotherapeutic agent.

### Methods for diagnosis/detection/treatment

The term "modulate" refers to a positive or negative change. Examples of modulations comprise changes of 1%, 2%, 10%, 25%, 50%, 75%, or 100%. In one embodiment, the change is negative.

The term "treatment" refers to interventions that attempt to change the course of a disease, either preventively or by intervening in the clinical pathology. The therapeutic effects include, but are not limited to, preventing the occurrence or recurrence of the disease, alleviating the symptoms, reducing any direct or indirect pathological consequences of the disease, preventing metastasis, slowing the progression of the disease, improving or alleviating the condition, relieving or improving prognosis, etc.

The term "prevention" refers to interventions that attempt to prevent disease (such as rejection of a cell transplant) before it develops.

The term "tumor antigen" refers to an antigen that emerges or is overexpressed during the development and progression of a hyperproliferative disease. In certain aspects, the hyperproliferative disease of the present application refers to a tumor.

The tumor antigen described in the present application may be a solid tumor antigen or a hematological tumor antigen.

The tumor antigens of the present application include but are not limited to: thyroid-stimulating hormone receptor (TSHR); CD171; CS-1; C-type lectin-like molecule-1; ganglioside GD3; Tn antigen; CD19; CD20; CD22; CD30; CD70; CD123; CD138; CD33; CD44; CD44v7/8; CD38; CD44v6; B7H3 (CD276), B7H6; KIT (CD117); interleukin 13 receptor subunit α (IL-13Rα); interleukin 11 receptor α (IL-11Rα); prostate stem cell antigen (PSCA); prostate specific membrane antigen (PSMA); carcinoembryonic antigen (CEA); NY-ESO-1; HIV-1 Gag; MART-1; gp100; tyrosinase; mesothelin; EpCAM; protease serine 21 (PRSS21); vascular endothelial growth factor receptor, vascular endothelial growth factor receptor 2 (VEGFR2); Lewis (Y) antigen; CD24; platelet-derived growth factor receptor β (PDGFR-β); stage-specific embryonic antigen-4 (SSEA-4); cell surface-associated mucin 1 (MUC1), MUC6; epidermal growth factor receptor family and its mutants (EGFR, EGFR2, ERBB3, ERBB4, EGFRvIII); neural cell adhesion molecule (NCAM); carbonic anhydrase IX (CAIX); LMP2; ephrin type A receptor 2 (EphA2); fucosyl GM1; sialyl Lewis adhesion molecule (sLe); ganglioside GM3; TGS5; high molecular weight melanoma-associated antigen (HMWMAA); o-acetyl GD2 ganglioside (OAcGD2); folate receptor; tumor endothelial marker 1 (TEM1/CD248); tumor endothelial marker 7-related (TEM7R); Claudin 6, Claudin18.2, Claudinl8.1; ASGPR1; CDH16; 5T4; 8H9; αvβ6 integrin; B cell maturation antigen (BCMA); CA9; κ light chain (kappa light chain); CSPG4; EGP2, EGP40; FAP; FAR; FBP; embryonic type AchR; HLA-A1, HLA-A2; MAGEA1, MAGE3; KDR; MCSP; NKG2D ligand; PSC1; ROR1; Sp17; SURVIVIN; TAG72; TEM1; fibronectin; tenascin; carcinoembryonic variant of tumor necrosis zone; G protein-coupled receptor class C group 5 member D (GPRC5D); X chromosome open reading frame 61 (CXORF61); CD97; CD179a; anaplastic lymphoma kinase (ALK); polysialic acid; placenta-specific 1 (PLAC1); hexose moiety of glycoceramide (GloboH); mammary differentiation antigen (NY-BR-1); uroplakin 2 (UPK2); hepatitis A virus cellular receptor 1 (HAVCR1); adrenergic receptor β3 (ADRB3); pannexin 3 (PANX3); G protein-coupled receptor 20 (GPR20); lymphocyte antigen 6 complex locus K9 (LY6K); olfactory receptor 51E2 (OR51E2); TCRγ alternate reading frame protein (TARP); Wilms tumor protein (WT1); ETS translocation variant gene 6 (ETV6-AML); sperm protein 17 (SPA17); X antigen family member 1A (XAGE1); angiopoietin binding cell surface receptor 2 (Tie2); melanoma cancer testis antigen-1 (MAD-CT-1); melanoma cancer testis antigen-2 (MAD-CT-2) T-2); Fos-related antigen 1; p53 mutant; human telomerase reverse transcriptase (hTERT); sarcoma translocation breakpoints; melanoma inhibitor of apoptosis (ML-IAP); ERG (transmembrane protease serine 2 (TMPRSS2) ETS fusion gene); N-acetylglucosaminyltransferase V (NA17); paired box protein Pax-3 (PAX3); androgen receptor; cyclin B1; V-myc avian myeloblastosis viral oncogene neuroblastoma-derived homolog (MYCN); Ras homolog family member C (RhoC); cytochrome P450 1B1 (CYP1B1); CCCTC-binding factor (zinc finger protein)-like (BORIS); squamous cell carcinoma antigen 3 recognized by T cells (SART3); paired box protein Pax-5 (PAX5); proacrosin binding protein sp32 (OYTES1); lymphocyte-specific protein tyrosine kinase (LCK); A kinase anchoring protein 4 (AKAP-4); synovial sarcoma X breakpoint 2 (SSX2); CD79a; CD79b; CD72; leukocyte-associated immunoglobulin-like receptor 1 (LAIR1); Fc fragment of IgA receptor (FCAR); leukocyte immunoglobulin-like receptor subfamily member 2 (LILRA2); CD300 molecule-like family member f (CD300LF); C-type lectin domain family 12 member A (CLEC12A); bone marrow stromal cell antigen 2 (BST2); mucin-like hormone receptor-like 2 containing an EGF-like module (EMR2); lymphocyte antigen 75 (LY75); glypican-3 (GPC3); Fc receptor-like 5 (FCRL5); immunoglobulin λ-like polypeptide 1 (IGLL1). Preferably, the tumor antigen is CS1, Claudin18.2, GPC3, BCMA or CD19.

The pathogen antigen is selected from the group consisting of: antigens of viruses, bacteria, fungi, protozoa, or parasites; the viral antigen is selected from the group consisting of: cytomegalovirus antigen, Epstein-Barr virus antigen, human immunodeficiency virus antigen, or influenza virus antigen.

As used herein, the term "individual" or "subject" refers to any animal, e.g. a mammal or marsupial. The individuals of the present application include, but are not limited to, humans, non-human primates (e.g., rhesus monkeys or other types of macaques), mice, pigs, horses, donkeys, cows, sheep, rats, and any kind of poultry.

As used herein, the term "effective amount" refers to an amount that provides a therapeutic or prophylactic benefit.

Any one of the anti-GPRC5D antibodies, immunoconjugates, chimeric receptor-modified host cells, pharmaceutical compositions or kits provided herein can be used in a therapeutic method.

In one aspect, provided is any one of the anti-GPRC5D antibodies, immunoconjugates, chimeric receptor-modified host cells, pharmaceutical compositions or kits for use as a medicament. In another aspect, provided is any one of the anti-GPRC5D antibodies, immunoconjugates, chimeric receptor-modified host cells, pharmaceutical compositions or kits for use in treating a disease. In certain embodiments, provided is any one of the anti-GPRC5D antibodies, immunoconjugates, chimeric receptor-modified host cells, pharmaceutical compositions or kits for use in a therapeutic method. In certain embodiments, the present application provides any one of the anti-GPRC5D antibodies, immunoconjugates, chimeric receptor-modified host cells, pharmaceutical compositions or kits for use in a method for treating an individual suffering from a disease, the method comprising administering to the individual an effective amount of any one of the anti-GPRC5D antibodies, immunoconjugates, chimeric receptor-modified host cells, pharmaceutical compositions or kits. In one embodiment, the method further comprises administering to the individual an effective amount of at least one additional therapeutic agent. The "individual" is preferably a human.

In another aspect, the present application provides use of any one of the anti-GPRC5D antibodies, immunoconjugates, chimeric receptor-modified host cells, pharmaceutical compositions or kits in preparation or formulation of a medicament. In one embodiment, the medicament is for treating a disease. In another embodiment, the medicament is for use in a method of treating a disease, the method comprising administering an effective amount of the medicament to a subject suffering from the disease. In one embodiment, the method further comprises administering to the individual an effective amount of at least one additional therapeutic agent. The "individual" is preferably a human.

In another aspect, the present application provides methods for treating a disease. In one embodiment, the method comprises administering an effective amount of any one of the anti-GPRC5D antibodies, immunoconjugates, chimeric receptor-modified host cells, pharmaceutical compositions or kits to an individual suffering from a disease expressing GPRC5D. In one embodiment, the method further comprises administering to the individual an effective amount of at least one additional therapeutic agent. The "individual" is preferably a human.

In another aspect, the present application provides a pharmaceutical formulation comprising any one of the anti-GPRC5D antibodies, immunoconjugates, chimeric receptor-modified host cells, pharmaceutical compositions or kits provided herein, for example for use in any of the above-mentioned treatment methods. In one embodiment, the pharmaceutical formulation comprises any one of the anti-GPRC5D antibodies, immunoconjugates, chimeric receptor-modified host cells, pharmaceutical compositions or kits provided herein and a pharmaceutically acceptable carrier. In another embodiment, the pharmaceutical formulation comprises any one of the anti-GPRC5D antibodies, immunoconjugates, chimeric receptor-modified host cells, pharmaceutical compositions or kits provided herein and at least one additional therapeutic agent.

In another aspect, the pharmaceutical formulation is for use in treating a disease. In one embodiment, the pharmaceutical formulation is administered to a sick individual. The "individual" in any of the above embodiments is preferably a human.

In another aspect, the present application provides a method for preparing an agent or pharmaceutical formulation, the method comprising mixing any one of the anti-GPRC5D antibodies, immunoconjugates, chimeric receptor-modified host cells, pharmaceutical compositions or kits provided herein with a pharmaceutically acceptable carrier, for example, for use in any of the above-mentioned therapeutic methods. In one embodiment, the method for preparing a medicament or pharmaceutical formulation further comprises adding at least one additional therapeutic agent to the medicament or pharmaceutical formulation.

Any one of the anti-GPRC5D antibodies, immunoconjugates, chimeric receptor-modified host cells, pharmaceutical compositions or kits of the present application can be used for treatment alone or in combination with other agents. Alternatively, any one of the anti-GPRC5D antibodies, immunoconjugates, chimeric receptor-modified host cells, pharmaceutical compositions or kits of the present application can be co-administered with at least one additional therapeutic agent.

Such combination therapy described above comprises combined administration (wherein two or more therapeutic agents are contained in the same or separate preparations) and separate administration, in which case, the administration of any one of the anti-GPRC5D antibodies, immunoconjugates, chimeric receptor-modified host cells, pharmaceutical compositions or kits of the present application may occur before, simultaneously with, and/or after the administration of the additional therapeutic agent or reagent. In one embodiment, administration of any one of the anti-GPRC5D antibodies, immunoconjugates, chimeric receptor-modified host cells, pharmaceutical compositions or kits of the present application and administration of the additional therapeutic agent occur within about one month, or within about one, two, or three weeks, or within about one, two, three, four, five, or six days of each other.

Any one of the anti-GPRC5D antibodies, immunoconjugates, chimeric receptor-modified host cells, pharmaceutical compositions or kits of the present application (and any additional therapeutic agent) can be administered by any suitable means, including parenteral administration, intrapulmonary administration or intranasal administration, and, if necessary for treatment, intralesional administration. Parenteral infusions comprise intramuscular administration, intravenous administration, intraarterial administration, intraperitoneal administration, or subcutaneous administration. Medication can be by any suitable approach, e.g., by injections, such as intravenous or subcutaneous injections, depending in part on whether the administration is brief or chronic. Various dosing regimens are contemplated herein, including, but not limited to, single administration or multiple administrations at various time points, push injection administration, and pulse infusion.

The preparation comprising a population of immunoreactive cells that is administered to an individual comprises a plurality of immunoreactive cells effective for treating and/or preventing a particular indication or disease. Thus, a therapeutically effective population of immunoreactive cells can be administered to an individual. Typically, a preparation containing about 1×10⁴ to about 1×10¹⁰ immunoreactive cells is administered. In most cases, the preparation will contain about 1×10⁵ to about 1×10⁹ immunoreactive cells, about 5×10⁵ to about 5×10⁸ immunoreactive cells, or about 1×10⁶ to about 1×10⁷ immunoreactive cells. However, the number of CAR-immunoreactive cells administered to an individual will vary between wide ranges depending on the location, origin, identity, extent and severity of the tumor, the age and physical condition of the individual to be treated, etc. The doctor will ultimately determine the appropriate dosage to be used.

In some embodiments, chimeric receptors are used to stimulate a host cell-mediated immune response. For example, a T cell-mediated immune response is an immune response involving the activation of T cells. Activated antigen-specific cytotoxic T cells are able to induce apoptosis in target cells displaying foreign antigen epitopes on their surfaces, e.g. cancer cells displaying tumor antigens. In other embodiments, chimeric antigen receptors are used to provide anti-tumor immunity in a mammal. The subject will develop anti-tumor immunity due to the T cell-mediated immune response.

In certain cases, a method of treating a subject having a tumor may involve administering one or more host cells of the present application to a subject in need of the treatment. The host cells can bind to tumor target molecules and induce cancer cell death. As described above, the present application also provides a method for treating a pathogen infection in an individual, comprising administering a therapeutically effective amount of the host cells of the present application to the individual.

The frequency with which the immunoreactive cells of the present application are administered will depend on factors including the disease being treated, the component of the particular immunoreactive cells, and the mode of administration. For example, the agent may be administered 4 times, 3 times, 2 times a day, or once a day, every other day, once every 3 days, once every 4 days, once every 5 days, once every 6 days, once a week, once every 8 days, once every 9 days, once every 10 days, once a week, or twice a month. As described herein, because the immune response cells of the present application have improved viability, they can be administered not only in a lower therapeutically effective amount than similar immune response cells that do not express exogenous type I interferon, but also in a lower frequency to obtain at least similar, and preferably more significant, therapeutic effects.

### Advantages of the present application:

The present application provides a fully human antibody that specifically recognizes GPRC5D, and CAR T cells prepared from the antibody show good killing effects on target cells both in vivo and in vitro.

The present application is further described below in conjunction with specific Examples. It should be understood that these Examples are only used to illustrate the present application but not to limit the scope of the present application. The experimental methods in the following Examples without specifying specific conditions are usually carried out according to conventional conditions such as the conditions described in J. Sambrook et al., Molecular Cloning Laboratory Manual, 3rd edition, Science Press, 2002, or the conditions recommended by the manufacturer.

### Example 1. Preparation of cell lines expressing GPRC5D

Human GPRC5D (amino acid sequence is shown as SEQ ID NO: 65) was transfected into CHOK1 (ATCC), 293T (ATCC), and NIH3T3 cells (ATCC) that do not express endogenous GPRC5D through lentivirus using conventional molecular biological techniques, and positive monoclonal clones were selected by limiting dilution method to construct CHOK1-huGPRC5D, 293T-huGPRC5D, and NIH3T3-huGPRC5D stably transfected cell lines.

CHOK1 cells were transfected by mouse GPRC5D (amino acid sequence is shown as SEQ ID NO: 66) via lentivirus using conventional molecular biological techniques, and positive clones were selected by limiting dilution method to construct a CHOK1-muGPRC5D stably transfected cell line.

### Example 2. Preparation of anti-GPRCSD hybridoma cell lines

The anti-GPRC5D hybridoma antibody was prepared using conventional hybridoma antibody preparation techniques in the art.

The full-length gene encoding human GPRC5D (SEQ ID No: 65) was constructed into the eukaryotic expression plasmid pCAGGS to form the eukaryotic expression plasmid pCAGGS-GPRC5D. Then, Balb/c mice (Vital River, female, 6-8 weeks old) were immunized four times using plasmid pCAGGS-GPRC5D and NIH3T3-huGPRC5D cells. The first three immunizations used 100 µg of plasmid pCAGGS-GPRC5D (diluted with saline, total volume 2 mL) and were completed within 5-8 seconds by tail vein high-dose injection; the fourth immunization used 2×10⁷ NIH3T3-huGPRC5D cells for intraperitoneal booster immunization. Three days later, the mouse spleens were taken and fused according to the published standard protocol (Kohler, Milstein, 1975). After HAT and flow cytometry specific screening, two hybridoma antibodies mIgG binding to human GPRC5D were obtained: AB1 (IgG2b-k) and AB2 (IgG3-k).

### Example 3. EC50 assay of binding of anti-GPRC5D hybridoma antibodies to CHOK1-huGPRC5D cells

2×10⁵ cells/well of CHOK1-huGPRC5D cells were plated in a 96-well round-bottom culture plate and washed twice with PBS containing 1% FBS; then hybridoma antibody was added with a starting concentration of 100µg/mL, and then 3-fold gradient dilution was performed 11 times, and incubated at 4°C for 45min; centrifuged at 500g for 5min, the supernatant was discarded, and the cells were washed twice with PBS containing 1% FBS; then Goat-anti-Mouse FITC (KANFCHEN, used at 1:200) was added 100µL/well, and incubated at 4°C for 45min; centrifuged at 500g for 5min, the supernatant was discarded, and the cells were washed twice with PBS containing 1% FBS, and then 200µL of PBS containing 1% FBS was added to resuspend the cells, and the results were detected by flow cytometry, and the results were statistically analyzed using FlowJo v.X.0.7 and the graphs were drawn using GraphPad Prism 8.0.

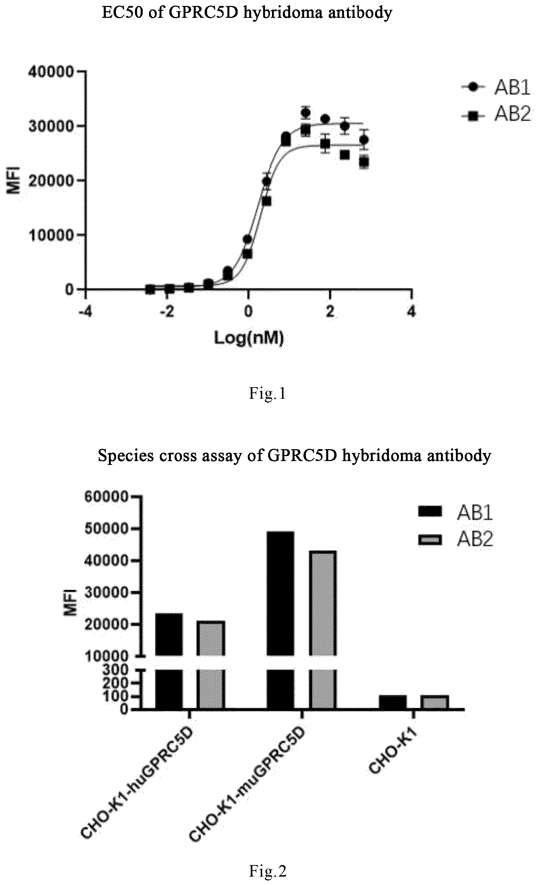

The results are shown in Fig. 1. The two hybridoma antibodies AB1 and AB2 have strong binding activity with CHOK1-huGPRC5D cells, with EC50 values of 1.692 nM and 1.930 nM, respectively. This indicates that both AB1 and AB2 can significantly bind to human GPRC5D.

### Example 4. Cross-reaction assay of anti-GPRC5D hybridoma antibodies with human GPRC5D and mouse GPRC5D

CHOK1 cells (as negative control cells), CHOK1-huGPRC5D cells, and CHOK1-muGPRC5D cells were respectively used to determine the cross-reactions of AB1 and AB2 with human GPRC5D and mouse GPRC5D according to the method in Example 3.

The results are shown in Fig. 2. Both hybridoma antibodies AB1 and AB2 significantly bound to human GPRC5D and mouse GPRC5D.

### Example 5. Obtaining the gene sequence of anti-GPRC5D hybridoma antibodies

RNA was extracted from hybridoma cells AB1 and AB2 and reverse transcribed to synthesize cDNA using conventional molecular biological techniques. PCRs were performed using UPM, mIgG2b-outer (SEQ ID NO: 67), mIgG3-outer (SEQ ID NO: 68), and mK-outer (SEQ ID NO: 69) primers, followed by cloning and sequencing to to obtain the AB1-VH gene sequence (SEQ ID NO: 36), AB1-VL gene sequence (SEQ ID NO: 38), AB2-VH gene sequence (SEQ ID NO: 40), and AB2-VL gene sequence (SEQ ID NO: 42).

The amino acid sequence of HCDR1 of AB1 was shown as SEQ ID NO:11, the amino acid sequence of HCDR2 of AB1 was shown as SEQ ID NO:12, the amino acid sequence of HCDR3 of AB1 was shown as SEQ ID NO:13, the amino acid sequence of LCDR1 of AB1 was shown as SEQ ID NO:14, the amino acid sequence of LCDR2 of AB1 was shown as SEQ ID NO:15, and the amino acid sequence of LCDR3 of AB1 was shown as SEQ ID NO:16. The amino acid sequence of the heavy chain variable region of AB1 was shown as SEQ ID NO:35, and the amino acid sequence of the light chain variable region of AB1 was shown as SEQ ID NO:37.

The amino acid sequence of HCDR1 of AB2 was shown as SEQ ID NO:17, the amino acid sequence of HCDR2 of AB2 was shown as SEQ ID NO:18, the amino acid sequence of HCDR3 of AB2 was shown as SEQ ID NO:19, the amino acid sequence of LCDR1 of AB2 was shown as SEQ ID NO:20, the amino acid sequence of LCDR2 of AB2 was shown as SEQ ID NO:21, and the amino acid sequence of LCDR3 of AB2 was shown as SEQ ID NO:22. The amino acid sequence of the heavy chain variable region of AB2 was shown as SEQ ID NO:39, and the amino acid sequence of the light chain variable region of AB2was shown as SEQ ID NO:41.

### Example 6. Binding activity assay of recombinant hybridoma scFv-mFc antibodies to cell lines expressing GPRC5D

The heavy chain and light chain gene sequences of AB1 and AB2 antibodies were cloned into a eukaryotic expression vector containing mFc (SEQ ID NO: 64) using molecular cloning technology to construct V152S-AB1-mFc and V152S-AB2-mFc plasmids, which were then transfected into 293F cells (Thermo Company) and cultured for 7 days before being purified by affinity chromatography to obtain anti-GPRC5D recombinant antibodies AB1 (scFv-mFc) and AB2 (scFv-mFc).

CHOK1-huGPRC5D, 293T-huGPRC5D, NIH3T3-huGPRC5D, and CHOK1 cells were taken respectively, and the binding activity of recombinant antibodies AB1 (scFv-mFc) and AB2 (scFv-mFc) to the cell line expressing human GPRC5D was assayed according to the method of Example 3.

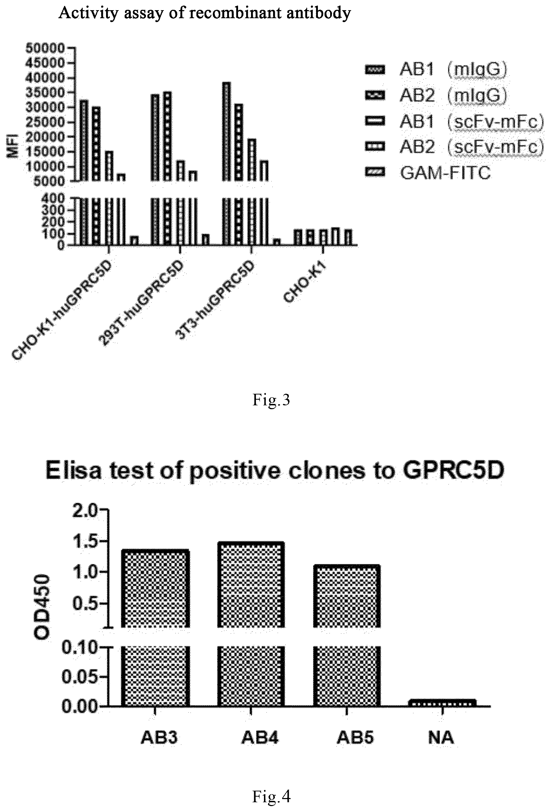

The results are shown in Fig. 3. Two recombinant antibodies AB1 (scFv-mFc) and AB2 (scFv-mFc) specifically bind to the cell line expressing human GPRC5D.

### Example 7. Screening and identification of GPRC5D antibodies

### 1. Screening of antibodies binding to human GPRC5D using a fully human phage display library

The phage display library used in the present application is a phage library constructed by our company, with a library capacity of 1E+11. Using human GPRC5D antigen (Kactus Biosystems), Fab fragments that specifically bind to human GPRC5D were obtained through screening methods known to those skilled in the art, and ultimately three antibodies that specifically bind to human GPRC5D were obtained and named AB3, AB4, and AB5.

The amino acid sequence of HCDR1 of AB3 was shown as SEQ ID NO:1, the amino acid sequence of HCDR2 of AB3 was shown as SEQ ID NO:2, the amino acid sequence of HCDR3 of AB3 was shown as SEQ ID NO:3, the amino acid sequence of LCDR1 of AB3 was shown as SEQ ID NO:4, the amino acid sequence of LCDR2 of AB3 was shown as SEQ ID NO:5, and the amino acid sequence of LCDR3 of AB3 was shown as SEQ ID NO:6. The amino acid sequence of the heavy chain variable region of AB3 was shown as SEQ ID NO:23, and the amino acid sequence of the light chain variable region of AB3 was shown as SEQ ID NO:25. The amino acid sequence of the heavy chain of AB3 was shown as SEQ ID NO:45, and the amino acid sequence of the light chain of AB3 was shown as SEQ ID NO:47.

The amino acid sequence of HCDR1 of AB4 was shown as SEQ ID NO:1, the amino acid sequence of HCDR2 of AB4 was shown as SEQ ID NO:2, the amino acid sequence of HCDR3 of AB4 was shown as SEQ ID NO:7, the amino acid sequence of LCDR1 of AB4 was shown as SEQ ID NO:4, the amino acid sequence of LCDR2 of AB4 was shown as SEQ ID NO:5, and the amino acid sequence of LCDR3 of AB4 was shown as SEQ ID NO:8. The amino acid sequence of the heavy chain variable region of AB4 was shown as SEQ ID NO:27, and the amino acid sequence of the light chain variable region of AB4 was shown as SEQ ID NO:29. The amino acid sequence of the heavy chain of AB4 was shown as SEQ ID NO:49, and the amino acid sequence of the light chain of AB4 was shown as SEQ ID NO:51.

The amino acid sequence of HCDR1 of AB5 was shown as SEQ ID NO:1, the amino acid sequence of HCDR2 of AB5 was shown as SEQ ID NO:2, the amino acid sequence of HCDR3 of AB5 was shown as SEQ ID NO:9, the amino acid sequence of LCDR1 of AB5 was shown as SEQ ID NO:4, the amino acid sequence of LCDR2 of AB5 was shown as SEQ ID NO:5, and the amino acid sequence of LCDR3 of AB5 was shown as SEQ ID NO: 10. The amino acid sequence of the heavy chain variable region of AB5 was shown as SEQ ID NO:31, and the amino acid sequence of the light chain variable region of AB5 was shown as SEQ ID NO:33. The amino acid sequence of the heavy chain of AB5 was shown as SEQ ID NO:53, and the amino acid sequence of the light chain of AB5 was shown as SEQ ID NO:55.

### 2. Specificity assay of antibodies using standard ELISA

50 µl of the supernatant of clones induced to express antibodies AB3, AB4, and AB5 were taken and incubated with 2 µg/ml human GPRC5D antigen at room temperature for 1 hour, and then the bound antibodies were detected by incubating with secondary antibody anti-Flag-HRP (Sigma, used at a dilution of 1:4000) at room temperature for 1 hour; TMB was then added for color development and the OD450 value was read using an microplate reader.

The results are shown in Fig. 4 (the blank group with no Fab antibody, i.e., NA), showing that antibodies AB3, AB4, and AB5 all specifically bound to the human GPRC5D antigen.

### 3. Specificity assay of antibodies binding to the target cells using FACs

50 µl of the supernatant of clones induced to express antibodies AB3, AB4 and AB5 were taken and incubated with 2×10⁵ 293T cells and 293T-huGPRC5D cells for 1 h. Then, the antibodies bound to the cells were fluorescently labeled and detected by secondary antibody anti-F(ab')₂-488 (Jackson ImmunoResearch, used at a dilution of 1:200). The experimental data were analyzed using FlowJo software to calculate the mean fluorescence intensity (MFI).

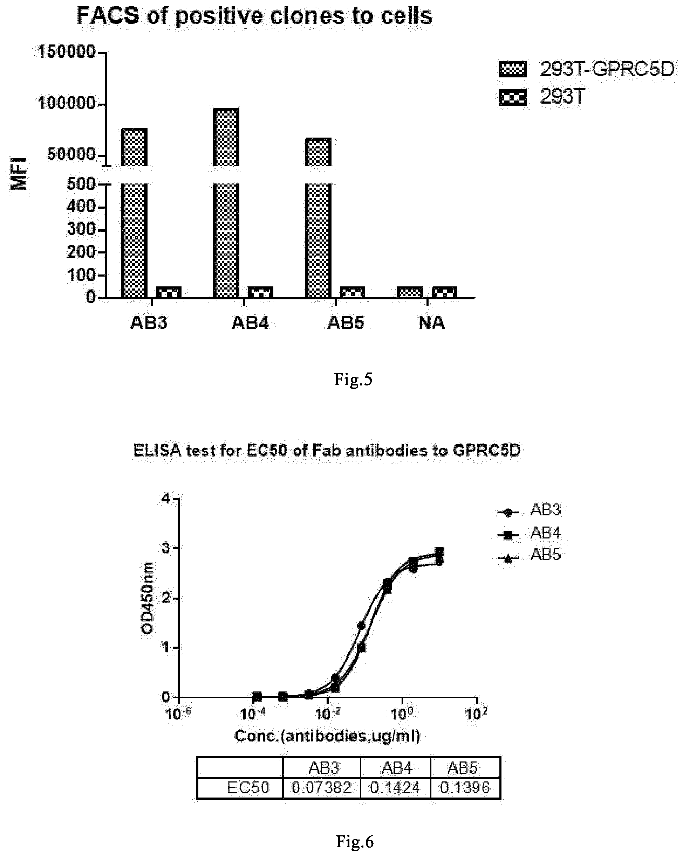

The results are shown in Fig. 5 (the blank group with no Fab antibody, i.e., NA), showing that antibodies AB3, AB4, and AB5 all specifically bound to 293T cells overexpressing human GPRC5D, but did not bind to 293T cells.

### Example 8. EC50 assay of the anti-GPRCSD-antibodies binding to the antigens using ELISA

The clones producing antibodies AB3, AB4, and AB5 in Example 7 were expressed respectively in prokaryotes and purified through a nickel column to obtain antibodies AB3, AB4, and AB5. The antibodies were then diluted with a 3-fold gradient from 10 µg/ml (8 gradients) and incubated with human GPRC5D antigen (coating concentration: 2 µg/ml) at room temperature for 1 h. The bound antibody was then detected by incubating at room temperature for 1 h with secondary antibody anti-Flag-HRP (Sigma, used at a dilution of 1:4000); TMB was then added for color development and the OD450 value was read using a microplate reader. The EC50 value was calculated by four-parameter fitting using GraphPad Prism5 software with the primary antibody concentration as the horizontal axis and the OD450 value as the vertical axis.

The results are shown in Fig. 6. Antibodies AB3, AB4, and AB5 all significantly bound to the human GPRC5D antigen, with EC50 values of 0.07382 µg/ml, 0.1424 µg/ml, and 0.1396 µg/ml, respectively.

### Example 9. Specific binding activity assay of the anti-GPRC5D antibodies to the target cells

Human lymphoma cells Daudi (Cell Bank of the Chinese Academy of Sciences) were cells that do not express endogenous GPRC5D; multiple myeloma cells MM.1S (Cell Bank of the Chinese Academy of Sciences) and NCI-H929 (ATCC) were cells that endogenously express GPRC5D.

The AB3, AB4 and AB5 antibodies (10 µg/ml) described in Example 7 were co-incubated with CHOK1, CHOK1-huGPRC5D, 293T, 293T-huGPRC5D, MM.1S, NCI-H929, and Daudi cells, respectively (no antibody was added to the blank group, i.e., NA), and the antibodies bound to the cells were fluorescently labeled using the secondary antibody anti-F(ab')₂-488 (Jackson ImmunoResearch, used at a dilution of 1:200), and then the fluorescence intensity was detected by a flow cytometer, and the experimental data were analyzed using FlowJo software to calculate the mean fluorescence intensity (MFI).

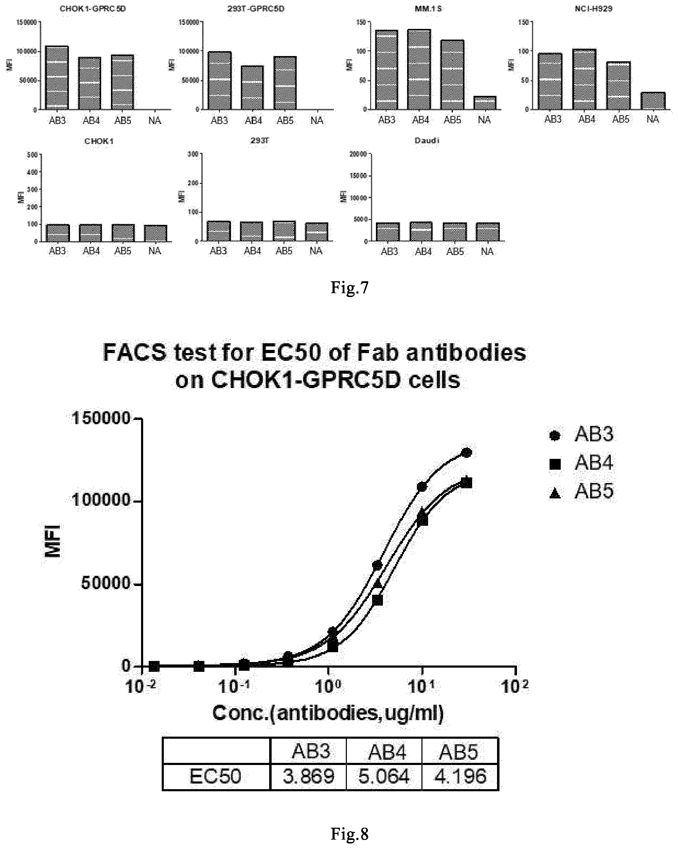

The detection results are shown in Fig. 7. AB3, AB4 and AB5 antibodies all significantly bound to cells expressing human GPRC5D (CHOK1-huGPRC5D, 293T-huGPRC5D, MM.1S and NCI-H929), but did not bind to cells not expressing GPRCSD (CHOK1, 293T and Daudi).

### Example 10. EC50 binding activity assay of anti-GPRC5D antibodies to cells expressing human GPRC5D

CHOK1-huGPRC5D cells and MM.1S cells were counted and plated on U-bottom plates, with about 2×10⁵ cells per well. The cells were then incubated with primary antibodies (AB3, AB4 or AB5 antibodies described in Example 7: starting at 30 µg/mL, 3-fold gradient dilutions for 8 gradients) and secondary antibodies (Anti-Fab-FITC: 1:200, Jackson ImmunoResearch), and then the fluorescence intensity was detected using a flow cytometry. The experimental data were analyzed using FlowJo software to calculate the mean fluorescence intensity (MFI), and then the EC50 value was calculated by four-parameter fitting using GraphPad PrismS software with the primary antibody concentration as the horizontal axis and the calibrated mean fluorescence intensity (MFI) as the vertical axis.

The results are shown in Fig.s 8 and 9. The EC50 of AB3, AB4, and AB5 antibodies binding to CHOK1-huGPRC5D cells were 3.869 µg/ml, 5.064 µg/ml, and 4.196 µg/ml, respectively; the EC50 of AB3, AB4, and AB5 binding to the MM.1S cells were 0.8512 µg/ml, 0.6578 µg/ml, and 0.8047 µg/ml, respectively.

### Example 11. Binding activity assay of anti-GPRC5D antibodies to CHOK1-muGPRC5D cells

The AB3, AB4 and AB5 antibodies (5 µg/ml) described in Example 7 were co-incubated with CHOK1-muGPRC5D cells (no antibody was added to the blank group, i.e., NA), and fluorescently labeled with secondary antibody anti-F(ab')2-488 (Jackson ImmunoResearch, used at a dilution of 1:200), and then the fluorescence intensity was detected by flow cytometry, and the experimental data were analyzed using FlowJo software to calculate the mean fluorescence intensity (MFI).

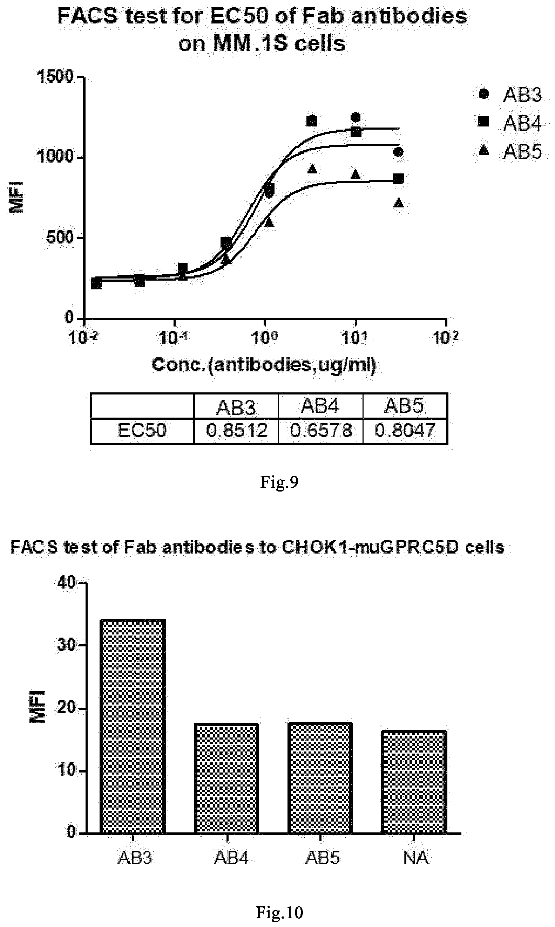

The test results are shown in Fig. 10, and the AB3 antibody can bind to mouse GPRC5D.

### Example 12. Preparation of GPRC5D CAR-T cells

### 1. Construction of the CAR vector

Using PRRLSIN-cPPT.EF-1α as the vector, a lentiviral plasmid expressing the second-generation chimeric antigen receptor of AB3 scFv (SEQ ID NO: 43) or the control antibody 18 scFv (SEQ ID NO: 57, sequence comes from patent CN107428829A) was constructed. It comprises CD8α signal peptide (SEQ ID NO: 58), CD8 hinge region (SEQ ID NO: 59) and the transmembrane region (SEQ ID NO: 60), CD137 intracellular signaling domain (SEQ ID NO: 61) and CD3ζ (SEQ ID NO: 62); the amino acid sequence of AB3-CAR was shown as SEQ ID NO: 63.

### 2. Preparation of GPRC5D CAR-T cells

The lentivirus was packaged using the calcium phosphate method, and the viral supernatant was purified using PEG8000/NaCl. After purification, the T cells activated by CD3/CD28 magnetic beads for 48 hours were infected to obtain AB3-BBZ CAR-T cells expressing AB3-BBZ and 18-BBZ CAR-T cells expressing 18-BBZ. T cells not infected with the virus were considered UTD. On the 5th day after infection, the CAR positivity rate was detected by FACS. The primary antibody was Biotin-anti-F(ab')₂-488 (Jackson ImmunoResearch, used at 1:100), and the secondary antibody was SA-PE (eBioscience, used at 1:200).

### Example 13. In vitro killing assay of GPRC5D CAR-T cells to target cells

First, the target cells MM.1S, 293T-huGPRCSD and 293T cells were adjusted to a density of 0.2×10⁶/mL respectively using AIM-V medium (AIM-V+2% ABS), and 50 µl was added to each well of a 96-well cell culture plate. According to the effector-target ratio of 3:1, 1:1, and 1:3, 50 µl of effector cells (CAR-T cells, UTD as the control) were added respectively. After incubation at 37°C for 16 hours, the supernatant was collected and detected using an LDH kit, and finally the OD490 reading was performed using an microplate reader.

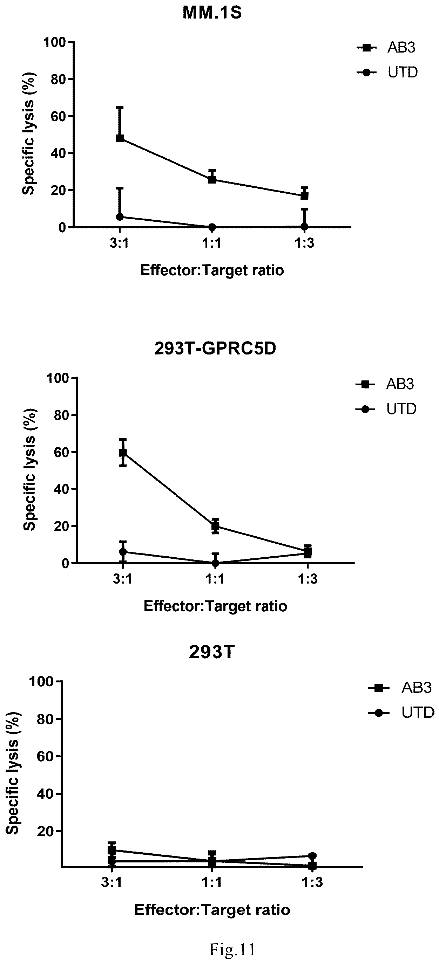

The results are shown in Fig. 11. AB3-BBZ CAR-T has no killing effect on 293T cells; however, it shows obvious killing effect on 293T-huGPRC5D and MM.1S cells expressing GPRC5D (the killing rate was as high as about 60% when the effector-target ratio was 3:1), and the killing effect was positively correlated with the effector-target ratio. The results showed that AB3-BBZ CAR-T had a specific in vitro killing effect on cells expressing GPRC5D.

### Example 14. In vivo anti-tumor ability of GPRC5D CAR-T cells

3×10⁶ human multiple myeloma cells MM.1S were inoculated subcutaneously in the right axilla of NPG female mice. The mice were divided into three groups (AB3-BBZ CAR T group, 18-BBZ CAR T group, and UTD group), with 8 mice in each group. The day of tumor cell inoculation was recorded as D0. On day 16 after inoculation, the average tumor volume was about 200mm³. AB3-BBZ CAR T, 18-BBZ CAR T, and UTD cells were injected into the tail vein at a dose of 3×10⁶ cells/mouse. On day 20 after CAR T cell injection (day 36 after tumor inoculation), the tumor volume of mice in the UTD group exceeded 2000mm³, and the experiment was terminated.

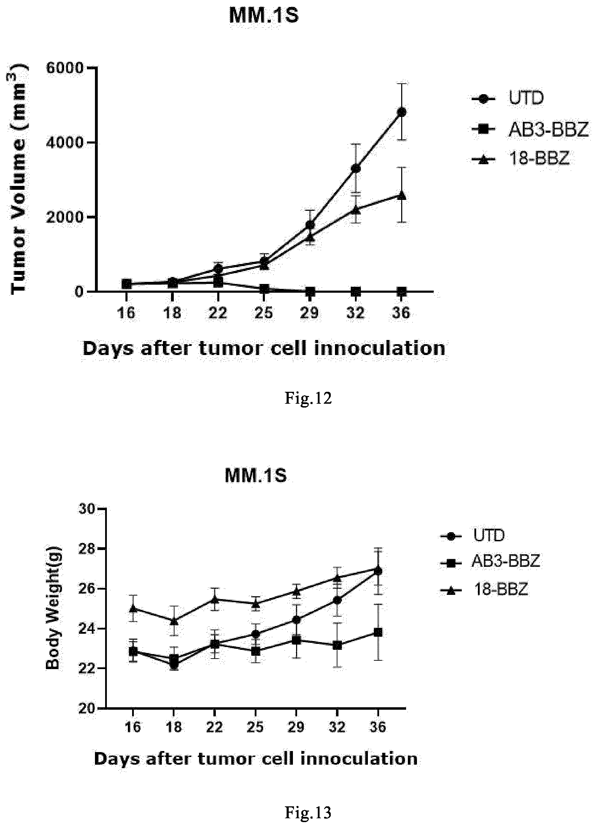

The results are shown in Fig. 12. The tumors of mice in the AB3-BBZ CAR-T group regressed on day 9 after CAR-T injection (day 25 after tumor inoculation), and the tumors of all 8 mice in the group regressed completely on day 13 after CAR-T injection (day 29 after tumor inoculation). In the 18-BBZ CAR-T control group, no mice showed tumor regression on day 20 after CAR-T injection (day 36 after tumor inoculation), and its tumor inhibition rate relative to the UTD group was only 46.02%. The results showed that AB3-BBZ CAR-T had a better anti-tumor effect in vivo than 18-BBZ CAR-T (P<0.0001). At the same time, the weight changes of mice in each treatment group were detected. The results are shown in Fig. 13.

### Example 15. Modified antibodies based on AB3

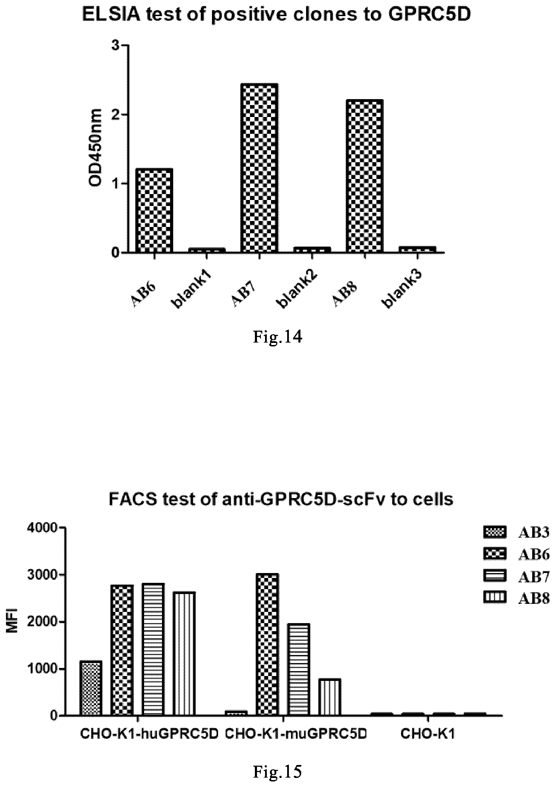

The light chain or heavy chain sequence of AB3 was randomly mutated to construct a phage library. The human GPRC5D antigen (Kactus Biosystems) was used to screen and obtain AB6, AB7 and AB8 antibodies. ELISA test showed that AB6, AB7 and AB8 had significant binding to human GPRC5D (Fig. 14). The sequencing results showed that the heavy chain variable region of AB6 was shown as SEQ ID NO:76, the heavy chain variable region of AB7 was shown as SEQ ID NO:23, the heavy chain variable region of AB8 was shown as SEQ ID NO:80, the light chain variable regions of AB6 and AB8 were shown in SEQ ID NO:25, and the light chain variable region of AB7 was shown as SEQ ID NO:78; the HCDR1 of AB6 was shown as SEQ ID NO:70, and the HCDR2 of AB6 was shown as SEQ ID NO:72, the HCDR1 of AB7 was shown as SEQ ID NO:1, and the HCDR2 of AB7 was shown as SEQ ID NO:2, the HCDR1 of AB8 was shown as SEQ ID NO:71, and the HCDR2 of AB8 was shown as SEQ ID NO:73, and the HCDR3 of AB6, AB7 and AB8 was shown as SEQ ID NO:3; the LCDR1 of AB6 and AB8 was shown as SEQ ID NO:4, and the LCDR2 of AB6 and AB8 was shown as SEQ ID NO:5, the LCDR1 of AB7 was shown as SEQ ID NO:74, and the LCDR2 of AB7 was shown as SEQ ID NO:75, and the LCDR3 of AB6, AB7 and AB8 was shown as SEQ ID NO:6.

### Example 16. Binding of modified antibodies to GPRC5D cell lines of different species

The three clones selected in Example 15 were expressed in prokaryotes and affinity purified using a nickel column to obtain purified single-chain antibodies (scFv) (shown as SEQ ID NO:82, SEQ ID NO:84, and SEQ ID NO:86, respectively), the C-terminus of which contained a his tag and a flag tag. CHO-K1-huGPRCSD and CHO-K1-muGPRCSD stably transfected cell lines were constructed according to the method of Example 1. The above-mentioned purified scFv (10ug/ml) was co-incubated with CHO-K1-huGPRCSD and CHO-K1-muGPRCSD cells and untransfected CHO-K1 cells, and the scFv bound to the cells was fluorescently labeled using the secondary antibody anti-Flag-488 (R&D, used at a dilution of 1:200). The fluorescence intensity was then detected by flow cytometry, and the experimental data were analyzed using FlowJo software to calculate the mean fluorescence intensity (MFI). As shown in Fig. 15, the detection results show that AB6, AB7 and AB8 have significantly higher binding abilities to cell lines expressing human GPRC5D and mouse GPRC5D than AB3, and do not bind to CHO-K1 cells that do not express GPRC5D. The results showed that antibodies AB6, AB7 and AB8 had good species binding properties and cell binding specificity.

### Example 17. EC50 assay of binding of anti-GPRC5D antibodies to GPRC5D expressing cell lines

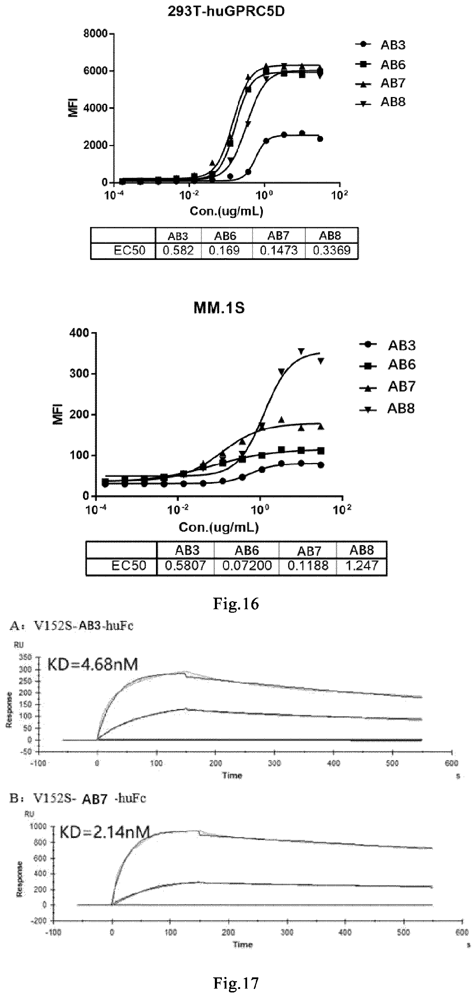

Using the 293T-huGPRCSD cells described in Example 1 and the cell line MM.1S endogenously expressing GPRC5D, the EC50 values of the antibodies described in Example 15 were determined according to the method of Example 10. The primary antibodies AB3 (Fab), AB6 (scFv), AB7 (scFv) and AB8 (scFv): starting from 30 µg/mL, 3-fold gradient dilutions for 12 gradients and incubated with the secondary antibody (Anti-Flag-488: 1:200, R&D). The results are shown in Fig. 16. The modified antibodies based on AB3 all have good cell binding activity, among which AB6 and AB7 have lower EC50s than that of AB3 on both cells.

### Example 18. Construction of anti-GPRC5D antibodies scFv-Fc format and its monomer rate assay

The anti-GPRC5D antibodies **(scFv)** AB3, AB6, AB7 and AB8 were fused with the Fc segment of human IgG (huFc, as shown as SEQ ID NO: 91) and transfected into 293F cells using 293Fectin transfection reagent. The supernatant was collected on day7 and affinity purified using Protein A filler. The purified protein passed through an empty GE XK16/40 chromatography column, the monomer peak was collected, and the monomer rate was detected. The results showed that the antibody monomer rates were all very high, with no significant differences. The antibody proteins were concentrated using a millipore ultrafiltration tube with a cut-off flow rate of 10KD, and the concentration was measured by OD280/extinction coefficient. The specific results are shown in Table 3.

**Table 3: Purification results of anti-GPRC5D antibodies scFv-Fc**

| Sample name | Extinction coefficient | Protein concentration (mg/ml) | Volume (ml) | amount of the proteins (mg) | purity (%) | Monomer rate (%) |
|---|---|---|---|---|---|---|
| AB3-huFc | 1.606 | 3.37 | 2.0 | 6.73 | 6.2 | 98.3 |
| AB6-huFc | 1.574 | 1.50 | 3.8 | 5.69 | 97.9 | 100.0 |
| AB7-huFc | 1.600 | 0.94 | 0.8 | 0.75 | 97.2 | 100.0 |
| AB8-huFc | 1.570 | 1.10 | 0.7 | 0.77 | 96.0 | 100.0 |

### Example 19. Affinity assay of Anti-GPRCSD Antibodies

The SA chip (Cat: BR100531) provided by CYTIVA was used to capture Bio-GPRC5D (Aero, Cat. No.GPD-H82D6)741.4Ru. The target antibody (AB3-huFc, AB7-huFc) was diluted at a 3-fold gradient starting from 150nM as the mobile phase, regenerated with Gly.HCl pH 1.7 for 180 s. Upon completion of the test, the affinity results were obtained by fitted using Evaluation Software. The affinity KD of AB3-huFc to Bio-GPRC5D was 4.68 nM; the affinity KD of AB7-huFc to Bio-GPRC5D was 2.14 nM (FIG. 17 ).

### Example 20. Construction of Chimeric Antigen Receptor (CAR) Plasmid

According to the method of Example 12, a lentiviral plasmid expressing the second-generation chimeric antigen receptors of AB6, AB7, and AB8 was constructed. The amino acid sequence of AB6-BBZ was shown as SEQ ID NO:88, the amino acid sequence of AB7-BBZ was shown as SEQ ID NO:89, and the amino acid sequence of AB8-BBZ was shown as SEQ ID NO:90.

### Example 21. Preparation of CAR-T cells and detection of their in vitro killing effect

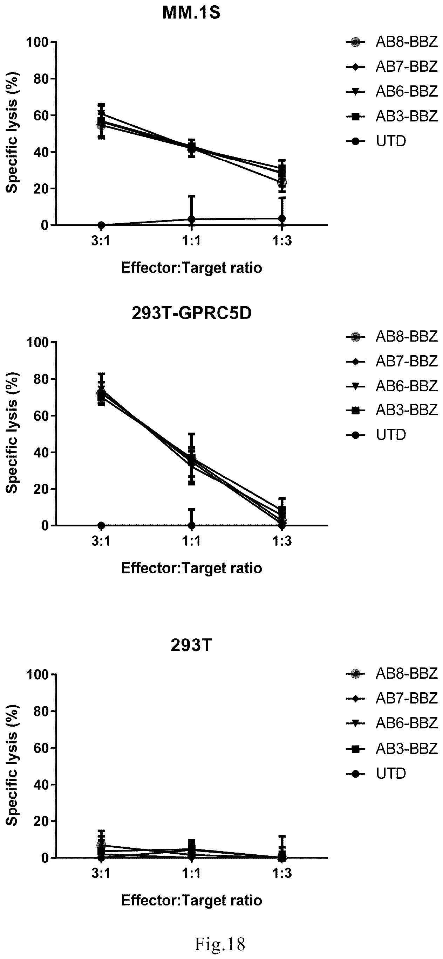

Using the CAR-expressing plasmids prepared in Examples 12 and 20, AB3-BBZ CAR-T cells, AB6-BBZ CAR-T cells, AB7-BBZ CAR-T cells, and AB8-BBZ CAR-T cells were prepared according to the method of Example 12, and the T cells not transfected with the virus were UTD. The cell killing experiment was performed according to the method of Example 13, the results are shown in Fig. 18. AB3 CAR-T, AB6 CAR-T, AB7 CAR-T, and AB8 CAR-T had no killing effect on 293T cells that did not express GPRC5D; while for MM.1S and 293T-huGPRC5D cells that expressed GPRC5D, they all showed obvious killing effects, and the killing effect was positively correlated with the effector-target ratio. The results showed that AB3 CAR-T, AB6 CAR-T, AB7 CAR-T and AB8 CAR-T had specific killing effects on cells expressing human GPRC5D.

All references mentioned in this application are cited as references in this application, just as each reference is cited separately as a reference. In addition, it should be understood that after reading the above teachings of this application, those skilled in the art may make various changes or modifications to this application, and these equivalent forms also fall within the scope defined by the claims attached to this application.

The sequences involved in this application are shown in the following table:

### Sequence Listing

| | | |
|---|---|---|
| 1 | Amino acid sequences of HCDR1 in AB3, AB4, AB5, and AB7 | SYAMS |
| 2 | Amino acid sequences of HCDR2 in AB3, AB4, AB5, and AB7 | AISGSGGSTYYADSVKG |
| 3 | Amino acid sequences of HCDR3 in AB3, | GWPSPVTFDY |
| | AB6, AB7, and AB8 | |
| 4 | Amino acid sequences of LCDR1 in AB3, AB4, AB5, AB6, and AB8 | RASQSVSSSYLA |
| 5 | Amino acid sequences of LCDR2 in AB3, AB4, AB5, AB6, and AB8 | GASSRAT |
| 6 | Amino acid sequences of LCDR3 in AB3, AB6, AB7, and AB8 | QQYKSHPIT |
| 7 | Amino acid sequence of HCDR3 in AB4 | GVRWVPLAFDY |
| 8 | Amino acid sequence of LCDR3 in AB4 | QQYGRSPMT |
| 9 | Amino acid sequence of HCDR3 in AB5 | GYPVPVSFDY |
| 10 | Amino acid sequence of LCDR3 inAB5 | QQYGNRPIT |
| 11 | Amino acid sequence of HCDR1 in AB1 | GYTMN |
| 12 | Amino acid sequence of HCDR2 in AB1 | LINPYNGGTSYNQKFKG |
| 13 | Amino acid sequence of HCDR3 in AB1 | GGFRAWFAF |
| 14 | Amino acid sequence of LCDR1 in AB1 | RASQDIGSNLN |
| 15 | Amino acid sequence of LCDR2 in AB1 | ATSILDS |
| 16 | Amino acid sequence of LCDR3 in AB1 | LQYATFPNT |
| 17 | Amino acid sequence of HCDR1 in AB2 | NYGMS |
| 18 | Amino acid sequence of HCDR2 in AB2 | TINSNGGNTYYPDSVKG |
| 19 | Amino acid sequence of HCDR3 in AB2 | AYSLTMDY |
| 20 | Amino acid sequence of LCDR1 in AB2 | RSSQSLVHSNGNTYLH |
| 21 | Amino acid sequence of LCDR2 in AB2 | KVSNRFS |
| 22 | Amino acid sequence of LCDR3 in AB2 | SQSTHVPPT |
| 23 | Amino acid sequences of VH in AB3 and AB7 | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSS |
| 24 | Polynucleotide sequences of VH in AB3 and AB7 | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTGGCCATCTCCAGTTA CTTTCGACTACTGGGGCCAAGGAACCCTGGTCACCGTCTCGAGT |
| 25 | Amino acid sequences of VL in AB3, AB6, and AB8 | EIVLTQSPGTLSLSPGERATLSCRASQSVSSSYLAWYQQKPGQAPRLLIYGASSRA TGIPDRFSGSGSGTDFTLTISRLEPEDFAVYYCQQYKSHPITFGQGTKVEIKR |
| 26 | Base sequences of VL in AB3, AB6, and AB 8 | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACAAATCTCATCCAATCACGTTCGGCCAGGGGACCAA AGTGGAAATCAAACGT |
| 27 | Amino acid sequence of VH | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCAKGVRWVPLAF |
| | in AB4 | DYWGQGTLVTVSS |
| 28 | Polynucleotide sequence of VH in AB4 | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGAAAGGTGTTCGTTGGGTTCCA CTGGCATTCGACTACTGGGGCCAAGGAACCCTGGTCACCGTCTCGAGT |
| 29 | Amino acid sequence of VL in AB4 | EIVLTQSPGTLSLSPGERATLSCRASQSVSSSYLAWYQQKPGQAPRLLIYGASSRA TGIPDRFSGSGSGTDFTLTISRLEPEDFAVYYCQQYGRSPMTFGQGTKVEIKR |
| 30 | Polynucleotide sequence of VL in AB4 | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACGGTAGATCTCCAATGACGTTCGGCCAGGGGACCAA AGTGGAAATCAAACGT |
| 31 | Amino acid sequence of VH in AB5 | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGYPVPVSFD YWGQGTLVTVSS |
| 32 | Polynucleotide sequence of VH in AB5 | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTACCCAGTTCCAGTTT CTTTCGACTACTGGGGCCAAGGAACCCTGGTCACCGTCTCGAGT |
| 33 | Amino acid sequence of VL in AB5 | EIVLTQSPGTLSLSPGERATLSCRASQSVSSSYLAWYQQKPGQAPRLLIYGASSRA TGIPDRFSGSGSGTDFTLTISRLEPEDFAVYYCQQYGNRPITFGQGTKVEIKR |
| 34 | Polynucleotide sequence of VL in AB5 | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACGGTAACAGACCAATCACGTTCGGCCAGGGGACCAA AGTGGAAATCAAACGT |
| 35 | Amino acid sequence of VH in AB1 | EVQLQQSGPEVVKPGASMKISCKASGYSFTGYTMNWVKQSHGKNFEWIGLINP YNGGTSYNQKFKGKATLTVDKSSSTAYMELLSLTSEDSAVYYCTRGGFRAWFAF WGQGTLVTVSA |
| 36 | Polynucleotide sequence of VH in AB1 | GAGGTCCAGCTGCAACAGTCTGGACCTGAGGTGGTGAAGCCTGGAGCTTCA ATGAAGATATCCTGCAAGGCTTCTGGTTACTCATTCACTGGCTACACCATGAA CTGGGTGAAGCAGAGCCATGGAAAGAATTTTGAGTGGATTGGACTTATTAAT CCTTACAATGGTGGTACTAGTTACAACCAGAAATTCAAGGGCAAGGCCACAT TAACTGTGGACAAGTCATCCAGCACAGCCTACATGGAACTCCTCAGTCTGAC ATCTGAGGACTCTGCAGTCTATTATTGTACAAGAGGAGGATTTCGAGCCTGGT TTGCTTTCTGGGGCCAGGGGACTCTGGTCACTGTCTCTGCA |
| 37 | Amino acid sequence of VL in AB1 | DIQMTQSPSSLSASLGERVSLTCRASQDIGSNLNWLQQEPDGTIKRLIYATSILDS GVPKRFSGSRSGSDYSLTISSLESEDFVDYYCLQYATFPNTFGGGTKLEIKR |
| 38 | Polynucleotide sequence of VL in AB1 | GACATCCAGATGACCCAGTCTCCATCCTCCTTATCTGCCTCTCTGGGAGAAAG AGTCAGTCTCACTTGTCGGGCAAGTCAGGACATTGGTAGTAACTTAAACTGG CTTCAGCAGGAACCAGATGGAACTATTAAACGCCTGATCTACGCCACATCCAT TTTAGATTCTGGTGTCCCCAAAAGGTTCAGTGGCAGTAGGTCTGGGTCAGAT TATTCTCTCACCATCAGCAGCCTTGAGTCTGAAGATTTTGTAGACTATTACTGT CTACAATATGCTACTTTTCCGAACACGTTCGGAGGGGGGACCAAGCTGGAAA TAAAACGG |
| 39 | Amino acid sequence of VH in AB2 | VVKLVESGGGLVQPGGSLKLSCAASGFTFSNYGMSWVRQTPDTRLELVATINSN GGNTYYPDSVKGRFTISRDNAKNTLYLQMSSLKSEDTAIYYCVRAYSLTMDYW GQGTSVTVSS |
| 40 | Polynucleotide sequence of VH in AB2 | GTGGTGAAGCTGGTGGAGTCGGGGGGAGGCTTAGTGCAGCCTGGAGGGTCC CTGAAACTCTCCTGTGCAGCCTCTGGATTCACTTTCAGTAACTATGGCATGTC TTGGGTTCGCCAGACTCCAGACACGAGGCTGGAGTTGGTCGCAACCATTAAT AGTAATGGTGGTAACACCTATTATCCAGACAGTGTGAAGGGCCGATTCACCAT CTCCAGAGACAATGCCAAGAACACCCTGTACCTGCAAATGAGCAGTCTGAA GTCTGAGGACACAGCCATATATTACTGTGTTAGAGCTTACTCCCTGACTATGG ACTACTGGGGTCAAGGAACCTCAGTCACCGTCTCCTCA |
| 41 | Amino acid sequence of VL in AB2 | DVVMTQTPLSLPVSLGDQASISCRSSQSLVHSNGNTYLHWYLQKPGQSPKLLIY KVSNRFSGVPDRFSGSGSGTDFTLKIIRVEAEDLGVYFCSQSTHVPPTFRAGTKL ELKR |
| 42 | Polynucleotide sequence of VL in AB2 | GATGTTGTGATGACCCAAACTCCACTCTCCCTGCCTGTCAGTCTTGGAGATCA AGCCTCCATCTCTTGTAGATCTAGTCAGAGCCTTGTACACAGTAATGGAAACA CCTATTTACATTGGTACCTGCAGAAGCCAGGCCAGTCTCCAAAGCTCCTGATC TACAAAGTTTCCAACCGATTTTCTGGGGTCCCAGACAGGTTCAGTGGCAGTG GATCAGGGACAGATTTCACACTCAAGATCATCAGAGTGGAGGCTGAGGATCT GGGAGTTTATTTCTGCTCTCAAAGTACACATGTTCCTCCTACGTTCCGTGCTG GGACCAAGCTGGAGCTGAAACGG |
| 43 | Amino acid sequence of AB3 scFv | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSSSYLAWYQQKPGQAPRLLIYGASSRATGIPDRFSGSGSGTDFTLTISRLEPED FAVYYCQQYKSHPITFGQGTKVEIKR |
| 44 | Polynucleotide sequence of AB3 | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG |
| | scFv | CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTGGCCATCTCCAGTTA CTTTCGACTACTGGGGCCAAGGAACCCTGGTCACCGTCTCGAGTGGTGGAG GCGGTTCAGGCGGAGGTGGTTCTGGCGGTGGCGGATCGGAAATCGTGTTAAC GCAGTCTCCAGGCACCCTGTCTTTGTCTCCAGGGGAAAGAGCCACCCTCTCT TGCAGGGCCAGTCAGAGTGTTAGCAGCAGCTACTTAGCCTGGTACCAGCAGA AACCTGGCCAGGCTCCCAGGCTCCTCATCTATGGAGCATCCAGCAGGGCCAC TGGCATCCCAGACAGGTTCAGTGGCAGTGGATCCGGGACAGACTTCACTCTC ACCATCAGCAGACTGGAGCCTGAAGATTTTGCAGTGTATTACTGTCAGCAGT ACAAATCTCATCCAATCACGTTCGGCCAGGGGACCAAAGTGGAAATCAAAC GT |
| 45 | Amino acid sequence of the heavy chain of AB3 Fab | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCD |
| 46 | Polynucleotide sequence of the heavy chain of AB3 Fab | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTGGCCATCTCCAGTTA CTTTCGACTACTGGGGCCAAGGAACCCTGGTCACCGTCTCGAGTGCTAGCAC CAAAGGCCCATCGGTCTTCCCCCTGGCACCCTCCTCCAAGAGCACCTCTGGG GGCACAGCGGCCCTGGGCTGCCTGGTCAAGGACTACTTCCCCGAACCGGTG ACGGTGTCGTGGAACTCAGGCGCCCTGACCAGCGGCGTGCACACCTTCCCG GCTGTCCTACAGTCCTCAGGACTCTACTCCCTCAGCAGCGTGGTGACCGTGC CCTCCAGCAGCTTGGGCACCCAGACCTACATCTGCAACGTGAATCACAAGCC CAGCAACACCAAAGTGGACAAGAAAGTTGAGCCCAAATCTTGTGAC |
| 47 | Amino acid sequence of the light chain of AB3 Fab | EIVLTQSPGTLSLSPGERATLSCRASQSVSSSYLAWYQQKPGQAPRLLIYGASSRA TGIPDRFSGSGSGTDFTLTISRLEPEDFAVYYCQQYKSHPITFGQGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| 48 | Polynucleotide sequence of the light chain of AB3 Fab | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACAAATCTCATCCAATCACGTTCGGCCAGGGGACCAA AGTGGAAATCAAACGTACGGTGGCTGCACCATCTGTCTTCATCTTCCCGCCAT CTGATGAGCAGTTGAAATCTGGAACTGCCTCTGTTGTGTGCCTGCTGAATAA CTTCTATCCCAGAGAGGCCAAAGTACAGTGGAAGGTGGATAACGCCCTCCAA |
| | | TCGGGTAACTCCCAGGAGAGTGTCACAGAGCAGGACAGCAAGGACAGCAC CTACAGCCTCAGCAGCACCCTGACGCTGAGCAAAGCAGACTACGAGAAACA CAAAGTCTACGCCTGCGAAGTCACCCATCAGGGCCTGAGCTCGCCCGTCACA AAGAGCTTCAACAGGGGAGAGTGT |
| 49 | Amino acid sequence of the heavy chain of AB4 Fab | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCAKGVRWVPLAF DYWGQGTLVTVSSASTKGPSVFPLAPSSKSTSGGTAALGCLVKDYFPEPVTVSW NSGALTSGVHTFPAVLQSSGLYSLSSVVTVPSSSLGTQTYICNVNHKPSNTKVDK KVEPKSCD |
| 50 | Polynucleotide sequence of the heavy chain of AB4 Fab | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGAAAGGTGTTCGTTGGGTTCCA CTGGCATTCGACTACTGGGGCCAAGGAACCCTGGTCACCGTCTCGAGTGCTA GCACCAAAGGCCCATCGGTCTTCCCCCTGGCACCCTCCTCCAAGAGCACCTC TGGGGGCACAGCGGCCCTGGGCTGCCTGGTCAAGGACTACTTCCCCGAACC GGTGACGGTGTCGTGGAACTCAGGCGCCCTGACCAGCGGCGTGCACACCTT CCCGGCTGTCCTACAGTCCTCAGGACTCTACTCCCTCAGCAGCGTGGTGACC GTGCCCTCCAGCAGCTTGGGCACCCAGACCTACATCTGCAACGTGAATCACA AGCCCAGCAACACCAAAGTGGACAAGAAAGTTGAGCCCAAATCTTGTGAC |
| 51 | Amino acid sequence of the light chain of AB4 Fab | EIVLTQSPGTLSLSPGERATLSCRASQSVSSSYLAWYQQKPGQAPRLLIYGASSRA TGIPDRFSGSGSGTDFTLTISRLEPEDFAVYYCQQYGRSPMTFGQGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| 52 | Polynucleotide sequence of the light chain of AB4 Fab | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACGGTAGATCTCCAATGACGTTCGGCCAGGGGACCAA AGTGGAAATCAAACGTACGGTGGCTGCACCATCTGTCTTCATCTTCCCGCCAT CTGATGAGCAGTTGAAATCTGGAACTGCCTCTGTTGTGTGCCTGCTGAATAA CTTCTATCCCAGAGAGGCCAAAGTACAGTGGAAGGTGGATAACGCCCTCCAA TCGGGTAACTCCCAGGAGAGTGTCACAGAGCAGGACAGCAAGGACAGCAC CTACAGCCTCAGCAGCACCCTGACGCTGAGCAAAGCAGACTACGAGAAACA CAAAGTCTACGCCTGCGAAGTCACCCATCAGGGCCTGAGCTCGCCCGTCACA AAGAGCTTCAACAGGGGAGAGTGT |
| 53 | Amino acid sequence of the heavy chain of AB5 Fab | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGYPVPVSFD YWGQGTLVTVSSASTKGPSVFPLAPSSKSTSGGTAALGCLVKDYFPEPVTVSWN SGALTSGVHTFPAVLQSSGLYSLSSVVTVPSSSLGTQTYICNVNHKPSNTKVDKK VEPKSCD |
| 54 | Polynucleotide sequence of the heavy chain of AB5 Fab | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTACCCAGTTCCAGTTT CTTTCGACTACTGGGGCCAAGGAACCCTGGTCACCGTCTCGAGTGCTAGCAC CAAAGGCCCATCGGTCTTCCCCCTGGCACCCTCCTCCAAGAGCACCTCTGGG GGCACAGCGGCCCTGGGCTGCCTGGTCAAGGACTACTTCCCCGAACCGGTG ACGGTGTCGTGGAACTCAGGCGCCCTGACCAGCGGCGTGCACACCTTCCCG GCTGTCCTACAGTCCTCAGGACTCTACTCCCTCAGCAGCGTGGTGACCGTGC CCTCCAGCAGCTTGGGCACCCAGACCTACATCTGCAACGTGAATCACAAGCC CAGCAACACCAAAGTGGACAAGAAAGTTGAGCCCAAATCTTGTGAC |
| 55 | Amino acid sequence of the light chain of AB5 Fab | EIVLTQSPGTLSLSPGERATLSCRASQSVSSSYLAWYQQKPGQAPRLLIYGASSRA TGIPDRFSGSGSGTDFTLTISRLEPEDFAVYYCQQYGNRPITFGQGTKVEIKRTVA APSVFIFPPSDEQLKSGTASVVCLLNNFYPREAKVQWKVDNALQSGNSQESVTE QDSKDSTYSLSSTLTLSKADYEKHKVYACEVTHQGLSSPVTKSFNRGEC |
| 56 | Polynucleotide sequence of the light chain of AB5 Fab | GAAATCGTGTTAACGCAGTCTCCAGGCACCCTGTCTTTGTCTCCAGGGGAAA GAGCCACCCTCTCTTGCAGGGCCAGTCAGAGTGTTAGCAGCAGCTACTTAGC CTGGTACCAGCAGAAACCTGGCCAGGCTCCCAGGCTCCTCATCTATGGAGCA TCCAGCAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACGGTAACAGACCAATCACGTTCGGCCAGGGGACCAA AGTGGAAATCAAACGTACGGTGGCTGCACCATCTGTCTTCATCTTCCCGCCAT CTGATGAGCAGTTGAAATCTGGAACTGCCTCTGTTGTGTGCCTGCTGAATAA CTTCTATCCCAGAGAGGCCAAAGTACAGTGGAAGGTGGATAACGCCCTCCAA TCGGGTAACTCCCAGGAGAGTGTCACAGAGCAGGACAGCAAGGACAGCAC CTACAGCCTCAGCAGCACCCTGACGCTGAGCAAAGCAGACTACGAGAAACA CAAAGTCTACGCCTGCGAAGTCACCCATCAGGGCCTGAGCTCGCCCGTCACA AAGAGCTTCAACAGGGGAGAGTGT |
| 57 | Amino acid sequence of 18 scFv | EVQLVESGGGLIQPGGSLRLSCAASGFTFSNYAMNWVRQAPGKGLEWVSTING RGSSTIYADSVKGRFTISRDNSKNTLYLQMNSLRAEDTATYYCARYISRGLGDS WGQGTLVTVSSGGGGSGGGGSGGGGSQSVVTQPPSMSAAPGQQVTISCSGGNS NIERNYVSWYLQLPGTAPKLVIFDNDRRPSGIPDRFSGSKSGTSATLGITGLQTGD EADYYCGTWDSSLRGWVFGGGTKLTVLG |
| 58 | Amino acid sequence of CD8α signaling peptide | MALPVTALLLPLALLLHAARP |
| 59 | Amino acid sequence of CD8α hinge region | TTTPAPRPPTPAPTIASQPLSLRPEACRPAAGGAVHTRGLDFACD |
| 60 | Amino acid | IYIWAPLAGTCGVLLLSLVIT LYC |
| | sequence of CD8 transmembrane region | |
| 61 | Amino acid sequence of CD137 intracellular signaling domain | KRGRKKLLYIFKQPFMRPVQTTQEEDGCSCRFPEEEEGGCEL |
| 62 | Amino acid sequence of CD3ζ | RVKFSRSADAPAYQQGQNQLYNELNLGRREEYDVLDKRRGRDPEMGGKPQRR KNPQEGLYNELQKDKMAEAYSEIGMKGERRRGKGHDGLYQGLSTATKDTYDA LHMQALPPR |
| 63 | Amino acid sequence of AB3-BBZ | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSSSYLAWYQQKPGQAPRLLIYGASSRATGIPDRFSGSGSGTDFTLTISRLEPED FAVYYCQQYKSHPITFGQGTKVEIKRTTTPAPRPPTPAPTIASQPLSLRPEACRPAA GGAVHTRGLDFACDIYIWAPLAGTCGVLLLSLVITLYCKRGRKKLLYIFKQPFMR PVQTTQEEDGCSCRFPEEEEGGCELRVKFSRSADAPAYQQGQNQLYNELNLGRR EEYDVLDKRRGRDPEMGGKPQRRKNPQEGLYNELQKDKMAEAYSEIGMKGER RRGKGHDGLYQGLSTATKDTYDALHMQALPPR |
| 64 | Amino acid sequence of mFc | RDCGCKPCICTVPEVSSVFIFPPKPKDVLTITLTPKVTCVVVDISKDDPEVQFSWF VDDVEVHTAQTQPREEQFNSTFRSVSELPIMHQDWLNGKEFKCRVNSAAFPAPI EKTISKTKGRPKAPQVYTIPPPKEQMAKDKVSLTCMITDFFPEDITVEWQWNGQ PAENYKNTQPIMDTDGSYFVYSKLNVQKSNWEAGNTFTCSVLHEGLHNHHTE KSLSHSPGK |
| 65 | Amino acid sequence of human GPRC5D | MYKDCIESTGDYFLLCDAEGPWGIILESLAILGIVVTILLLLAFLFLMRKIQDCSQ WNVLPTQLLFLLSVLGLFGLAFAFIIELNQQTAPVRYFLFGVLFALCFSCLLAHAS NLVKLVRGCVSFSWTTILCIAIGCSLLQIIIATEYVTLIMTRGMMFVNMTPCQLN VDFVVLLVYVLFLMALTFFVSKATFCGPCENWKQHGRLIFITVLFSIIIWVVWIS MLLRGNPQFQRQPQWDDPVVCIALVTNAWVFLLLYIVPELCILYRSCRQECPLQ GNACPVTAYQHSFQVENQELSRARDSDGAEEDVALTSYGTPIQPQTVDPTQECFI PQAKLSPQQDAGGV |
| 66 | Amino acid sequence of mouse GPRC5D | MYEDCVKSTEDYYLFCDNEGPWAIVLESLAVIGIVVTILLLLAFLFLMRKVQDC SQWNVLPTQFLFLLAVLGLFGLTFAFIIQLNHQTAPVRYFLFGVLFAICFSCLLAH ASNLVKLVRGRVSFCWTTILFIAIGVSLLQTIIAIEYVTLIMTRGLMFEHMTPYQL NVDFVCLLIYVLFLMALTFFVSKATFCGPCENWKQHGRLIFATVLVSIIIWVVWIS MLLRGNPQLQRQPHWDDAVICIGLVTNAWVFLLIYIIPELSILYRSCRQECPTQG NVCQVPVYQRSFRMDTQEPTREC |
| 67 | mIgG2b-outer primer | ACACTGCTGGACAGGGATCC |
| 68 | mIgG3-outer primer | ACAGGGCTCCATAGTTCCATTTTAC |
| 69 | mK-outer primer | AGGTGCTGTCTTTGCTGTCCTG |
| 70 | Amino acid | RRYAIS |
| | sequence of AB6 HCDR1 | |
| 71 | Amino acid sequence of AB8 HCDR1 | SRKALS |
| 72 | Amino acid sequence of AB6 HCDR2 | AISGTGGSTKYADSVKG |
| 73 | Amino acid sequence of AB8 HCDR2 | TINGRGGRTYYADSVKG |
| 74 | Amino acid sequence of AB7 LCDR1 | RASQSVSRRYLA |
| 75 | Amino acid sequence of AB7 LCDR2 | GGSRRAT |
| 76 | Amino acid sequence of AB6 VH | EVQLLESGGGLVQPGGSLRLSCAASGFTFRRYAISWVRQAPGKGLEWVSAISGT GGSTKYADSVKGRFTISRDNSKNTLYLQMNSLRAEDTAVYYCARGWPSPVTFD YWGQGTLVTVSS |
| 77 | Polynucleotide sequence of AB6 VH | GAGGTGCAATTGCTGGAGTCTGGGGGAGGCTTGGTACAGCCTGGGGGGTCC CTGAGACTCTCCTGTGCAGCCTCCGGATTCACCTTTCGCCGTTATGCGATCAG CTGGGTCCGCCAGGCTCCAGGGAAGGGGCTGGAGTGGGTCTCAGCTATTAGT GGCACTGGAGGTAGCACAAAGTACGCAGACTCCGTGAAGGGCCGGTTCACC ATCTCCAGAGACAATTCCAAGAACACGCTGTATCTGCAGATGAACAGCCTGA GAGCCGAGGACACGGCCGTATATTACTGTGCGCGTGGTTGGCCATCTCCAGTT ACTTTCGACTACTGGGGCCAAGGAACCCTGGTCACCGTCTCGAGT |
| 78 | Amino acid sequence of AB7 VL | EIVLTQSPGTLSLSPGERATLSCRASQSVSRRYLAWYQQKPGQAPRLLIYGGSRR ATGIPDRFSGSGSGTDFTLTISRLEPEDFAVYYCQQYKSHPITFGQGTKVEIKR |
| 79 | Polynucleotide sequence of AB7 VL | GAAATCGTGTTAACGCAGTCTCCAGGCACCCTGTCTTTGTCTCCAGGGGAAA GAGCCACCCTCTCTTGCAGGGCCAGTCAGAGCGTCAGTAGGCGATACTTAGC CTGGTACCAGCAGAAACCTGGCCAGGCTCCCAGGCTCCTCATCTACGGAGGT TCGCGGAGGGCCACTGGCATCCCAGACAGGTTCAGTGGCAGTGGATCCGGG ACAGACTTCACTCTCACCATCAGCAGACTGGAGCCTGAAGATTTTGCAGTGT ATTACTGTCAGCAGTACAAATCTCATCCAATCACGTTCGGCCAGGGGACCAA AGTGGAAATCAAACGT |
| 80 | Amino acid sequence of AB8 VH | EVQLLESGGGLVQPGGSLRLSCAASGFTFSRKALSWVRQAPGKGLEWVSTING RGGRTYYADSVKGRFTISRDNSKNTLYLQMNSLRAEDTAVYYCARGWPSPVTF DYWGQGTLVTVSS |
| 81 | Polynucleotide sequence of AB8 VH | GAGGTGCAATTGCTGGAGTCTGGGGGAGGCTTGGTACAGCCTGGGGGGTCC CTGAGACTCTCCTGTGCAGCCTCCGGATTCACCTTTAGCAGAAAGGCCCTAA GCTGGGTCCGCCAGGCTCCAGGGAAGGGGCTGGAGTGGGTCTCAACTATTAA TGGTAGAGGTGGTCGCACATACTACGCAGACTCCGTGAAGGGCCGGTTCACC |
| | | ATCTCCAGAGACAATTCCAAGAACACGCTGTATCTGCAGATGAACAGCCTGA GAGCCGAGGACACGGCCGTATATTACTGTGCGCGTGGTTGGCCATCTCCAGTT ACTTTCGACTACTGGGGCCAAGGAACCCTGGTCACCGTCTCGAGT |
| 82 | Amino acid sequence of AB6 scFv | EVQLLESGGGLVQPGGSLRLSCAASGFTFRRYAISWVRQAPGKGLEWVSAISGT GGSTKYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSSSYLAWYQQKPGQAPRLLIYGASSRATGIPDRFSGSGSGTDFTLTISRLEPED FAVYYCQQYKSHPITFGQGTKVEIKR |
| 83 | Polynucleotide sequence of AB6 scFv | GAGGTGCAATTGCTGGAGTCTGGGGGAGGCTTGGTACAGCCTGGGGGGTCC CTGAGACTCTCCTGTGCAGCCTCCGGATTCACCTTTCGCCGTTATGCGATCAG CTGGGTCCGCCAGGCTCCAGGGAAGGGGCTGGAGTGGGTCTCAGCTATTAGT GGCACTGGAGGTAGCACAAAGTACGCAGACTCCGTGAAGGGCCGGTTCACC ATCTCCAGAGACAATTCCAAGAACACGCTGTATCTGCAGATGAACAGCCTGA GAGCCGAGGACACGGCCGTATATTACTGTGCGCGTGGTTGGCCATCTCCAGTT ACTTTCGACTACTGGGGCCAAGGAACCCTGGTCACCGTCTCGAGTGGTGGA GGCGGTTCAGGCGGAGGTGGTTCTGGCGGTGGCGGATCGGAAATCGTGTTA ACGCAGTCTCCAGGCACCCTGTCTTTGTCTCCAGGGGAAAGAGCCACCCTCT CTTGCAGGGCCAGTCAGAGTGTTAGCAGCAGCTACTTAGCCTGGTACCAGCA GAAACCTGGCCAGGCTCCCAGGCTCCTCATCTATGGAGCATCCAGCAGGGCC ACTGGCATCCCAGACAGGTTCAGTGGCAGTGGATCCGGGACAGACTTCACTC TCACCATCAGCAGACTGGAGCCTGAAGATTTTGCAGTGTATTACTGTCAGCA GTACAAATCTCATCCAATCACGTTCGGCCAGGGGACCAAAGTGGAAATCAAA CGT |
| 84 | Amino acid sequence of AB7 scFv | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSRRYLAWYQQKPGQAPRLLIYGGSRRATGIPDRFSGSGSGTDFTLTISRLEPED FAVYYCQQYKSHPITFGQGTKVEIKR |
| 85 | Polynucleotide sequence of AB7 scFv | GAGGTGCAATTGCTGGAGTCTGGGGGAGGCTTGGTACAGCCTGGGGGGTCC CTGAGACTCTCCTGTGCAGCCTCCGGATTCACCTTTAGCAGTTATGCCATGAG CTGGGTCCGCCAGGCTCCAGGGAAGGGGCTGGAGTGGGTCTCAGCTATTAGT GGTAGTGGTGGTAGCACATACTACGCAGACTCCGTGAAGGGCCGGTTCACCA TCTCCAGAGACAATTCCAAGAACACGCTGTATCTGCAGATGAACAGCCTGAG AGCCGAGGACACGGCCGTATATTACTGTGCGCGTGGTTGGCCATCTCCAGTTA CTTTCGACTACTGGGGCCAAGGAACCCTGGTCACCGTCTCGAGTGGTGGAG GCGGTTCAGGCGGAGGTGGTTCTGGCGGTGGCGGATCGGAAATCGTGTTAAC GCAGTCTCCAGGCACCCTGTCTTTGTCTCCAGGGGAAAGAGCCACCCTCTCT TGCAGGGCCAGTCAGAGCGTCAGTAGGCGATACTTAGCCTGGTACCAGCAGA AACCTGGCCAGGCTCCCAGGCTCCTCATCTACGGAGGTTCGCGGAGGGCCA CTGGCATCCCAGACAGGTTCAGTGGCAGTGGATCCGGGACAGACTTCACTCT CACCATCAGCAGACTGGAGCCTGAAGATTTTGCAGTGTATTACTGTCAGCAG TACAAATCTCATCCAATCACGTTCGGCCAGGGGACCAAAGTGGAAATCAAAC GT |
| 86 | Amino acid | EVQLLESGGGLVQPGGSLRLSCAASGFTFSRKALSWVRQAPGKGLEWVSTING |
| | sequence of AB8 scFv | RGGRTYYADSVKGRFTISRDNSKNTLYLQMNSLRAEDTAVYYCARGWPSPVTF DYWGQGTLVTVSSGGGGSGGGGSGGGGSEIVLTQSPGTLSLSPGERATLSCRAS QSVSSSYLAWYQQKPGQAPRLLIYGASSRATGIPDRFSGSGSGTDFTLTISRLEPE DFAVYYCQQYKSHPITFGQGTKVEIKR |
| 87 | Polynucleotide sequence of AB8 scFv | GAGGTGCAATTGCTGGAGTCTGGGGGAGGCTTGGTACAGCCTGGGGGGTCC CTGAGACTCTCCTGTGCAGCCTCCGGATTCACCTTTAGCAGAAAGGCCCTAA GCTGGGTCCGCCAGGCTCCAGGGAAGGGGCTGGAGTGGGTCTCAACTATTAA TGGTAGAGGTGGTCGCACATACTACGCAGACTCCGTGAAGGGCCGGTTCACC ATCTCCAGAGACAATTCCAAGAACACGCTGTATCTGCAGATGAACAGCCTGA GAGCCGAGGACACGGCCGTATATTACTGTGCGCGTGGTTGGCCATCTCCAGTT ACTTTCGACTACTGGGGCCAAGGAACCCTGGTCACCGTCTCGAGTGGTGGA GGCGGTTCAGGCGGAGGTGGTTCTGGCGGTGGCGGATCGGAAATCGTGTTA ACGCAGTCTCCAGGCACCCTGTCTTTGTCTCCAGGGGAAAGAGCCACCCTCT CTTGCAGGGCCAGTCAGAGTGTTAGCAGCAGCTACTTAGCCTGGTACCAGCA GAAACCTGGCCAGGCTCCCAGGCTCCTCATCTATGGAGCATCCAGCAGGGCC ACTGGCATCCCAGACAGGTTCAGTGGCAGTGGATCCGGGACAGACTTCACTC TCACCATCAGCAGACTGGAGCCTGAAGATTTTGCAGTGTATTACTGTCAGCA GTACAAATCTCATCCAATCACGTTCGGCCAGGGGACCAAAGTGGAAATCAAA CGT |
| 88 | Amino acid sequence of AB6-BBZ | EVQLLESGGGLVQPGGSLRLSCAASGFTFRRYAISWVRQAPGKGLEWVSAISGT GGSTKYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSSSYLAWYQQKPGQAPRLLIYGASSRATGIPDRFSGSGSGTDFTLTISRLEPED FAVYYCQQYKSHPITFGQGTKVEIKRTTTPAPRPPTPAPTIASQPLSLRPEACRPAA GGAVHTRGLDFACDIYIWAPLAGTCGVLLLSLVITLYCKRGRKKLLYIFKQPFMR PVQTTQEEDGCSCRFPEEEEGGCELRVKFSRSADAPAYQQGQNQLYNELNLGRR EEYDVLDKRRGRDPEMGGKPQRRKNPQEGLYNELQKDKMAEAYSEIGMKGER RRGKGHDGLYQGLSTATKDTYDALHMQALPPR |
| 89 | Amino acid sequence of AB7-BBZ | EVQLLESGGGLVQPGGSLRLSCAASGFTFSSYAMSWVRQAPGKGLEWVSAISGS GGSTYYADSVKGRFTISRDNSKNTLYLQMNSLRAEDTAVYYCARGWPSPVTFD YWGQGTLVTVSSGGGGSGGGGSGGGGSEIVLTQSPGTLSLSPGERATLSCRASQ SVSRRYLAWYQQKPGQAPRLLIYGGSRRATGIPDRFSGSGSGTDFTLTISRLEPED FAVYYCQQYKSHPITFGQGTKVEIKRTTTPAPRPPTPAPTIASQPLSLRPEACRPAA GGAVHTRGLDFACDIYIWAPLAGTCGVLLLSLVITLYCKRGRKKLLYIFKQPFMR PVQTTQEEDGCSCRFPEEEEGGCELRVKFSRSADAPAYQQGQNQLYNELNLGRR EEYDVLDKRRGRDPEMGGKPQRRKNPQEGLYNELQKDKMAEAYSEIGMKGER RRGKGHDGLYQGLSTATKDTYDALHMQALPPR |
| 90 | Amino acid sequence of AB8-BBZ | EVQLLESGGGLVQPGGSLRLSCAASGFTFSRKALSWVRQAPGKGLEWVSTING RGGRTYYADSVKGRFTISRDNSKNTLYLQMNSLRAEDTAVYYCARGWPSPVTF DYWGQGTLVTVSSGGGGSGGGGSGGGGSEIVLTQSPGTLSLSPGERATLSCRAS QSVSSSYLAWYQQKPGQAPRLLIYGASSRATGIPDRFSGSGSGTDFTLTISRLEPE DFAVYYCQQYKSHPITFGQGTKVEIKRTTTPAPRPPTPAPTIASQPLSLRPEACRPA AGGAVHTRGLDFACDIYIWAPLAGTCGVLLLSLVITLYCKRGRKKLLYIFKQPFM RPVQTTQEEDGCSCRFPEEEEGGCELRVKFSRSADAPAYQQGQNQLYNELNLGR |
| | | REEYDVLDKRRGRDPEMGGKPQRRKNPQEGLYNELQKDKMAEAYSEIGMKGE RRRGKGHDGLYQGLSTATKDTYDALHMQALPPR |
| 91 | Amino acid sequence of Fc in human IgG | DKTHTCPPCPAPELLGGPSVFLFPPKPKDTLMISRTPEVTCVVVDVSHEDPEVKF NWYVDGVEVHNAKTKPREEQYNSTYRVVSVLTVLHQDWLNGKEYKCKVSNK ALPAPIEKTISKAKGQPREPQVYTLPPSRDELTKNQVSLTCLVKGFYPSDIAVEWE SNGQPENNYKTTPPVLDSDGSFFLYSKLTVDKSRWQQGNVFSCSVMHEALHNH YTQKSLSLSPGK |

## Claims

1. An antibody recognizing GPRC5D, wherein the antibody is any one selected from the group consisting of:
(1) an antibody, comprising a heavy chain variable region, wherein the heavy chain variable region comprises a HCDR1 as shown in any one of SEQ ID NOs: 1, 11, 17, 70 or 71, and/or comprises a HCDR2 as shown in any one of SEQ ID NOs: 2, 12, 18, 72 or 73, and/or comprises a HCDR3 as shown in any one of SEQ ID NOs: 3, 7, 9, 13 or 19;
(2) an antibody, comprising a light chain variable region, wherein the light chain variable region comprises a LCDR1 as shown in any one of SEQ ID NOs: 4, 14, 20, or 74, and/or comprises a LCDR2 as shown in any one of SEQ ID NOs: 5, 15, 21, or 75, and/or comprises a LCDR3 as shown in any one of SEQ ID NOs: 6, 8, 10, 16, or 22;
(3) an antibody, comprising the heavy chain variable region of the antibody described in (1) and the light chain variable region of the antibody described in (2); and
(4) an antibody, which is a variant of the antibody described in any one of (1) to (3), and has the same or similar activity as the antibody described in any one of (1) to (3).

2. The antibody of claim 1, comprising at least one CDR in the heavy chain variable region, wherein the heavy chain variable region comprises an amino acid sequence as shown in any one of SEQ ID NOs: 23, 27, 31, 35, 39, 76 or 80 or a variant thereof, or comprises an amino acid sequence having at least 80% similarity to any one of the above sequences; and/or comprising at least one CDR in the light chain variable region, wherein the light chain variable region comprises an amino acid sequence as shown in any one of SEQ ID NOs: 25, 29, 33, 37, 41 or 78 or a variant thereof, or comprises an amino acid sequence having at least 80% similarity to any one of the above sequences.

3. The antibody of claim 1, comprising HCDR1, HCDR2, and HCDR3 in the heavy chain variable region, wherein the heavy chain variable region comprises an amino acid sequence as shown in any one of SEQ ID NOs: 23, 27, 31, 35, 39, 76 or 80 or a variant thereof, or comprises an amino acid sequence having at least 80% similarity to any one of the above sequences; and/or comprising LCDR1, LCDR2, and LCDR3 in the light chain variable region, wherein the light chain variable region comprises an amino acid sequence as shown in any one of SEQ ID NOs: 25, 29, 33, 37, 41 or 78 or a variant thereof, or comprises an amino acid sequence having at least 80% similarity to any one of the above sequences.

4. The antibody of claim 1, wherein the CDR regions of the heavy chain variable region and/or the CDR regions of the light chain variable region of the antibody are selected from the CDR sequences of any one of the following antibodies:
(1) an antibody, comprising HCDR1 of SEQ ID NO: 1, HCDR2 of SEQ ID NO: 2, and HCDR3 of SEQ ID NO: 3; LCDR1 of SEQ ID NO: 4, LCDR2 of SEQ ID NO: 5, and LCDR3 of SEQ ID NO: 6; or
(2) an antibody, comprising HCDR1 of SEQ ID NO: 1, HCDR2 of SEQ ID NO: 2, and HCDR3 of SEQ ID NO: 7; LCDR1 of SEQ ID NO: 4, LCDR2 of SEQ ID NO: 5, and LCDR3 of SEQ ID NO: 8; or
(3) an antibody, comprising HCDR1 of SEQ ID NO: 1, HCDR2 of SEQ ID NO: 2, and HCDR3 of SEQ ID NO: 9; LCDR1 of SEQ ID NO: 4, LCDR2 of SEQ ID NO: 5, and LCDR3 of SEQ ID NO: 10; or
(4) an antibody, comprising HCDR1 of SEQ ID NO: 11, HCDR2 of SEQ ID NO: 12, and HCDR3 of SEQ ID NO: 13; LCDR1 of SEQ ID NO: 14, LCDR2 of SEQ ID NO: 15, and LCDR3 of SEQ ID NO: 16; or
(5) an antibody, comprising HCDR1 of SEQ ID NO: 17, HCDR2 of SEQ ID NO: 18, and HCDR3 of SEQ ID NO: 19; LCDR1 of SEQ ID NO: 20, LCDR2 of SEQ ID NO: 21, and LCDR3 of SEQ ID NO: 22; or
(6) an antibody, comprising HCDR1 of SEQ ID NO:70, HCDR2 of SEQ ID NO:72, and HCDR3 of SEQ ID NO:3; LCDR1 of SEQ ID NO:4, LCDR2 of SEQ ID NO:5, and LCDR3 of SEQ ID NO:6; or
(7) an antibody, comprising HCDR1 of SEQ ID NO:71, HCDR2 of SEQ ID NO:73, and HCDR3 of SEQ ID NO:3; LCDR1 of SEQ ID NO:4, LCDR2 of SEQ ID NO:5, and LCDR3 of SEQ ID NO:6; or
(8) an antibody, comprising HCDR1 of SEQ ID NO: 1, HCDR2 of SEQ ID NO: 2, and HCDR3 of SEQ ID NO: 3; LCDR1 of SEQ ID NO: 74, LCDR2 of SEQ ID NO: 75, and LCDR3 of SEQ ID NO: 6;
(9) an antibody, which is a variant of the antibody described in any one of (1) to (8), and has the same or similar activity as the antibody described in any one of (1) to (8).

5. The antibody of claim 2 or 3, wherein the antibody is any one selected from the group consisting of:
(1) an antibody, wherein the heavy chain variable region of the antibody has an amino acid sequence of SEQ ID NO:23 or an amino acid sequence having at least 80% similarity to SEQ ID NO:23, and the light chain variable region of the antibody has an amino acid sequence of SEQ ID NO:25 or an amino acid sequence having at least 80% similarity to SEQ ID NO:25;
(2) an antibody, wherein the heavy chain variable region of the antibody has an amino acid sequence of SEQ ID NO:27 or an amino acid sequence having at least 80% similarity to SEQ ID NO:27, and the light chain variable region of the antibody has an amino acid sequence of SEQ ID NO:29 or an amino acid sequence having at least 80% similarity to SEQ ID NO:29;
(3) an antibody, wherein the heavy chain variable region of the antibody has an amino acid sequence of SEQ ID NO:31 or an amino acid sequence having at least 80% similarity to SEQ ID NO:31, and the light chain variable region of the antibody has an amino acid sequence of SEQ ID NO:33 or an amino acid sequence having at least 80% similarity to SEQ ID NO:33;
(4) an antibody, wherein the heavy chain variable region of the antibody has an amino acid sequence of SEQ ID NO:35 or an amino acid sequence having at least 80% similarity to SEQ ID NO:35, and the light chain variable region of the antibody has an amino acid sequence of SEQ ID NO:37 or an amino acid sequence having at least 80% similarity to SEQ ID NO:37;
(5) an antibody, wherein the heavy chain variable region of the antibody has an amino acid sequence of SEQ ID NO:39 or an amino acid sequence having at least 80% similarity to SEQ ID NO:39, and the light chain variable region of the antibody has an amino acid sequence of SEQ ID NO:41 or an amino acid sequence having at least 80% similarity to SEQ ID NO:40;
(6) an antibody, wherein the heavy chain variable region of the antibody has an amino acid sequence of SEQ ID NO:76 or an amino acid sequence having at least 80% similarity to SEQ ID NO:76, and the light chain variable region of the antibody has an amino acid sequence of SEQ ID NO:25 or an amino acid sequence having at least 80% similarity to SEQ ID NO:25;
(7) an antibody, wherein the heavy chain variable region of the antibody has an amino acid sequence of SEQ ID NO:80 or an amino acid sequence having at least 80% similarity to SEQ ID NO:80, and the light chain variable region of the antibody has an amino acid sequence of SEQ ID NO:25 or an amino acid sequence having at least 80% similarity to SEQ ID NO:25;
(8) an antibody, wherein the heavy chain variable region of the antibody has an amino acid sequence of SEQ ID NO:23 or an amino acid sequence having at least 80% similarity to SEQ ID NO:23, and the light chain variable region of the antibody has an amino acid sequence of SEQ ID NO:78 or an amino acid sequence having at least 80% similarity to SEQ ID NO:78; and
(9) an antibody, which is a variant of the antibody described in any one of (1) to (8), and has the same or similar activity as the antibody described in any one of (1) to (8).

6. The antibody of any one of claims 1 to 5, wherein the antibody is selected from the group consisting of a whole antibody, a scFv, a single domain antibody, a Fab fragment, a Fab' fragment, a Fv fragment, a F(ab')₂ fragment, a Fd fragment, a dAb fragment, a multifunctional antibody or a scFv-Fc antibody, a hybridoma antibody, a chimeric antibody, a humanized antibody, a fully human antibody, and a monoclonal antibody.

7. The antibody of claim 6, wherein the antibody is a hybridoma antibody selected from the group consisting of:
(1) an antibody, wherein the heavy chain variable region of the antibody has an amino acid sequence of SEQ ID NO:35 or an amino acid sequence having at least 80% similarity to SEQ ID NO:35, and the light chain variable region of the antibody has an amino acid sequence of SEQ ID NO:37 or an amino acid sequence having at least 80% similarity to SEQ ID NO:37;
(2) an antibody, wherein the heavy chain variable region of the antibody has an amino acid sequence of SEQ ID NO:39 or an amino acid sequence having at least 80% similarity to SEQ ID NO:39, and the light chain variable region of the antibody has an amino acid sequence of SEQ ID NO:41 or an amino acid sequence having at least 80% similarity to SEQ ID NO:41; and
(3) an antibody, which is a variant of the antibody described in any one of (1) to (2), and has the same or similar activity as the antibody described in any one of (1) to (2).

8. The antibody of claim 6, wherein the antibody is a fully human antibody selected from the group consisting of:
(1) an antibody, wherein the heavy chain variable region of the antibody has an amino acid sequence of SEQ ID NO:23 or an amino acid sequence having at least 80% similarity to SEQ ID NO:23, and the light chain variable region of the antibody has an amino acid sequence of SEQ ID NO:25 or an amino acid sequence having at least 80% similarity to SEQ ID NO:25;
(2) an antibody, wherein the heavy chain variable region of the antibody has an amino acid sequence of SEQ ID NO:27 or an amino acid sequence having at least 80% similarity to SEQ ID NO:27, and the light chain variable region of the antibody has an amino acid sequence of SEQ ID NO:29 or an amino acid sequence having at least 80% similarity to SEQ ID NO:29;
(3) an antibody, wherein the heavy chain variable region of the antibody has an amino acid sequence of SEQ ID NO:31 or an amino acid sequence having at least 80% similarity to SEQ ID NO:31, and the light chain variable region of the antibody has an amino acid sequence of SEQ ID NO:33 or an amino acid sequence having at least 80% similarity to SEQ ID NO:33;
(4) an antibody, wherein the heavy chain variable region of the antibody has an amino acid sequence of SEQ ID NO:76 or an amino acid sequence having at least 80% similarity to SEQ ID NO:76, and the light chain variable region of the antibody has an amino acid sequence of SEQ ID NO:25 or an amino acid sequence having at least 80% similarity to SEQ ID NO:25;
(5) an antibody, wherein the heavy chain variable region of the antibody has an amino acid sequence of SEQ ID NO:80 or an amino acid sequence having at least 80% similarity to SEQ ID NO:80, and the light chain variable region of the antibody has an amino acid sequence of SEQ ID NO:25 or an amino acid sequence having at least 80% similarity to SEQ ID NO:25;
(6) an antibody, wherein the heavy chain variable region of the antibody has an amino acid sequence of SEQ ID NO:23 or an amino acid sequence having at least 80% similarity to SEQ ID NO:23, and the light chain variable region of the antibody has an amino acid sequence of SEQ ID NO:78 or an amino acid sequence having at least 80% similarity to SEQ ID NO:78; and
(7) an antibody, which is a variant of the antibody described in any one of (1) to (6), and has the same or similar activity as the antibody described in any one of (1) to (6).

9. The antibody of claim 8, wherein the antibody is any one selected from the group consisting of:
(1) an antibody, wherein the heavy chain of the antibody has an amino acid sequence of SEQ ID NO:45 or an amino acid sequence having at least 80% similarity to SEQ ID NO:45, and the light chain of the antibody has an amino acid sequence of SEQ ID NO:47 or an amino acid sequence having at least 80% similarity to SEQ ID NO:47;
(2) an antibody, wherein the heavy chain of the antibody has an amino acid sequence of SEQ ID NO:49 or an amino acid sequence having at least 80% similarity to SEQ ID NO:49, and the light chain of the antibody has an amino acid sequence of SEQ ID NO:51 or an amino acid sequence having at least 80% similarity to SEQ ID NO:51;
(3) an antibody, wherein the heavy chain of the antibody has an amino acid sequence of SEQ ID NO:53 or an amino acid sequence having at least 80% similarity to SEQ ID NO:53, and the light chain of the antibody has an amino acid sequence of SEQ ID NO:55 or an amino acid sequence having at least 80% similarity to SEQ ID NO:55; and
(4) an antibody, which is a variant of the antibody described in any one of (1) to (3), and has the same or similar activity as the antibody described in any one of (1) to (3).

10. An immunoconjugate, wherein the immunoconjugate comprises: the antibody according to any one of claims 1 to 9, and a functional molecule connected thereto.

11. A chimeric receptor, wherein an extracellular domain of the chimeric receptor comprises the antibody according to any one of claims 1-9, and the chimeric receptor comprises: a chimeric antigen receptor (CAR), a chimeric T cell receptor, a T cell antigen coupler (TAC) or a combination thereof.

12. The chimeric receptor of claim 11, wherein the chimeric receptor is a chimeric antigen receptor (CAR), comprising the antibody according to any one of claims 1 to 9, a transmembrane region and an intracellular signaling region.

13. The chimeric receptor of claim 12, wherein the intracellular signaling region is selected from the group consisting of: intracellular signaling region sequences of CD3ζ, FcεRIy, CD27, CD28, CD137, CD134, MyD88, or CD40 and a combination thereof; and/or the transmembrane region comprises the transmembrane region of CD8 or CD28.

14. The chimeric receptor of claim 13, wherein the chimeric receptor is selected from any one of the following groups:
the antibody according to any one of claims 1 to 9, the transmembrane region of CD8/CD28 and CD3ζ; or
the antibody according to any one of claims 1 to 9, the transmembrane region of CD8/CD28, the intracellular signaling region of CD137 and CD3ζ; or
the antibody according to any one of claims 1 to 9, the transmembrane region of CD8/CD28, the intracellular signaling region of CD28 and CD3ζ; or
the antibody according to any one of claims 1 to 9, the transmembrane region of CD8/CD28, the intracellular signaling region of CD28, CD137 and CD3ζ.

15. The chimeric receptor of claim 11, wherein that the amino acid sequence of the chimeric receptor is shown in any one of SEQ ID NOs: 63, 88, 89, and 90.

16. A biological material, which is any one selected from the group consisting of:
(1) a nucleic acid, encoding the antibody of any one of claims 1 to 9, the immunoconjugate of claim 10, or the chimeric receptor of any one of claims 11 to 15;
(2) a vector comprising the nucleic acid described in (1); and
(3) a virus comprising the nucleic acid or vector described in (1) or (2).

17. A cell, comprising the antibody according to any one of claims 1 to 9, the immunoconjugate of claim 10, the chimeric receptor according to any one of claims 11 to 15, the nucleic acid described in (1) of claim 16, and/or the vector described in (2) of claim 16.

18. The cell of claim 17, wherein the cell comprises a T cell, a natural killer cell, a natural killer T cell, a NK92 cell, an immune effector cell comes from stem cell, or a combination thereof.

19. The cell of claim 18, wherein the T cells comprises a natural T cell and/or a T cell induced by a pluripotent stem cell;
preferably, the T cell comprises an autologous T cell and/or an allogeneic T cell;
preferably, the T cell is a primary T cell;
preferably, the T cell comes from a human autologous T cell.

20. The cell described in any one of claims 17 to 19, wherein it further carries a coding sequence of an exogenous cytokine; and/or
it further expresses a chimeric receptor not targeting GPRC5D; and/or
it further expresses a chemokine; and/or
it further expresses a chemokine receptor; and/or
it further expresses a safety switch.

21. A pharmaceutical composition, comprising the antibody according to any one of claims 1 to 9, the immunoconjugate according to claim 10, the chimeric receptor according to any one of claims 11 to 15, the nucleic acid described in (1) of claim 16, the vector described in (2) of claim 16, and/or the cell according to any one of claims 17 to 20, and a pharmaceutically acceptable adjuvant.

22. A combination therapy, wherein the antibody according to any one of claims 1 to 9, the immunoconjugate according to claim 10, the chimeric receptor according to any one of claims 11 to 15, the cell according to any one of claims 17 to 20, the pharmaceutical composition according to claim 21 is administered in combination with an agent that enhances the function thereof, preferably in combination with a chemotherapeutic agent; and/or
co-administered with an agent that ameliorates one or more side effects associated therewith; and/or
co-administered with cells expressing a chimeric antigen receptor targeting a target other than GPRC5D.

23. A method for preparing the antibody according to any one of claims 1 to 9, the immunoconjugate according to claim 10, and the chimeric receptor according to any one of claims 11 to 15, comprising culturing the cell according to any one of claims 17 to 20 under a condition suitable for expressing the antibody, immunoconjugate, or chimeric receptor, and isolating the antibody, immunoconjugate, or chimeric receptor expressed by the cell.

24. A kit, wherein it comprises the antibody according to any one of claims 1 to 9, the immunoconjugate according to claim 10, the chimeric receptor according to any one of claims 11 to 15, the nucleic acid described in (1) of claim 16, the vector described in (2) of claim 16, the cell according to any one of claims 17 to 20, and/or the pharmaceutical composition according to claim 21.

25. A method for treating/diagnosing a disease, comprising administering to a subject in need thereof an effective amount of the antibody according to any one of claims 1 to 9, the immunoconjugate according to claim 10, the cell according to any one of claims 17 to 20, the pharmaceutical composition according to claim 21, or the kit according to claim 24;
preferably, the disease is selected from the group consisting of: inflammatory diseases, infections, autoimmune diseases and tumors;
preferably, the subject is a human;
preferably, wherein the cell is an autologous or allogeneic T cell to the subject.

26. Use of the antibody according to any one of claims 1 to 9, the immunoconjugate according to claim 10, the cell according to any one of claims 17 to 20, the pharmaceutical composition according to claim 21, and/or the kit according to claim 24 in the preparation of a medicament for treating/diagnosing a disease, wherein the disease comprises expression of GPRC5D; preferably, the disease is selected from the group consisting of: inflammatory diseases, infections, autoimmune diseases and tumors.
